# FASCICULE DE BREVET EUROPEEN

(11) **EP 2 125 885 B1**
(45) Date de publication et mention de la délivrance du brevet: **16.10.2013**
(21) Numéro de dépôt: 08775649.0
(22) Date de dépôt: 22.02.2008
(51) Int. Cl.: C07K 14/755

(54) **NOUVEAUX FACTEURS VIII POUR LE TRAITEMENT DE HEMOPHILES DE TYPE A**
NEUARTIGE VIII-FAKTOREN ZUR BEHANDLUNG VON HÄMOPHILIE DES TYPS A
NOVEL VIII FACTORS FOR THE TREATMENT OF TYPE A HEMOPHILIA

(30) Priorité: 23.02.2007 FR 0753450
(43) Date de publication de la demande: 02.12.2009
(73) Titulaire: Biomethodes, 91030 Evry (FR); Hospices Civils De Lyon, 69424 Lyon Cedex 03 (FR)
(72) Inventeur: SABOULARD, Didier, F-31800 Saint Gaudens (FR); PLANTIER, Jean-Luc, F-69520 Gringy (FR); DELCOURT, Marc, F-75013 Paris (FR); NEGRIER, Claude, F-69540 Irigny (FR); MENGUY, Thierry, F-91190 Gif sur Yvette (FR); BLESA, Stéphane, F-77166 Grisy-suisnes (FR); MARIN, Sylvie, F-69360 COMMUNAY (FR)
(74) Mandataire: Gallois, Valérie
(86) Numéro de dépôt international: PCT/FR2008/050301
(87) Numéro de publication internationale: WO 2008/129180

(56) Documents cités:
- EP-B- 1 200 105
- WO-A-2005/046583
- US-B2- 6 919 311
- BARROW R T ET AL: "Reduction of the antigenicity of factor VIII toward complex inhibitory antibody plasmas using multiply-substituted hybrid human/oorcine factor VIII molecules" BLOOD, W.B.SAUNDERS COMPANY, ORLANDO, FL, US, vol. 95, no. 2, 15 janvier 2000 (2000-01-15), pages 564-568, XP002963601 ISSN: 0006-4971

### SEQUENCE LISTING

<110> BIOMETHODES et HOSPICES CIVILS DE LYON
<120> NOUVEAUX FACTEURS VIII POUR LE TRAITEMENT DES HEMOPHILES DE TYPE A
<130> B0474WO
<160> 5
<170> PatentIn version 3.1
<210> 1
   <211> 9028
   <212> DNA
   <213> Homo sapiens
<220>
   <221> CDS
   <222> (172)..(7227)
   <223>
<220>
   <221> sig_peptide
   <222> (172)..(228)
   <223>
<400> 1
   gcttagtgct gagcacatcc agtgggtaaa gttccttaaa atgctctgca aagaaattgg      60
   gacttttcat taaatcagaa attttacttt tttcccctcc tgggagctaa agatatttta     120
   gagaagaatt aaccttttgc ttctccagtt gaacatttgt agcaataagt c atg caa      177
                                                            Met Gln
                                                            1
   ata gag ctc tcc acc tgc ttc ttt ctg tgc ctt ttg cga ttc tgc ttt       225
   Ile Glu Leu Ser Thr Cys Phe Phe Leu Cys Leu Leu Arg Phe Cys Phe
           5                   10                  15
   agt gcc acc aga aga tac tac ctg ggt gca gtg gaa ctg tca tgg gac       273
   Ser Ala Thr Arg Arg Tyr Tyr Leu Gly Ala Val Glu Leu Ser Trp Asp
            20                      25                      30
   tat atg caa agt gat ctc ggt gag ctg cct gtg gac gca aga ttt cct        321
   Tyr Met Gln Ser Asp Leu Gly Glu Leu Pro Val Asp Ala Arg Phe Pro
   35                  40                  45                  50
   cct aga gtg cca aaa tct ttt cca ttc aac acc tca gtc gtg tac aaa        369
   Pro Arg Val Pro Lys Ser Phe Pro Phe Asn Thr Ser Val Val Tyr Lys
                   55                  60                  65
   aag act ctg ttt gta gaa ttc acg gat cac ctt ttc aac atc gct aag        417
   Lys Thr Leu Phe Val Glu Phe Thr Asp His Leu Phe Asn Ile Ala Lys
               70                  75                  80
   cca agg cca ccc tgg atg ggt ctg cta ggt cct acc atc cag gct gag        465
   Pro Arg Pro Pro Trp Met Gly Leu Leu Gly Pro Thr Ile Gln Ala Glu
           85                  90                  95
   gtt tat gat aca gtg gtc att aca ctt aag aac atg gct tcc cat cct        513
   Val Tyr Asp Thr Val Val Ile Thr Leu Lys Asn Met Ala Ser His Pro
       100                 105                 110
   gtc agt ctt cat gct gtt ggt gta tcc tac tgg aaa gct tct gag gga        561
   Val Ser Leu His Ala Val Gly Val Ser Tyr Trp Lys Ala Ser Glu Gly
   115                 120                 125                 130
   gct gaa tat gat gat cag acc agt caa agg gag aaa gaa gat gat aaa        609
   Ala Glu Tyr Asp Asp Gln Thr Ser Gln Arg Glu Lys Glu Asp Asp Lys
                   135                 140                 145
   gtc ttc cct ggt gga agc cat aca tat gtc tgg cag gtc ctg aaa gag        657
   Val Phe Pro Gly Gly Ser His Thr Tyr Val Trp Gln Val Leu Lys Glu
               150                 155                 160
   aat ggt cca atg gcc tct gac cca ctg tgc ctt acc tac tca tat ctt        705
   Asn Gly Pro Met Ala Ser Asp Pro Leu Cys Leu Thr Tyr Ser Tyr Leu
           165                 170                 175
   tct cat gtg gac ctg gta aaa gac ttg aat tca ggc ctc att gga gcc        753
   Ser His Val Asp Leu Val Lys Asp Leu Asn Ser Gly Leu Ile Gly Ala
       180                 185                 190
   cta cta gta tgt aga gaa ggg agt ctg gcc aag gaa aag aca cag acc        801
   Leu Leu Val Cys Arg Glu Gly Ser Leu Ala Lys Glu Lys Thr Gln Thr
   195                 200                 205                 210
   ttg cac aaa ttt ata cta ctt ttt gct gta ttt gat gaa ggg aaa agt        849
   Leu His Lys Phe Ile Leu Leu Phe Ala Val Phe Asp Glu Gly Lys Ser
                   215                 220                 225
   tgg cac tca gaa aca aag aac tcc ttg atg cag gat agg gat gct gca        897
   Trp His Ser Glu Thr Lys Asn Ser Leu Met Gln Asp Arg Asp Ala Ala
               230                 235                 240
   tct gct cgg gcc tgg cct aaa atg cac aca gtc aat ggt tat gta aac        945
   Ser Ala Arg Ala Trp Pro Lys Met His Thr Val Asn Gly Tyr Val Asn
           245                 250                 255
   agg tct ctg cca ggt ctg att gga tgc cac agg aaa tca gtc tat tgg        993
   Arg Ser Leu Pro Gly Leu Ile Gly Cys His Arg Lys Ser Val Tyr Trp
       260                 265                 270
   cat gtg att gga atg ggc acc act cct gaa gtg cac tca ata ttc ctc       1041
   His Val Ile Gly Met Gly Thr Thr Pro Glu Val His Ser Ile Phe Leu
   275                 280                 285                 290
   gaa ggt cac aca ttt ctt gtg agg aac cat cgc cag gcg tcc ttg gaa       1089
   Glu Gly His Thr Phe Leu Val Arg Asn His Arg Gln Ala Ser Leu Glu
                   295                 300                 305
   atc tcg cca ata act ttc ctt act gct caa aca ctc ttg atg gac ctt     1137
   Ile Ser Pro Ile Thr Phe Leu Thr Ala Gln Thr Leu Leu Met Asp Leu
               310                 315                 320
   gga cag ttt cta ctg ttt tgt cat atc tct tcc cac caa cat gat ggc     1185
   Gly Gln Phe Leu Leu Phe Cys His Ile Ser Ser His Gln His Asp Gly
           325                 330                 335
   atg gaa gct tat gtc aaa gta gac agc tgt cca gag gaa ccc caa cta     1233
   Met Glu Ala Tyr Val Lys Val Asp Ser Cys Pro Glu Glu Pro Gln Leu
       340                 345                 350
   cga atg aaa aat aat gaa gaa gcg gaa gac tat gat gat gat ctt act     1281
   Arg Met Lys Asn Asn Glu Glu Ala Glu Asp Tyr Asp Asp Asp Leu Thr
   355                 360                 365                 370
   gat tct gaa atg gat gtg gtc agg ttt gat gat gac aac tct cct tcc     1329
   Asp Ser Glu Met Asp Val Val Arg Phe Asp Asp Asp Asn Ser Pro Ser
                   375                 380                 385
   ttt atc caa att cgc tca gtt gcc aag aag cat cct aaa act tgg gta     1377
   Phe Ile Gln Ile Arg Ser Val Ala Lys Lys His Pro Lys Thr Trp Val
               390                 395                 400
   cat tac att gct gct gaa gag gag gac tgg gac tat gct ccc tta gtc     1425
   His Tyr Ile Ala Ala Glu Glu Glu Asp Trp Asp Tyr Ala Pro Leu Val
           405                 410                 415
   ctc gcc ccc gat gac aga agt tat aaa agt caa tat ttg aac aat ggc     1473
   Leu Ala Pro Asp Asp Arg Ser Tyr Lys Ser Gln Tyr Leu Asn Asn Gly
       420                 425                 430
   cct cag cgg att ggt agg aag tac aaa aaa gtc cga ttt atg gca tac     1521
   Pro Gln Arg Ile Gly Arg Lys Tyr Lys Lys Val Arg Phe Met Ala Tyr
   435                 440                 445                 450
   aca gat gaa acc ttt aag act cgt gaa gct att cag cat gaa tca gga     1569
   Thr Asp Glu Thr Phe Lys Thr Arg Glu Ala Ile Gln His Glu Ser Gly
                   455                 460                 465
   atc ttg gga cct tta ctt tat ggg gaa gtt gga gac aca ctg ttg att     1617
   Ile Leu Gly Pro Leu Leu Tyr Gly Glu Val Gly Asp Thr Leu Leu Ile
               470                 475                 480
   ata ttt aag aat caa gca agc aga cca tat aac atc tac cct cac gga     1665
   Ile Phe Lys Asn Gln Ala Ser Arg Pro Tyr Asn Ile Tyr Pro His Gly
           485                 490                 495
   atc act gat gtc cgt cct ttg tat tca agg aga tta cca aaa ggt gta     1713
   Ile Thr Asp Val Arg Pro Leu Tyr Ser Arg Arg Leu Pro Lys Gly Val
       500                 505                 510
   aaa cat ttg aag gat ttt cca att ctg cca gga gaa ata ttc aaa tat     1761
   Lys His Leu Lys Asp Phe Pro Ile Leu Pro Gly Glu Ile Phe Lys Tyr
   515                 520                 525                 530
   aaa tgg aca gtg act gta gaa gat ggg cca act aaa tca gat cct cgg     1809
   Lys Trp Thr Val Thr Val Glu Asp Gly Pro Thr Lys Ser Asp Pro Arg
                   535                 540                 545
   tgc ctg acc cgc tat tac tct agt ttc gtt aat atg gag aga gat cta     1857
   Cys Leu Thr Arg Tyr Tyr Ser Ser Phe Val Asn Met Glu Arg Asp Leu
               550                 555                 560
   gct tca gga ctc att ggc cct ctc ctc atc tgc tac aaa gaa tct gta     1905
   Ala Ser Gly Leu Ile Gly Pro Leu Leu Ile Cys Tyr Lys Glu Ser Val
           565                 570                 575
   gat caa aga gga aac cag ata atg tca gac aag agg aat gtc atc ctg       1953
   Asp Gln Arg Gly Asn Gln Ile Met Ser Asp Lys Arg Asn Val Ile Leu
       580                 585                 590
   ttt tct gta ttt gat gag aac cga agc tgg tac ctc aca gag aat ata       2001
   Phe Ser Val Phe Asp Glu Asn Arg Ser Trp Tyr Leu Thr Glu Asn Ile
   595                 600                 605                 610
   caa cgc ttt ctc ccc aat cca gct gga gtg cag ctt gag gat cca gag       2049
   Gln Arg Phe Leu Pro Asn Pro Ala Gly Val Gln Leu Glu Asp Pro Glu
                   615                 620                 625
   ttc caa gcc tcc aac atc atg cac agc atc aat ggc tat gtt ttt gat       2097
   Phe Gln Ala Ser Asn Ile Met His Ser Ile Asn Gly Tyr Val Phe Asp
               630                 635                 640
   agt ttg cag ttg tca gtt tgt ttg cat gag gtg gca tac tgg tac att       2145
   Ser Leu Gln Leu Ser Val Cys Leu His Glu Val Ala Tyr Trp Tyr Ile
           645                 650                 655
   cta agc att gga gca cag act gac ttc ctt tct gtc ttc ttc tct gga       2193
   Leu Ser Ile Gly Ala Gln Thr Asp Phe Leu Ser Val Phe Phe Ser Gly
       660                 665                 670
   tat acc ttc aaa cac aaa atg gtc tat gaa gac aca ctc acc cta ttc       2241
   Tyr Thr Phe Lys His Lys Met Val Tyr Glu Asp Thr Leu Thr Leu Phe
   675                 680                 685                 690
   cca ttc tca gga gaa act gtc ttc atg tcg atg gaa aac cca ggt cta       2289
   Pro Phe Ser Gly Glu Thr Val Phe Met Ser Met Glu Asn Pro Gly Leu
                   695                 700                 705
   tgg att ctg ggg tgc cac aac tca gac ttt cgg aac aga ggc atg acc       2337
   Trp Ile Leu Gly Cys His Asn Ser Asp Phe Arg Asn Arg Gly Met Thr
               710                 715                 720
   gcc tta ctg aag gtt tct agt tgt gac aag aac act ggt gat tat tac       2385
   Ala Leu Leu Lys Val Ser Ser Cys Asp Lys Asn Thr Gly Asp Tyr Tyr
           725                 730                 735
   gag gac agt tat gaa gat att tca gca tac ttg ctg agt aaa aac aat       2433
   Glu Asp Ser Tyr Glu Asp Ile Ser Ala Tyr Leu Leu Ser Lys Asn Asn
       740                 745                 750
   gcc att gaa cca aga agc ttc tcc cag aat tca aga cac cct agc act       2481
   Ala Ile Glu Pro Arg Ser Phe Ser Gln Asn Ser Arg His Pro Ser Thr
   755                 760                 765                 770
   agg caa aag caa ttt aat gcc acc aca att cca gaa aat gac ata gag       2529
   Arg Gln Lys Gln Phe Asn Ala Thr Thr Ile Pro Glu Asn Asp Ile Glu
                   775                 780                 785
   aag act gac cct tgg ttt gca cac aga aca cct atg cct aaa ata caa       2577
   Lys Thr Asp Pro Trp Phe Ala His Arg Thr Pro Met Pro Lys Ile Gln
               790                 795                 800
   aat gtc tcc tct agt gat ttg ttg atg ctc ttg cga cag agt cct act       2625
   Asn Val Ser Ser Ser Asp Leu Leu Met Leu Leu Arg Gln Ser Pro Thr
           805                 810                 815
   cca cat ggg cta tcc tta tct gat ctc caa gaa gcc aaa tat gag act       2673
   Pro His Gly Leu Ser Leu Ser Asp Leu Gln Glu Ala Lys Tyr Glu Thr
       820                 825                 830
   ttt tct gat gat cca tca cct gga gca ata gac agt aat aac agc ctg       2721
   Phe Ser Asp Asp Pro Ser Pro Gly Ala Ile Asp Ser Asn Asn Ser Leu
   835                 840                 845                 850
   tct gaa atg aca cac ttc agg cca cag ctc cat cac agt ggg gac atg       2769
   Ser Glu Met Thr His Phe Arg Pro Gln Leu His His Ser Gly Asp Met
                   855                 860                 865
   gta ttt acc cct gag tca ggc ctc caa tta aga tta aat gag aaa ctg      2817
   Val Phe Thr Pro Glu Ser Gly Leu Gln Leu Arg Leu Asn Glu Lys Leu
               870                 875                 880
   ggg aca act gca gca aca gag ttg aag aaa ctt gat ttc aaa gtt tct      2865
   Gly Thr Thr Ala Ala Thr Glu Leu Lys Lys Leu Asp Phe Lys Val Ser
           885                 890                 895
   agt aca tca aat aat ctg att tca aca att cca tca gac aat ttg gca      2913
   Ser Thr Ser Asn Asn Leu Ile Ser Thr Ile Pro Ser Asp Asn Leu Ala
       900                 905                 910
   gca ggt act gat aat aca agt tcc tta gga ccc cca agt atg cca gtt      2961
   Ala Gly Thr Asp Asn Thr Ser Ser Leu Gly Pro Pro Ser Met Pro Val
   915                 920                 925                 930
   cat tat gat agt caa tta gat acc act cta ttt ggc aaa aag tca tct      3009
   His Tyr Asp Ser Gln Leu Asp Thr Thr Leu Phe Gly Lys Lys Ser Ser
                   935                 940                 945
   ccc ctt act gag tct ggt gga cct ctg agc ttg agt gaa gaa aat aat      3057
   Pro Leu Thr Glu Ser Gly Gly Pro Leu Ser Leu Ser Glu Glu Asn Asn
               950                 955                 960
   gat tca aag ttg tta gaa tca ggt tta atg aat agc caa gaa agt tca      3105
   Asp Ser Lys Leu Leu Glu Ser Gly Leu Met Asn Ser Gln Glu Ser Ser
           965                 970                 975
   tgg gga aaa aat gta tcg tca aca gag agt ggt agg tta ttt aaa ggg      3153
   Trp Gly Lys Asn Val Ser Ser Thr Glu Ser Gly Arg Leu Phe Lys Gly
       980                 985                 990
   aaa aga gct cat gga cct  gct ttg ttg act aaa  gat aat gcc tta        3198
   Lys Arg Ala His Gly Pro  Ala Leu Leu Thr Lys  Asp Asn Ala Leu
   995                 1000                 1005
   ttc  aaa gtt agc atc tct  ttg tta aag aca aac  aaa act tcc aat      3243
   Phe  Lys Val Ser Ile Ser  Leu Leu Lys Thr Asn  Lys Thr Ser Asn
   1010                 1015                 1020
   aat  tca gca act aat aga  aag act cac att gat  ggc cca tca tta      3288
   Asn  Ser Ala Thr Asn Arg  Lys Thr His Ile Asp  Gly Pro Ser Leu
   1025                 1030                 1035
   tta  att gag aat agt cca  tca gtc tgg caa aat  ata tta gaa agt      3333
   Leu  Ile Glu Asn Ser Pro  Ser Val Trp Gln Asn  Ile Leu Glu Ser
   1040                 1045                 1050
   gac  act gag ttt aaa aaa  gtg aca cct ttg att  cat gac aga atg      3378
   Asp  Thr Glu Phe Lys Lys  Val Thr Pro Leu Ile  His Asp Arg Met
   1055                 1060                 1065
   ctt  atg gac aaa aat gct  aca gct ttg agg cta  aat cat atg tca      3423
   Leu  Met Asp Lys Asn Ala  Thr Ala Leu Arg Leu  Asn His Met Ser
   1070                 1075                 1080
   aat  aaa act act tca tca  aaa aac atg gaa atg  gtc caa cag aaa      3468
   Asn  Lys Thr Thr Ser Ser  Lys Asn Met Glu Met  Val Gln Gln Lys
   1085                 1090                 1095
   aaa  gag ggc ccc att cca  cca gat gca caa aat  cca gat atg tcg      3513
   Lys  Glu Gly Pro Ile Pro  Pro Asp Ala Gln Asn  Pro Asp Met Ser
   1100                 1105                 1110
   ttc  ttt aag atg cta ttc  ttg cca gaa tca gca  agg tgg ata caa      3558
   Phe  Phe Lys Met Leu Phe  Leu Pro Glu Ser Ala  Arg Trp Ile Gln
   1115                    1120                    1125
   agg  act cat gga aag aac  tct ctg aac tct ggg  caa ggc ccc agt      3603
   Arg  Thr His Gly Lys Asn  Ser Leu Asn Ser Gly  Gln Gly Pro Ser
   1130                 1135                 1140
   cca  aag caa tta gta tcc  tta gga cca gaa aaa  tct gtg gaa ggt      3648
   Pro  Lys Gln Leu Val Ser  Leu Gly Pro Glu Lys  Ser Val Glu Gly
   1145                 1150                 1155
   cag  aat ttc ttg tct gag  aaa aac aaa gtg gta  gta gga aag ggt      3693
   Gln  Asn Phe Leu Ser Glu  Lys Asn Lys Val Val  Val Gly Lys Gly
   1160                 1165                 1170
   gaa  ttt aca aag gac gta  gga ctc aaa gag atg  gtt ttt cca agc      3738
   Glu  Phe Thr Lys Asp Val  Gly Leu Lys Glu Met  Val Phe Pro Ser
   1175                 1180                 1185
   agc  aga aac cta ttt ctt  act aac ttg gat aat  tta cat gaa aat      3783
   Ser  Arg Asn Leu Phe Leu  Thr Asn Leu Asp Asn  Leu His Glu Asn
   1190                 1195                 1200
   aat  aca cac aat caa gaa  aaa aaa att cag gaa  gaa ata gaa aag      3828
   Asn  Thr His Asn Gln Glu  Lys Lys Ile Gln Glu  Glu Ile Glu Lys
   1205                 1210                 1215
   aag  gaa aca tta atc caa  gag aat gta gtt ttg  cct cag ata cat      3873
   Lys  Glu Thr Leu Ile Gln  Glu Asn Val Val Leu  Pro Gln Ile His
   1220                 1225                 1230
   aca  gtg act ggc act aag  aat ttc atg aag aac  ctt ttc tta ctg      3918
   Thr  Val Thr Gly Thr Lys  Asn Phe Met Lys Asn  Leu Phe Leu Leu
   1235                 1240                 1245
   agc  act agg caa aat gta  gaa ggt tca tat gac  ggg gca tat gct      3963
   Ser  Thr Arg Gln Asn Val  Glu Gly Ser Tyr Asp  Gly Ala Tyr Ala
   1250                 1255                 1260
   cca  gta ctt caa gat ttt  agg tca tta aat gat  tca aca aat aga      4008
   Pro  Val Leu Gln Asp Phe  Arg Ser Leu Asn Asp  Ser Thr Asn Arg
   1265                 1270                 1275
   aca  aag aaa cac aca gct  cat ttc tca aaa aaa  ggg gag gaa gaa      4053
   Thr  Lys Lys His Thr Ala  His Phe Ser Lys Lys  Gly Glu Glu Glu
   1280                 1285                 1290
   aac  ttg gaa ggc ttg gga  aat caa acc aag caa  att gta gag aaa      4098
   Asn  Leu Glu Gly Leu Gly  Asn Gln Thr Lys Gln  Ile Val Glu Lys
   1295                 1300                 1305
   tat  gca tgc acc aca agg  ata tct cct aat aca  agc cag cag aat      4143
   Tyr  Ala Cys Thr Thr Arg  Ile Ser Pro Asn Thr  Ser Gln Gln Asn
   1310                 1315                 1320
   ttt  gtc acg caa cgt agt  aag aga gct ttg aaa  caa ttc aga ctc      4188
   Phe  Val Thr Gln Arg Ser  Lys Arg Ala Leu Lys  Gln Phe Arg Leu
   1325                 1330                 1335
   cca  cta gaa gaa aca gaa  ctt gaa aaa agg ata  att gtg gat gac      4233
   Pro  Leu Glu Glu Thr Glu  Leu Glu Lys Arg Ile  Ile Val Asp Asp
   1340                 1345                 1350
   acc  tca acc cag tgg tcc  aaa aac atg aaa cat  ttg acc ccg agc      4278
   Thr  Ser Thr Gln Trp Ser  Lys Asn Met Lys His  Leu Thr Pro Ser
   1355                 1360                 1365
   acc  ctc aca cag ata gac  tac aat gag aag gag  aaa ggg gcc att      4323
   Thr  Leu Thr Gln Ile Asp  Tyr Asn Glu Lys Glu  Lys Gly Ala Ile
   1370                 1375                 1380
   act  cag tct ccc tta tca  gat tgc ctt acg agg  agt cat agc atc     4368
   Thr  Gln Ser Pro Leu Ser  Asp Cys Leu Thr Arg  Ser His Ser Ile
   1385                 1390                 1395
   cct  caa gca aat aga tct  cca tta ccc att gca  aag gta tca tca     4413
   Pro  Gln Ala Asn Arg Ser  Pro Leu Pro Ile Ala  Lys Val Ser Ser
   1400                 1405                 1410
   ttt  cca tct att aga cct  ata tat ctg acc agg  gtc cta ttc caa     4458
   Phe  Pro Ser Ile Arg Pro  Ile Tyr Leu Thr Arg  Val Leu Phe Gln
   1415                 1420                 1425
   gac  aac tct tct cat ctt  cca gca gca tct tat  aga aag aaa gat     4503
   Asp  Asn Ser Ser His Leu  Pro Ala Ala Ser Tyr  Arg Lys Lys Asp
   1430                 1435                 1440
   tct  ggg gtc caa gaa agc  agt cat ttc tta caa  gga gcc aaa aaa     4548
   Ser  Gly Val Gln Glu Ser  Ser His Phe Leu Gln  Gly Ala Lys Lys
   1445                 1450                 1455
   aat  aac ctt tct tta gcc  att cta acc ttg gag  atg act ggt gat     4593
   Asn  Asn Leu Ser Leu Ala  Ile Leu Thr Leu Glu  Met Thr Gly Asp
   1460                 1465                 1470
   caa  aga gag gtt ggc tcc  ctg ggg aca agt gcc  aca aat tca gtc     4638
   Gln  Arg Glu Val Gly Ser  Leu Gly Thr Ser Ala  Thr Asn Ser Val
   1475                 1480                 1485
   aca  tac aag aaa gtt gag  aac act gtt ctc ccg  aaa cca gac ttg     4683
   Thr  Tyr Lys Lys Val Glu  Asn Thr Val Leu Pro  Lys Pro Asp Leu
   1490                 1495                 1500
   ccc  aaa aca tct ggc aaa  gtt gaa ttg ctt cca  aaa gtt cac att     4728
   Pro  Lys Thr Ser Gly Lys  Val Glu Leu Leu Pro  Lys Val His Ile
   1505                 1510                 1515
   tat  cag aag gac cta ttc  cct acg gaa act agc  aat ggg tct cct     4773
   Tyr  Gln Lys Asp Leu Phe  Pro Thr Glu Thr Ser  Asn Gly Ser Pro
   1520                 1525                 1530
   ggc  cat ctg gat ctc gtg  gaa ggg agc ctt ctt  cag gga aca gag     4818
   Gly  His Leu Asp Leu Val  Glu Gly Ser Leu Leu  Gln Gly Thr Glu
   1535                 1540                 1545
   gga  gcg att aag tgg aat  gaa gca aac aga cct  gga aaa gtt ccc     4863
   Gly  Ala Ile Lys Trp Asn  Glu Ala Asn Arg Pro  Gly Lys Val Pro
   1550                 1555                 1560
   ttt  ctg aga gta gca aca  gaa agc tct gca aag  act ccc tcc aag     4908
   Phe  Leu Arg Val Ala Thr  Glu Ser Ser Ala Lys  Thr Pro Ser Lys
   1565                 1570                 1575
   cta  ttg gat cct ctt gct  tgg gat aac cac tat  ggt act cag ata     4953
   Leu  Leu Asp Pro Leu Ala  Trp Asp Asn His Tyr  Gly Thr Gln Ile
   1580                 1585                 1590
   cca  aaa gaa gag tgg aaa  tcc caa gag aag tca  cca gaa aaa aca     4998
   Pro  Lys Glu Glu Trp Lys  Ser Gln Glu Lys Ser  Pro Glu Lys Thr
   1595                 1600                 1605
   gct  ttt aag aaa aag gat  acc att ttg tcc ctg  aac gct tgt gaa     5043
   Ala  Phe Lys Lys Lys Asp  Thr Ile Leu Ser Leu  Asn Ala Cys Glu
   1610                 1615                 1620
   agc  aat cat gca ata gca  gca ata aat gag gga  caa aat aag ccc     5088
   Ser  Asn His Ala Ile Ala  Ala Ile Asn Glu Gly  Gln Asn Lys Pro
   1625                 1630                 1635
   gaa  ata gaa gtc acc tgg  gca aag caa ggt agg  act gaa agg ctg     5133
   Glu  Ile Glu Val Thr Trp  Ala Lys Gln Gly Arg  Thr Glu Arg Leu
   1640                 1645                 1650
   tgc  tct caa aac cca cca  gtc ttg aaa cgc cat  caa cgg gaa ata     5178
   Cys  Ser Gln Asn Pro Pro  Val Leu Lys Arg His  Gln Arg Glu Ile
   1655                 1660                 1665
   act  cgt act act ctt cag  tca gat caa gag gaa  att gac tat gat     5223
   Thr  Arg Thr Thr Leu Gln  Ser Asp Gln Glu Glu  Ile Asp Tyr Asp
   1670                 1675                 1680
   gat  acc ata tca gtt gaa  atg aag aag gaa gat  ttt gac att tat     5268
   Asp  Thr Ile Ser Val Glu  Met Lys Lys Glu Asp  Phe Asp Ile Tyr
   1685                 1690                 1695
   gat  gag gat gaa aat cag  agc ccc cgc agc ttt  caa aag aaa aca     5313
   Asp  Glu Asp Glu Asn Gln  Ser Pro Arg Ser Phe  Gln Lys Lys Thr
   1700                 1705                 1710
   cga  cac tat ttt att gct  gca gtg gag agg ctc  tgg gat tat ggg     5358
   Arg  His Tyr Phe Ile Ala  Ala Val Glu Arg Leu  Trp Asp Tyr Gly
   1715                 1720                 1725
   atg  agt agc tcc cca cat  gtt cta aga aac agg  gct cag agt ggc     5403
   Met  Ser Ser Ser Pro His  Val Leu Arg Asn Arg  Ala Gln Ser Gly
   1730                 1735                 1740
   agt  gtc cct cag ttc aag  aaa gtt gtt ttc cag  gaa ttt act gat     5448
   Ser  Val Pro Gln Phe Lys  Lys Val Val Phe Gln  Glu Phe Thr Asp
   1745                 1750                 1755
   ggc  tcc ttt act cag ccc  tta tac cgt gga gaa  cta aat gaa cat     5493
   Gly  Ser Phe Thr Gln Pro  Leu Tyr Arg Gly Glu  Leu Asn Glu His
   1760                 1765                 1770
   ttg  gga ctc ctg ggg cca  tat ata aga gca gaa  gtt gaa gat aat     5538
   Leu  Gly Leu Leu Gly Pro  Tyr Ile Arg Ala Glu  Val Glu Asp Asn
   1775                 1780                 1785
   atc  atg gta act ttc aga  aat cag gcc tct cgt  ccc tat tcc ttc     5583
   Ile  Met Val Thr Phe Arg  Asn Gln Ala Ser Arg  Pro Tyr Ser Phe
   1790                 1795                 1800
   tat  tct agc ctt att tct  tat gag gaa gat cag  agg caa gga gca     5628
   Tyr  Ser Ser Leu Ile Ser  Tyr Glu Glu Asp Gln  Arg Gln Gly Ala
   1805                 1810                 1815
   gaa  cct aga aaa aac ttt  gtc aag cct aat gaa  acc aaa act tac     5673
   Glu  Pro Arg Lys Asn Phe  Val Lys Pro Asn Glu  Thr Lys Thr Tyr
   1820                 1825                 1830
   ttt  tgg aaa gtg caa cat  cat atg gca ccc act  aaa gat gag ttt     5718
   Phe  Trp Lys Val Gln His  His Met Ala Pro Thr  Lys Asp Glu Phe
   1835                 1840                 1845
   gac  tgc aaa gcc tgg gct  tat ttc tct gat gtt  gac ctg gaa aaa     5763
   Asp  Cys Lys Ala Trp Ala  Tyr Phe Ser Asp Val  Asp Leu Glu Lys
   1850                 1855                 1860
   gat  gtg cac tca ggc ctg  att gga ccc ctt ctg  gtc tgc cac act     5808
   Asp  Val His Ser Gly Leu  Ile Gly Pro Leu Leu  Val Cys His Thr
   1865                 1870                 1875
   aac  aca ctg aac cct gct  cat ggg aga caa gtg  aca gta cag gaa     5853
   Asn  Thr Leu Asn Pro Ala  His Gly Arg Gln Val  Thr Val Gln Glu
   1880                 1885                 1890
   ttt  gct ctg ttt ttc acc  atc ttt gat gag acc  aaa agc tgg tac     5898
   Phe Ala Leu Phe Phe Thr Ile Phe Asp Glu Thr Lys Ser Trp Tyr
   1895 1900 1905
   ttc act gaa aat atg gaa aga aac tgc agg gct ccc tgc aat atc 5943
   Phe Thr Glu Asn Met Glu Arg Asn Cys Arg Ala Pro Cys Asn Ile
   1910 1915 1920
   cag atg gaa gat ccc act ttt aaa gag aat tat cgc ttc cat gca 5988
   Gln Met Glu Asp Pro Thr Phe Lys Glu Asn Tyr Arg Phe His Ala
   1925 1930 1935
   atc aat ggc tac ata atg gat aca cta cct ggc tta gta atg gct 6033
   Ile Asn Gly Tyr Ile Met Asp Thr Leu Pro Gly Leu Val Met Ala
   1940 1945 1950
   cag gat caa agg att cga tgg tat ctg ctc agc atg ggc agc aat 6078
   Gln Asp Gln Arg Ile Arg Trp Tyr Leu Leu Ser Met Gly Ser Asn
   1955 1960 1965
   gaa aac atc cat tct att cat ttc agt gga cat gtg ttc act gta 6123
   Glu Asn Ile His Ser Ile His Phe Ser Gly His Val Phe Thr Val
   1970 1975 1980
   cga aaa aaa gag gag tat aaa atg gca ctg tac aat ctc tat cca 6168
   Arg Lys Lys Glu Glu Tyr Lys Met Ala Leu Tyr Asn Leu Tyr Pro
   1985 1990 1995
   ggt gtt ttt gag aca gtg gaa atg tta cca tcc aaa gct gga att 6213
   Gly Val Phe Glu Thr Val Glu Met Leu Pro Ser Lys Ala Gly Ile
   2000 2005 2010
   tgg cgg gtg gaa tgc ctt att ggc gag cat cta cat gct ggg atg 6258
   Trp Arg Val Glu Cys Leu Ile Gly Glu His Leu His Ala Gly Met
   2015 2020 2025
   agc aca ctt ttt ctg gtg tac agc aat aag tgt cag act ccc ctg 6303
   Ser Thr Leu Phe Leu Val Tyr Ser Asn Lys Cys Gln Thr Pro Leu
   2030 2035 2040
   gga atg gct tct gga cac att aga gat ttt cag att aca gct tca 6348
   Gly Met Ala Ser Gly His Ile Arg Asp Phe Gln Ile Thr Ala Ser
   2045 2050 2055
   gga caa tat gga cag tgg gcc cca aag ctg gcc aga ctt cat tat 6393
   Gly Gln Tyr Gly Gln Trp Ala Pro Lys Leu Ala Arg Leu His Tyr
   2060 2065 2070
   tcc gga tca atc aat gcc tgg agc acc aag gag ccc ttt tct tgg 6438
   Ser Gly Ser Ile Asn Ala Trp Ser Thr Lys Glu Pro Phe Ser Trp
   2075 2080 2085
   atc aag gtg gat ctg ttg gca cca atg att att cac ggc atc aag 6483
   Ile Lys Val Asp Leu Leu Ala Pro Met Ile Ile His Gly Ile Lys
   2090 2095 2100
   acc cag ggt gcc cgt cag aag ttc tcc agc ctc tac atc tct cag 6528
   Thr Gln Gly Ala Arg Gln Lys Phe Ser Ser Leu Tyr Ile Ser Gln
   2105 2110 2115
   ttt atc atc atg tat agt ctt gat ggg aag aag tgg cag act tat 6573
   Phe Ile Ile Met Tyr Ser Leu Asp Gly Lys Lys Trp Gln Thr Tyr
   2120 2125 2130
   cga gga aat tcc act gga acc tta atg gtc ttc ttt ggc aat gtg 6618
   Arg Gly Asn Ser Thr Gly Thr Leu Met Val Phe Phe Gly Asn Val
   2135 2140 2145
   gat tca tct ggg ata aaa cac aat att ttt aac cct cca att att 6663
   Asp Ser Ser Gly Ile Lys His Asn Ile Phe Asn Pro Pro Ile Ile
   2150 2155 2160
   gct cga tac atc cgt ttg cac cca act cat tat agc att cgc agc 6708
   Ala Arg Tyr Ile Arg Leu His Pro Thr His Tyr Ser Ile Arg Ser
   2165 2170 2175
   act ctt cgc atg gag ttg atg ggc tgt gat tta aat agt tgc agc 6753
   Thr Leu Arg Met Glu Leu Met Gly Cys Asp Leu Asn Ser Cys Ser
   2180 2185 2190
   atg cca ttg gga atg gag agt aaa gca ata tca gat gca cag att 6798
   Met Pro Leu Gly Met Glu Ser Lys Ala Ile Ser Asp Ala Gln Ile
   2195 2200 2205
   act gct tca tcc tac ttt acc aat atg ttt gcc acc tgg tct cct 6843
   Thr Ala Ser Ser Tyr Phe Thr Asn Met Phe Ala Thr Trp Ser Pro
   2210 2215 2220
   tca aaa gct cga ctt cac ctc caa ggg agg agt aat gcc tgg aga 6888
   Ser Lys Ala Arg Leu His Leu Gln Gly Arg Ser Asn Ala Trp Arg
   2225 2230 2235
   cct cag gtg aat aat cca aaa gag tgg ctg caa gtg gac ttc cag 6933
   Pro Gln Val Asn Asn Pro Lys Glu Trp Leu Gln Val Asp Phe Gln
   2240 2245 2250
   aag aca atg aaa gtc aca gga gta act act cag gga gta aaa tct 6978
   Lys Thr Met Lys Val Thr Gly Val Thr Thr Gln Gly Val Lys Ser
   2255 2260 2265
   ctg ctt acc agc atg tat gtg aag gag ttc ctc atc tcc agc agt 7023
   Leu Leu Thr Ser Met Tyr Val Lys Glu Phe Leu Ile Ser Ser Ser
   2270 2275 2280
   caa gat ggc cat cag tgg act ctc ttt ttt cag aat ggc aaa gta 7068
   Gln Asp Gly His Gln Trp Thr Leu Phe Phe Gln Asn Gly Lys Val
   2285 2290 2295
   aag gtt ttt cag gga aat caa gac tcc ttc aca cct gtg gtg aac 7113
   Lys Val Phe Gln Gly Asn Gln Asp Ser Phe Thr Pro Val Val Asn
   2300 2305 2310
   tct cta gac cca ccg tta ctg act cgc tac ctt cga att cac ccc 7158
   Ser Leu Asp Pro Pro Leu Leu Thr Arg Tyr Leu Arg Ile His Pro
   2315 2320 2325
   cag agt tgg gtg cac cag att gcc ctg agg atg gag gtt ctg ggc 7203
   Gln Ser Trp Val His Gln Ile Ala Leu Arg Met Glu Val Leu Gly
   2330 2335 2340
   tgc gag gca cag gac ctc tac tga gggtggccac tgcagcacct 7247
   Cys Glu Ala Gln Asp Leu Tyr
   2345 2350
   gccactgccg tcacctctcc ctcctcagct ccagggcagt gtccctccct ggcttgcctt 7307
   ctacctttgt gctaaatcct agcagacact gccttgaagc ctcctgaatt aactatcatc 7367
   agtcctgcat ttctttggtg gggggccagg agggtgcatc caatttaact taactcttac 7427
   ctattttctg cagctgctcc cagattactc cttccttcca atataactag gcaaaaagaa 7487
   gtgaggagaa acctgcatga aagcattctt ccctgaaaag ttaggcctct cagagtcacc 7547
   acttcctctg ttgtagaaaa actatgtgat gaaactttga aaaagatatt tatgatgtta 7607
   acatttcagg ttaagcctca tacgtttaaa ataaaactct cagttgttta ttatcctgat 7667
   caagcatgga acaaagcatg tttcaggatc agatcaatac aatcttggag tcaaaaggca 7727
   aatcatttgg acaatctgca aaatggagag aatacaataa ctactacagt aaagtctgtt    7787
   tctgcttcct tacacataga tataattatg ttatttagtc attatgaggg gcacattctt    7847
   atctccaaaa ctagcattct taaactgaga attatagatg gggttcaaga atccctaagt    7907
   cccctgaaat tatataaggc attctgtata aatgcaaatg tgcatttttc tgacgagtgt    7967
   ccatagatat aaagccattt ggtcttaatt ctgaccaata aaaaaataag tcaggaggat    8027
   gcaattgttg aaagctttga aataaaataa caatgtcttc ttgaaatttg tgatggccaa    8087
   gaaagaaaat gatgatgaca ttaggcttct aaaggacata catttaatat ttctgtggaa    8147
   atatgaggaa aatccatggt tatctgagat aggagataca aactttgtaa ttctaataat    8207
   gcactcagtt tactctctcc ctctactaat ttcctgctga aaataacaca acaaaaatgt    8267
   aacaggggaa attatatacc gtgactgaaa actagagtcc tacttacata gttgaaatat    8327
   caaggaggtc agaagaaaat tggactggtg aaaacagaaa aaacactcca gtctgccata    8387
   tcaccacaca ataggatccc ccttcttgcc ctccaccccc ataagattgt gaagggttta    8447
   ctgctccttc catctgcctg accccttcac tatgactaca cagaatctcc tgatagtaaa    8507
   gggggctgga ggcaaggata agttatagag cagttggagg aagcatccaa agattgcaac    8567
   ccagggcaaa tggaaaacag gagatcctaa tatgaaagaa aaatggatcc caatctgaga    8627
   aaaggcaaaa gaatggctac ttttttctat gctggagtat tttctaataa tcctgcttga    8687
   cccttatctg acctctttgg aaactataac atagctgtca cagtatagtc acaatccaca    8747
   aatgatgcag gtgcaaatgg tttatagccc tgtgaagttc ttaaagttta gaggctaact    8807
   tacagaaatg aataagttgt tttgttttat agcccggtag aggagttaac cccaaaggtg    8867
   atatggtttt atttcctgtt atgtttaact tgataatctt attttggcat tcttttccca    8927
   ttgactatat acatctctat ttctcaaatg ttcatggaac tagctctttt attttcctgc    8987
   tggtttcttc agtaatgagt taaataaaac attgacacat a                        9028
<210> 2
   <211> 2351
   <212> PRT
   <213> Homo sapiens
<400> 2
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
   50 55 60
   Tyr Lys Lys Thr Leu Phe Val Glu Phe Thr Asp His Leu Phe Asn Ile
   65 70 75 80
   Ala Lys Pro Arg Pro Pro Trp Met Gly Leu Leu Gly Pro Thr Ile Gln
   85 90 95
   Ala Glu Val Tyr Asp Thr Val Val Ile Thr Leu Lys Asn Met Ala Ser
   100 105 110
   His Pro Val Ser Leu His Ala Val Gly Val Ser Tyr Trp Lys Ala Ser
   115 120 125
   Glu Gly Ala Glu Tyr Asp Asp Gln Thr Ser Gln Arg Glu Lys Glu Asp
   130 135 140
   Asp Lys Val Phe Pro Gly Gly Ser His Thr Tyr Val Trp Gln Val Leu
   145 150 155 160
   Lys Glu Asn Gly Pro Met Ala Ser Asp Pro Leu Cys Leu Thr Tyr Ser
   165 170 175
   Tyr Leu Ser His Val Asp Leu Val Lys Asp Leu Asn Ser Gly Leu Ile
   180 185 190
   Gly Ala Leu Leu Val Cys Arg Glu Gly Ser Leu Ala Lys Glu Lys Thr
   195 200 205
   Gln Thr Leu His Lys Phe Ile Leu Leu Phe Ala Val Phe Asp Glu Gly
   210 215 220
   Lys Ser Trp His Ser Glu Thr Lys Asn Ser Leu Met Gln Asp Arg Asp
   225 230 235 240
   Ala Ala Ser Ala Arg Ala Trp Pro Lys Met His Thr Val Asn Gly Tyr
   245 250 255
   Val Asn Arg Ser Leu Pro Gly Leu Ile Gly Cys His Arg Lys Ser Val
   260 265 270
   Tyr Trp His Val Ile Gly Met Gly Thr Thr Pro Glu Val His Ser Ile
   275 280 285
   Phe Leu Glu Gly His Thr Phe Leu Val Arg Asn His Arg Gln Ala Ser
   290 295 300
   Leu Glu Ile Ser Pro Ile Thr Phe Leu Thr Ala Gln Thr Leu Leu Met
   305 310 315 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
               595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Asn Ser Arg His Pro
           755                 760                 765
   Ser Thr Arg Gln Lys Gln Phe Asn Ala Thr Thr Ile Pro Glu Asn Asp
       770                 775                 780
   Ile Glu Lys Thr Asp Pro Trp Phe Ala His Arg Thr Pro Met Pro Lys
   785                 790                 795                 800
   Ile Gln Asn Val Ser Ser Ser Asp Leu Leu Met Leu Leu Arg Gln Ser
                   805                 810                 815
   Pro Thr Pro His Gly Leu Ser Leu Ser Asp Leu Gln Glu Ala Lys Tyr
               820                 825                 830
   Glu Thr Phe Ser Asp Asp Pro Ser Pro Gly Ala Ile Asp Ser Asn Asn
           835                 840                 845
   Ser Leu Ser Glu Met Thr His Phe Arg Pro Gln Leu His His Ser Gly
       850                 855                 860
   Asp Met Val Phe Thr Pro Glu Ser Gly Leu Gln Leu Arg Leu Asn Glu
   865                 870                 875                 880
   Lys Leu Gly Thr Thr Ala Ala Thr Glu Leu Lys Lys Leu Asp Phe Lys
   885 890 895
   Val Ser Ser Thr Ser Asn Asn Leu Ile Ser Thr Ile Pro Ser Asp Asn
   900 905 910
   Leu Ala Ala Gly Thr Asp Asn Thr Ser Ser Leu Gly Pro Pro Ser Met
   915 920 925
   Pro Val His Tyr Asp Ser Gln Leu Asp Thr Thr Leu Phe Gly Lys Lys
   930 935 940
   Ser Ser Pro Leu Thr Glu Ser Gly Gly Pro Leu Ser Leu Ser Glu Glu
   945 950 955 960
   Asn Asn Asp Ser Lys Leu Leu Glu Ser Gly Leu Met Asn Ser Gln Glu
   965 970 975
   Ser Ser Trp Gly Lys Asn Val Ser Ser Thr Glu Ser Gly Arg Leu Phe
   980 985 990
   Lys Gly Lys Arg Ala His Gly Pro Ala Leu Leu Thr Lys Asp Asn Ala
   995 1000 1005
   Leu Phe Lys Val Ser Ile Ser Leu Leu Lys Thr Asn Lys Thr Ser
   1010 1015 1020
   Asn Asn Ser Ala Thr Asn Arg Lys Thr His Ile Asp Gly Pro Ser
   1025 1030 1035
   Leu Leu Ile Glu Asn Ser Pro Ser Val Trp Gln Asn Ile Leu Glu
   1040 1045 1050
   Ser Asp Thr Glu Phe Lys Lys Val Thr Pro Leu Ile His Asp Arg
   1055 1060 1065
   Met Leu Met Asp Lys Asn Ala Thr Ala Leu Arg Leu Asn His Met
   1070 1075 1080
   Ser Asn Lys Thr Thr Ser Ser Lys Asn Met Glu Met Val Gln Gln
   1085 1090 1095
   Lys Lys Glu Gly Pro Ile Pro Pro Asp Ala Gln Asn Pro Asp Met
   1100 1105 1110
   Ser Phe Phe Lys Met Leu Phe Leu Pro Glu Ser Ala Arg Trp Ile
   1115 1120 1125
   Gln Arg Thr His Gly Lys Asn Ser Leu Asn Ser Gly Gln Gly Pro
   1130 1135 1140
   Ser Pro Lys Gln Leu Val Ser Leu Gly Pro Glu Lys Ser Val Glu
   1145 1150 1155
   Gly Gln Asn Phe Leu Ser Glu Lys Asn Lys Val Val Val Gly Lys
   1160 1165 1170
   Gly Glu Phe Thr Lys Asp Val Gly Leu Lys Glu Met Val Phe Pro
   1175 1180 1185
   Ser Ser Arg Asn Leu Phe Leu Thr Asn Leu Asp Asn Leu His Glu
   1190 1195 1200
   Asn Asn Thr His Asn Gln Glu Lys Lys Ile Gln Glu Glu Ile Glu
   1205 1210 1215
   Lys Lys Glu Thr Leu Ile Gln Glu Asn Val Val Leu Pro Gln Ile
   1220 1225 1230
   His Thr Val Thr Gly Thr Lys Asn Phe Met Lys Asn Leu Phe Leu
   1235 1240 1245
   Leu Ser Thr Arg Gln Asn Val Glu Gly Ser Tyr Asp Gly Ala Tyr
   1250 1255 1260
   Ala Pro Val Leu Gln Asp Phe Arg Ser Leu Asn Asp Ser Thr Asn
   1265 1270 1275
   Arg Thr Lys Lys His Thr Ala His Phe Ser Lys Lys Gly Glu Glu
   1280 1285 1290
   Glu Asn Leu Glu Gly Leu Gly Asn Gln Thr Lys Gln Ile Val Glu
   1295 1300 1305
   Lys Tyr Ala Cys Thr Thr Arg Ile Ser Pro Asn Thr Ser Gln Gln
   1310 1315 1320
   Asn Phe Val Thr Gln Arg Ser Lys Arg Ala Leu Lys Gln Phe Arg
   1325 1330 1335
   Leu Pro Leu Glu Glu Thr Glu Leu Glu Lys Arg Ile Ile Val Asp
   1340 1345 1350
   Asp Thr Ser Thr Gln Trp Ser Lys Asn Met Lys His Leu Thr Pro
   1355 1360 1365
   Ser Thr Leu Thr Gln Ile Asp Tyr Asn Glu Lys Glu Lys Gly Ala
   1370 1375 1380
   Ile Thr Gln Ser Pro Leu Ser Asp Cys Leu Thr Arg Ser His Ser
   1385 1390 1395
   Ile Pro  Gln Ala Asn Arg Ser  Pro Leu Pro Ile Ala  Lys Val Ser
       1400                 1405                 1410
   Ser Phe  Pro Ser Ile Arg Pro  Ile Tyr Leu Thr Arg  Val Leu Phe
       1415                 1420                 1425
   Gln Asp  Asn Ser Ser His Leu  Pro Ala Ala Ser Tyr  Arg Lys Lys
       1430                 1435                 1440
   Asp Ser  Gly Val Gln Glu Ser  Ser His Phe Leu Gln  Gly Ala Lys
       1445                 1450                 1455
   Lys Asn  Asn Leu Ser Leu Ala  Ile Leu Thr Leu Glu  Met Thr Gly
       1460                 1465                 1470
   Asp Gln  Arg Glu Val Gly Ser  Leu Gly Thr Ser Ala  Thr Asn Ser
       1475                 1480                 1485
   Val Thr  Tyr Lys Lys Val Glu  Asn Thr Val Leu Pro  Lys Pro Asp
       1490                 1495                 1500
   Leu Pro  Lys Thr Ser Gly Lys  Val Glu Leu Leu Pro  Lys Val His
       1505                 1510                 1515
   Ile Tyr  Gln Lys Asp Leu Phe  Pro Thr Glu Thr Ser  Asn Gly Ser
       1520                 1525                 1530
   Pro Gly  His Leu Asp Leu Val  Glu Gly Ser Leu Leu  Gln Gly Thr
       1535                 1540                 1545
   Glu Gly  Ala Ile Lys Trp Asn  Glu Ala Asn Arg Pro  Gly Lys Val
       1550                 1555                 1560
   Pro Phe  Leu Arg Val Ala Thr  Glu Ser Ser Ala Lys  Thr Pro Ser
       1565                 1570                 1575
   Lys Leu  Leu Asp Pro Leu Ala  Trp Asp Asn His Tyr  Gly Thr Gln
       1580                 1585                 1590
   Ile Pro  Lys Glu Glu Trp Lys  Ser Gln Glu Lys Ser  Pro Glu Lys
       1595                 1600                 1605
   Thr Ala  Phe Lys Lys Lys Asp  Thr Ile Leu Ser Leu  Asn Ala Cys
       1610                 1615                 1620
   Glu Ser  Asn His Ala Ile Ala  Ala Ile Asn Glu Gly  Gln Asn Lys
       1625                 1630                 1635
   Pro Glu  Ile Glu Val Thr Trp  Ala Lys Gln Gly Arg  Thr Glu Arg
       1640                 1645                 1650
   Leu Cys  Ser Gln Asn Pro Pro  Val Leu Lys Arg His  Gln Arg Glu
   1655 1660 1665
   Ile Thr Arg Thr Thr Leu Gln Ser Asp Gln Glu Glu Ile Asp Tyr
   1670 1675 1680
   Asp Asp Thr Ile Ser Val Glu Met Lys Lys Glu Asp Phe Asp Ile
   1685 1690 1695
   Tyr Asp Glu Asp Glu Asn Gln Ser Pro Arg Ser Phe Gln Lys Lys
   1700 1705 1710
   Thr Arg His Tyr Phe Ile Ala Ala Val Glu Arg Leu Trp Asp Tyr
   1715 1720 1725
   Gly Met Ser Ser Ser Pro His Val Leu Arg Asn Arg Ala Gln Ser
   1730 1735 1740
   Gly Ser Val Pro Gln Phe Lys Lys Val Val Phe Gln Glu Phe Thr
   1745 1750 1755
   Asp Gly Ser Phe Thr Gln Pro Leu Tyr Arg Gly Glu Leu Asn Glu
   1760 1765 1770
   His Leu Gly Leu Leu Gly Pro Tyr Ile Arg Ala Glu Val Glu Asp
   1775 1780 1785
   Asn Ile Met Val Thr Phe Arg Asn Gln Ala Ser Arg Pro Tyr Ser
   1790 1795 1800
   Phe Tyr Ser Ser Leu Ile Ser Tyr Glu Glu Asp Gln Arg Gln Gly
   1805 1810 1815
   Ala Glu Pro Arg Lys Asn Phe Val Lys Pro Asn Glu Thr Lys Thr
   1820 1825 1830
   Tyr Phe Trp Lys Val Gln His His Met Ala Pro Thr Lys Asp Glu
   1835 1840 1845
   Phe Asp Cys Lys Ala Trp Ala Tyr Phe Ser Asp Val Asp Leu Glu
   1850 1855 1860
   Lys Asp Val His Ser Gly Leu Ile Gly Pro Leu Leu Val Cys His
   1865 1870 1875
   Thr Asn Thr Leu Asn Pro Ala His Gly Arg Gln Val Thr Val Gln
   1880 1885 1890
   Glu Phe Ala Leu Phe Phe Thr Ile Phe Asp Glu Thr Lys Ser Trp
   1895 1900 1905
   Tyr Phe Thr Glu Asn Met Glu Arg Asn Cys Arg Ala Pro Cys Asn
   1910 1915 1920
   Ile Gln Met Glu Asp Pro Thr Phe Lys Glu Asn Tyr Arg Phe His
   1925 1930 1935
   Ala Ile Asn Gly Tyr Ile Met Asp Thr Leu Pro Gly Leu Val Met
   1940 1945 1950
   Ala Gln Asp Gln Arg Ile Arg Trp Tyr Leu Leu Ser Met Gly Ser
   1955 1960 1965
   Asn Glu Asn Ile His Ser Ile His Phe Ser Gly His Val Phe Thr
   1970 1975 1980
   Val Arg Lys Lys Glu Glu Tyr Lys Met Ala Leu Tyr Asn Leu Tyr
   1985 1990 1995
   Pro Gly Val Phe Glu Thr Val Glu Met Leu Pro Ser Lys Ala Gly
   2000 2005 2010
   Ile Trp Arg Val Glu Cys Leu Ile Gly Glu His Leu His Ala Gly
   2015 2020 2025
   Met Ser Thr Leu Phe Leu Val Tyr Ser Asn Lys Cys Gln Thr Pro
   2030 2035 2040
   Leu Gly Met Ala Ser Gly His Ile Arg Asp Phe Gln Ile Thr Ala
   2045 2050 2055
   Ser Gly Gln Tyr Gly Gln Trp Ala Pro Lys Leu Ala Arg Leu His
   2060 2065 2070
   Tyr Ser Gly Ser Ile Asn Ala Trp Ser Thr Lys Glu Pro Phe Ser
   2075 2080 2085
   Trp Ile Lys Val Asp Leu Leu Ala Pro Met Ile Ile His Gly Ile
   2090 2095 2100
   Lys Thr Gln Gly Ala Arg Gln Lys Phe Ser Ser Leu Tyr Ile Ser
   2105 2110 2115
   Gln Phe Ile Ile Met Tyr Ser Leu Asp Gly Lys Lys Trp Gln Thr
   2120 2125 2130
   Tyr Arg Gly Asn Ser Thr Gly Thr Leu Met Val Phe Phe Gly Asn
   2135 2140 2145
   Val Asp Ser Ser Gly Ile Lys His Asn Ile Phe Asn Pro Pro Ile
   2150 2155 2160
   Ile Ala Arg Tyr Ile Arg Leu His Pro Thr His Tyr Ser Ile Arg
   2165 2170 2175
   Ser Thr Leu Arg Met Glu Leu Met Gly Cys Asp Leu Asn Ser Cys
   2180 2185 2190
   Ser Met Pro Leu Gly Met Glu Ser Lys Ala Ile Ser Asp Ala Gln
   2195 2200 2205
   Ile Thr Ala Ser Ser Tyr Phe Thr Asn Met Phe Ala Thr Trp Ser
   2210 2215 2220
   Pro Ser Lys Ala Arg Leu His Leu Gln Gly Arg Ser Asn Ala Trp
   2225 2230 2235
   Arg Pro Gln Val Asn Asn Pro Lys Glu Trp Leu Gln Val Asp Phe
   2240 2245 2250
   Gln Lys Thr Met Lys Val Thr Gly Val Thr Thr Gln Gly Val Lys
   2255 2260 2265
   Ser Leu Leu Thr Ser Met Tyr Val Lys Glu Phe Leu Ile Ser Ser
   2270 2275 2280
   Ser Gln Asp Gly His Gln Trp Thr Leu Phe Phe Gln Asn Gly Lys
   2285 2290 2295
   Val Lys Val Phe Gln Gly Asn Gln Asp Ser Phe Thr Pro Val Val
   2300 2305 2310
   Asn Ser Leu Asp Pro Pro Leu Leu Thr Arg Tyr Leu Arg Ile His
   2315 2320 2325
   Pro Gln Ser Trp Val His Gln Ile Ala Leu Arg Met Glu Val Leu
   2330 2335 2340
   Gly Cys Glu Ala Gln Asp Leu Tyr
   2345 2350
<210> 3
   <211> 2332
   <212> PRT
   <213> Homo sapiens
<400> 3
   Ala Thr Arg Arg Tyr Tyr Leu Gly Ala Val Glu Leu Ser Trp Asp Tyr
   1 5 10 15
   Met Gln Ser Asp Leu Gly Glu Leu Pro Val Asp Ala Arg Phe Pro Pro
   20 25 30
   Arg Val Pro Lys Ser Phe Pro Phe Asn Thr Ser Val Val Tyr Lys Lys
   35 40 45
   Thr Leu Phe Val Glu Phe Thr Asp His Leu Phe Asn Ile Ala Lys Pro
   50 55 60
   Arg Pro Pro Trp Met Gly Leu Leu Gly Pro Thr Ile Gln Ala Glu Val
   65 70 75 80
   Tyr Asp Thr Val Val Ile Thr Leu Lys Asn Met Ala Ser His Pro Val
   85 90 95
   Ser Leu His Ala Val Gly Val Ser Tyr Trp Lys Ala Ser Glu Gly Ala
   100 105 110
   Glu Tyr Asp Asp Gln Thr Ser Gln Arg Glu Lys Glu Asp Asp Lys Val
   115 120 125
   Phe Pro Gly Gly Ser His Thr Tyr Val Trp Gln Val Leu Lys Glu Asn
   130 135 140
   Gly Pro Met Ala Ser Asp Pro Leu Cys Leu Thr Tyr Ser Tyr Leu Ser
   145 150 155 160
   His Val Asp Leu Val Lys Asp Leu Asn Ser Gly Leu Ile Gly Ala Leu
   165 170 175
   Leu Val Cys Arg Glu Gly Ser Leu Ala Lys Glu Lys Thr Gln Thr Leu
   180 185 190
   His Lys Phe Ile Leu Leu Phe Ala Val Phe Asp Glu Gly Lys Ser Trp
   195 200 205
   His Ser Glu Thr Lys Asn Ser Leu Met Gln Asp Arg Asp Ala Ala Ser
   210 215 220
   Ala Arg Ala Trp Pro Lys Met His Thr Val Asn Gly Tyr Val Asn Arg
   225 230 235 240
   Ser Leu Pro Gly Leu Ile Gly Cys His Arg Lys Ser Val Tyr Trp His
   245 250 255
   Val Ile Gly Met Gly Thr Thr Pro Glu Val His Ser Ile Phe Leu Glu
   260 265 270
   Gly His Thr Phe Leu Val Arg Asn His Arg Gln Ala Ser Leu Glu Ile
   275 280 285
   Ser Pro Ile Thr Phe Leu Thr Ala Gln Thr Leu Leu Met Asp Leu Gly
   290 295 300
   Gln Phe Leu Leu Phe Cys His Ile Ser Ser His Gln His Asp Gly Met
   305 310 315 320
   Glu Ala Tyr Val Lys Val Asp Ser Cys Pro Glu Glu Pro Gln Leu Arg
   325 330 335
   Met Lys Asn Asn Glu Glu Ala Glu Asp Tyr Asp Asp Asp Leu Thr Asp
   340 345 350
   Ser Glu Met Asp Val Val Arg Phe Asp Asp Asp Asn Ser Pro Ser Phe
   355 360 365
   Ile Gln Ile Arg Ser Val Ala Lys Lys His Pro Lys Thr Trp Val His
   370 375 380
   Tyr Ile Ala Ala Glu Glu Glu Asp Trp Asp Tyr Ala Pro Leu Val Leu
   385 390 395 400
   Ala Pro Asp Asp Arg Ser Tyr Lys Ser Gln Tyr Leu Asn Asn Gly Pro
   405 410 415
   Gln Arg Ile Gly Arg Lys Tyr Lys Lys Val Arg Phe Met Ala Tyr Thr
   420 425 430
   Asp Glu Thr Phe Lys Thr Arg Glu Ala Ile Gln His Glu Ser Gly Ile
   435 440 445
   Leu Gly Pro Leu Leu Tyr Gly Glu Val Gly Asp Thr Leu Leu Ile Ile
   450 455 460
   Phe Lys Asn Gln Ala Ser Arg Pro Tyr Asn Ile Tyr Pro His Gly Ile
   465 470 475 480
   Thr Asp Val Arg Pro Leu Tyr Ser Arg Arg Leu Pro Lys Gly Val Lys
   485 490 495
   His Leu Lys Asp Phe Pro Ile Leu Pro Gly Glu Ile Phe Lys Tyr Lys
   500 505 510
   Trp Thr Val Thr Val Glu Asp Gly Pro Thr Lys Ser Asp Pro Arg Cys
   515 520 525
   Leu Thr Arg Tyr Tyr Ser Ser Phe Val Asn Met Glu Arg Asp Leu Ala
   530 535 540
   Ser Gly Leu Ile Gly Pro Leu Leu Ile Cys Tyr Lys Glu Ser Val Asp
   545 550 555 560
   Gln Arg Gly Asn Gln Ile Met Ser Asp Lys Arg Asn Val Ile Leu Phe
   565 570 575
   Ser Val Phe Asp Glu Asn Arg Ser Trp Tyr Leu Thr Glu Asn Ile Gln
   580 585 590
   Arg Phe Leu Pro Asn Pro Ala Gly Val Gln Leu Glu Asp Pro Glu Phe
           595                 600                 605
   Gln Ala Ser Asn Ile Met His Ser Ile Asn Gly Tyr Val Phe Asp Ser
       610                 615                 620
   Leu Gln Leu Ser Val Cys Leu His Glu Val Ala Tyr Trp Tyr Ile Leu
   625                 630                 635                 640
   Ser Ile Gly Ala Gln Thr Asp Phe Leu Ser Val Phe Phe Ser Gly Tyr
                   645                 650                 655
   Thr Phe Lys His Lys Met Val Tyr Glu Asp Thr Leu Thr Leu Phe Pro
               660                 665                 670
   Phe Ser Gly Glu Thr Val Phe Met Ser Met Glu Asn Pro Gly Leu Trp
           675                 680                 685
   Ile Leu Gly Cys His Asn Ser Asp Phe Arg Asn Arg Gly Met Thr Ala
       690                 695                 700
   Leu Leu Lys Val Ser Ser Cys Asp Lys Asn Thr Gly Asp Tyr Tyr Glu
   705                 710                 715                 720
   Asp Ser Tyr Glu Asp Ile Ser Ala Tyr Leu Leu Ser Lys Asn Asn Ala
                   725                 730                 735
   Ile Glu Pro Arg Ser Phe Ser Gln Asn Ser Arg His Pro Ser Thr Arg
               740                 745                 750
   Gln Lys Gln Phe Asn Ala Thr Thr Ile Pro Glu Asn Asp Ile Glu Lys
           755                 760                 765
   Thr Asp Pro Trp Phe Ala His Arg Thr Pro Met Pro Lys Ile Gln Asn
       770                 775                 780
   Val Ser Ser Ser Asp Leu Leu Met Leu Leu Arg Gln Ser Pro Thr Pro
   785                 790                 795                 800
   His Gly Leu Ser Leu Ser Asp Leu Gln Glu Ala Lys Tyr Glu Thr Phe
                   805                 810                 815
   Ser Asp Asp Pro Ser Pro Gly Ala Ile Asp Ser Asn Asn Ser Leu Ser
               820                 825                 830
   Glu Met Thr His Phe Arg Pro Gln Leu His His Ser Gly Asp Met Val
           835                 840                 845
   Phe Thr Pro Glu Ser Gly Leu Gln Leu Arg Leu Asn Glu Lys Leu Gly
       850                 855                 860
   Thr Thr Ala Ala Thr Glu Leu Lys Lys Leu Asp Phe Lys Val Ser Ser
   865                 870                 875                 880
   Thr Ser Asn Asn Leu Ile Ser Thr Ile Pro Ser Asp Asn Leu Ala Ala
                   885                 890                 895
   Gly Thr Asp Asn Thr Ser Ser Leu Gly Pro Pro Ser Met Pro Val His
               900                 905                 910
   Tyr Asp Ser Gln Leu Asp Thr Thr Leu Phe Gly Lys Lys Ser Ser Pro
           915                 920                 925
   Leu Thr Glu Ser Gly Gly Pro Leu Ser Leu Ser Glu Glu Asn Asn Asp
       930                 935                 940
   Ser Lys Leu Leu Glu Ser Gly Leu Met Asn Ser Gln Glu Ser Ser Trp
   945                 950                 955                 960
   Gly Lys Asn Val Ser Ser Thr Glu Ser Gly Arg Leu Phe Lys Gly Lys
                   965                 970                 975
   Arg Ala His Gly Pro Ala Leu Leu Thr Lys Asp Asn Ala Leu Phe Lys
               980                 985                 990
   Val Ser Ile Ser Leu Leu Lys Thr  Asn Lys Thr Ser Asn  Asn Ser Ala
           995                 1000                1005
   Thr Asn  Arg Lys Thr His Ile  Asp Gly Pro Ser Leu  Leu Ile Glu
       1010                 1015                 1020
   Asn Ser  Pro Ser Val Trp Gln  Asn Ile Leu Glu Ser  Asp Thr Glu
       1025                 1030                 1035
   Phe Lys  Lys Val Thr Pro Leu  Ile His Asp Arg Met  Leu Met Asp
       1040                 1045                 1050
   Lys Asn  Ala Thr Ala Leu Arg  Leu Asn His Met Ser  Asn Lys Thr
       1055                 1060                 1065
   Thr Ser  Ser Lys Asn Met Glu  Met Val Gln Gln Lys  Lys Glu Gly
       1070                 1075                 1080
   Pro Ile  Pro Pro Asp Ala Gln  Asn Pro Asp Met Ser  Phe Phe Lys
       1085                 1090                 1095
   Met Leu  Phe Leu Pro Glu Ser  Ala Arg Trp Ile Gln  Arg Thr His
       1100                 1105                 1110
   Gly Lys  Asn Ser Leu Asn Ser  Gly Gln Gly Pro Ser  Pro Lys Gln
       1115                 1120                 1125
   Leu Val Ser Leu Gly Pro Glu Lys Ser Val Glu Gly Gln Asn Phe
   1130 1135 1140
   Leu Ser Glu Lys Asn Lys Val Val Val Gly Lys Gly Glu Phe Thr
   1145 1150 1155
   Lys Asp Val Gly Leu Lys Glu Met Val Phe Pro Ser Ser Arg Asn
   1160 1165 1170
   Leu Phe Leu Thr Asn Leu Asp Asn Leu His Glu Asn Asn Thr His
   1175 1180 1185
   Asn Gln Glu Lys Lys Ile Gln Glu Glu Ile Glu Lys Lys Glu Thr
   1190 1195 1200
   Leu Ile Gln Glu Asn Val Val Leu Pro Gln Ile His Thr Val Thr
   1205 1210 1215
   Gly Thr Lys Asn Phe Met Lys Asn Leu Phe Leu Leu Ser Thr Arg
   1220 1225 1230
   Gln Asn Val Glu Gly Ser Tyr Asp Gly Ala Tyr Ala Pro Val Leu
   1235 1240 1245
   Gln Asp Phe Arg Ser Leu Asn Asp Ser Thr Asn Arg Thr Lys Lys
   1250 1255 1260
   His Thr Ala His Phe Ser Lys Lys Gly Glu Glu Glu Asn Leu Glu
   1265 1270 1275
   Gly Leu Gly Asn Gln Thr Lys Gln Ile Val Glu Lys Tyr Ala Cys
   1280 1285 1290
   Thr Thr Arg Ile Ser Pro Asn Thr Ser Gln Gln Asn Phe Val Thr
   1295 1300 1305
   Gln Arg Ser Lys Arg Ala Leu Lys Gln Phe Arg Leu Pro Leu Glu
   1310 1315 1320
   Glu Thr Glu Leu Glu Lys Arg Ile Ile Val Asp Asp Thr Ser Thr
   1325 1330 1335
   Gln Trp Ser Lys Asn Met Lys His Leu Thr Pro Ser Thr Leu Thr
   1340 1345 1350
   Gln Ile Asp Tyr Asn Glu Lys Glu Lys Gly Ala Ile Thr Gln Ser
   1355 1360 1365
   Pro Leu Ser Asp Cys Leu Thr Arg Ser His Ser Ile Pro Gln Ala
   1370 1375 1380
   Asn Arg Ser Pro Leu Pro Ile Ala Lys Val Ser Ser Phe Pro Ser
   1385 1390 1395
   Ile Arg Pro Ile Tyr Leu Thr Arg Val Leu Phe Gln Asp Asn Ser
   1400 1405 1410
   Ser His Leu Pro Ala Ala Ser Tyr Arg Lys Lys Asp Ser Gly Val
   1415 1420 1425
   Gln Glu Ser Ser His Phe Leu Gln Gly Ala Lys Lys Asn Asn Leu
   1430 1435 1440
   Ser Leu Ala Ile Leu Thr Leu Glu Met Thr Gly Asp Gln Arg Glu
   1445 1450 1455
   Val Gly Ser Leu Gly Thr Ser Ala Thr Asn Ser Val Thr Tyr Lys
   1460 1465 1470
   Lys Val Glu Asn Thr Val Leu Pro Lys Pro Asp Leu Pro Lys Thr
   1475 1480 1485
   Ser Gly Lys Val Glu Leu Leu Pro Lys Val His Ile Tyr Gln Lys
   1490 1495 1500
   Asp Leu Phe Pro Thr Glu Thr Ser Asn Gly Ser Pro Gly His Leu
   1505 1510 1515
   Asp Leu Val Glu Gly Ser Leu Leu Gln Gly Thr Glu Gly Ala Ile
   1520 1525 1530
   Lys Trp Asn Glu Ala Asn Arg Pro Gly Lys Val Pro Phe Leu Arg
   1535 1540 1545
   Val Ala Thr Glu Ser Ser Ala Lys Thr Pro Ser Lys Leu Leu Asp
   1550 1555 1560
   Pro Leu Ala Trp Asp Asn His Tyr Gly Thr Gln Ile Pro Lys Glu
   1565 1570 1575
   Glu Trp Lys Ser Gln Glu Lys Ser Pro Glu Lys Thr Ala Phe Lys
   1580 1585 1590
   Lys Lys Asp Thr Ile Leu Ser Leu Asn Ala Cys Glu Ser Asn His
   1595 1600 1605
   Ala Ile Ala Ala Ile Asn Glu Gly Gln Asn Lys Pro Glu Ile Glu
   1610 1615 1620
   Val Thr Trp Ala Lys Gln Gly Arg Thr Glu Arg Leu Cys Ser Gln
   1625 1630 1635
   Asn Pro Pro Val Leu Lys Arg His Gln Arg Glu Ile Thr Arg Thr
   1640 1645 1650
   Thr Leu Gln Ser Asp Gln Glu Glu Ile Asp Tyr Asp Asp Thr Ile
   1655 1660 1665
   Ser Val Glu Met Lys Lys Glu Asp Phe Asp Ile Tyr Asp Glu Asp
   1670 1675 1680
   Glu Asn Gln Ser Pro Arg Ser Phe Gln Lys Lys Thr Arg His Tyr
   1685 1690 1695
   Phe Ile Ala Ala Val Glu Arg Leu Trp Asp Tyr Gly Met Ser Ser
   1700 1705 1710
   Ser Pro His Val Leu Arg Asn Arg Ala Gln Ser Gly Ser Val Pro
   1715 1720 1725
   Gln Phe Lys Lys Val Val Phe Gln Glu Phe Thr Asp Gly Ser Phe
   1730 1735 1740
   Thr Gln Pro Leu Tyr Arg Gly Glu Leu Asn Glu His Leu Gly Leu
   1745 1750 1755
   Leu Gly Pro Tyr Ile Arg Ala Glu Val Glu Asp Asn Ile Met Val
   1760 1765 1770
   Thr Phe Arg Asn Gln Ala Ser Arg Pro Tyr Ser Phe Tyr Ser Ser
   1775 1780 1785
   Leu Ile Ser Tyr Glu Glu Asp Gln Arg Gln Gly Ala Glu Pro Arg
   1790 1795 1800
   Lys Asn Phe Val Lys Pro Asn Glu Thr Lys Thr Tyr Phe Trp Lys
   1805 1810 1815
   Val Gln His His Met Ala Pro Thr Lys Asp Glu Phe Asp Cys Lys
   1820 1825 1830
   Ala Trp Ala Tyr Phe Ser Asp Val Asp Leu Glu Lys Asp Val His
   1835 1840 1845
   Ser Gly Leu Ile Gly Pro Leu Leu Val Cys His Thr Asn Thr Leu
   1850 1855 1860
   Asn Pro Ala His Gly Arg Gln Val Thr Val Gln Glu Phe Ala Leu
   1865 1870 1875
   Phe Phe Thr Ile Phe Asp Glu Thr Lys Ser Trp Tyr Phe Thr Glu
   1880 1885 1890
   Asn Met Glu Arg Asn Cys Arg Ala Pro Cys Asn Ile Gln Met Glu
   1895 1900 1905
   Asp Pro Thr Phe Lys Glu Asn Tyr Arg Phe His Ala Ile Asn Gly
   1910 1915 1920
   Tyr Ile Met Asp Thr Leu Pro Gly Leu Val Met Ala Gln Asp Gln
   1925 1930 1935
   Arg Ile Arg Trp Tyr Leu Leu Ser Met Gly Ser Asn Glu Asn Ile
   1940 1945 1950
   His Ser Ile His Phe Ser Gly His Val Phe Thr Val Arg Lys Lys
   1955 1960 1965
   Glu Glu Tyr Lys Met Ala Leu Tyr Asn Leu Tyr Pro Gly Val Phe
   1970 1975 1980
   Glu Thr Val Glu Met Leu Pro Ser Lys Ala Gly Ile Trp Arg Val
   1985 1990 1995
   Glu Cys Leu Ile Gly Glu His Leu His Ala Gly Met Ser Thr Leu
   2000 2005 2010
   Phe Leu Val Tyr Ser Asn Lys Cys Gln Thr Pro Leu Gly Met Ala
   2015 2020 2025
   Ser Gly His Ile Arg Asp Phe Gln Ile Thr Ala Ser Gly Gln Tyr
   2030 2035 2040
   Gly Gln Trp Ala Pro Lys Leu Ala Arg Leu His Tyr Ser Gly Ser
   2045 2050 2055
   Ile Asn Ala Trp Ser Thr Lys Glu Pro Phe Ser Trp Ile Lys Val
   2060 2065 2070
   Asp Leu Leu Ala Pro Met Ile Ile His Gly Ile Lys Thr Gln Gly
   2075 2080 2085
   Ala Arg Gln Lys Phe Ser Ser Leu Tyr Ile Ser Gln Phe Ile Ile
   2090 2095 2100
   Met Tyr Ser Leu Asp Gly Lys Lys Trp Gln Thr Tyr Arg Gly Asn
   2105 2110 2115
   Ser Thr Gly Thr Leu Met Val Phe Phe Gly Asn Val Asp Ser Ser
   2120 2125 2130
   Gly Ile Lys His Asn Ile Phe Asn Pro Pro Ile Ile Ala Arg Tyr
   2135 2140 2145
   Ile Arg Leu His Pro Thr His Tyr Ser Ile Arg Ser Thr Leu Arg
   2150 2155 2160
   Met Glu Leu Met Gly Cys Asp Leu Asn Ser Cys Ser Met Pro Leu
   2165 2170 2175
   Gly Met Glu Ser Lys Ala Ile Ser Asp Ala Gln Ile Thr Ala Ser
   2180 2185 2190
   Ser Tyr Phe Thr Asn Met Phe Ala Thr Trp Ser Pro Ser Lys Ala
   2195 2200 2205
   Arg Leu His Leu Gln Gly Arg Ser Asn Ala Trp Arg Pro Gln Val
   2210 2215 2220
   Asn Asn Pro Lys Glu Trp Leu Gln Val Asp Phe Gln Lys Thr Met
   2225 2230 2235
   Lys Val Thr Gly Val Thr Thr Gln Gly Val Lys Ser Leu Leu Thr
   2240 2245 2250
   Ser Met Tyr Val Lys Glu Phe Leu Ile Ser Ser Ser Gln Asp Gly
   2255 2260 2265
   His Gln Trp Thr Leu Phe Phe Gln Asn Gly Lys Val Lys Val Phe
   2270 2275 2280
   Gln Gly Asn Gln Asp Ser Phe Thr Pro Val Val Asn Ser Leu Asp
   2285 2290 2295
   Pro Pro Leu Leu Thr Arg Tyr Leu Arg Ile His Pro Gln Ser Trp
   2300 2305 2310
   Val His Gln Ile Ala Leu Arg Met Glu Val Leu Gly Cys Glu Ala
   2315 2320 2325
   Gln Asp Leu Tyr
   2330
<210> 4
   <211> 5012
   <212> DNA
   <213> artificial sequence
<220>
   <223> FVIII sans domaine B
<400> 4
   ccgccagtgt gatggatatc tgcagaattc ggcttacacc catggaaata gagctctcca 60
   cctgcttctt tctgtgcctt ttgcgattct gctttagtgc caccagaaga tactacctgg 120
   gtgcagtgga actgtcatgg gactatatgc aaagtgatct cggtgagctg cctgtggacg 180
   caaggtttgt ttatgcatcc ttttttaaaa tacattgagt atgcttgcct tttagatata 240
   gaaatatctg atgctgtctt cttcactaaa ttttgattac atgatttgac agcaatattg 300
   aagagtctaa cagccagcac gcaggttggt aagtactgtg ggaacatcac agattttggc 360
   tccatgccct aaagagaaat tggctttcag attatttgga ttaaaaacaa agactttctt 420
   aagagatgta aaattttcat gatgtttctt tttttgctaa aactaaagaa ttaacgcgta 480
   ttcttttaca tttcagattt cctcctagag tgccaaaatc ttttccattc aacacctcag 540
   tcgtgtacaa aaagactctg tttgtagaat tcacggatca ccttttcaac atcgctaagc 600
   caaggccacc ctggatgggt ctgctaggtc ctaccatcca ggctgaggtt tatgatacag 660
   tggtcattac acttaagaac atggcttccc atcctgtcag tcttcatgct gttggtgtat 720
   cctactggaa agcttctgag ggagctgaat atgatgatca gaccagtcaa agggagaaag 780
   aagatgataa agtcttccct ggtggaagcc atacatatgt ctggcaggtc ctgaaagaga 840
   atggtccaat ggcctctgac ccactgtgcc ttacctactc atatctttct catgtggacc 900
   tggtaaaaga cttgaattca ggcctcattg gagccctact agtatgtaga gaagggagtc 960
   tggccaagga aaagacacag accttgcaca aatttatact actttttgct gtatttgatg 1020
   aagggaaaag ttggcactca gaaacaaaga actccttgat gcaggatagg gatgctgcat 1080
   ctgctcgggc ctggcctaaa atgcacacag tcaatggtta tgtaaacagg tctctgccag 1140
   gtctgattgg atgccacagg aaatcagtct attggcatgt gattggaatg ggcaccactc 1200
   ctgaagtgca ctcaatattc ctcgaaggtc acacatttct tgtgaggaac catcgccagg 1260
   cgtccttgga aatctcgcca ataactttcc ttactgctca aacactcttg atggaccttg 1320
   gacagtttct actgttttgt catatctctt cccaccaaca tgatggcatg gaagcttatg 1380
   tcaaagtaga cagctgtcca gaggaacccc aactacgaat gaaaaataat gaagaagcgg 1440
   aagactatga tgatgatctt actgattctg aaatggatgt ggtcaggttt gatgatgaca 1500
   actctccttc ctttatccaa attcgctcag ttgccaagaa gcatcctaaa acttgggtac 1560
   attacattgc tgctgaagag gaggactggg actatgctcc cttagtcctc gcccccgatg 1620
   acagaagtta taaaagtcaa tatttgaaca atggccctca gcggattggt aggaagtaca 1680
   aaaaagtccg atttatggca tacacagatg aaacctttaa gactcgtgaa gctattcagc 1740
   atgaatcagg aatcttggga cctttacttt atggggaagt tggagacaca ctgttgatta 1800
   tatttaagaa tcaagcaagc agaccatata acatctaccc tcacggaatc actgatgtcc 1860
   gtcctttgta ttcaaggaga ttaccaaaag gtgtaaaaca tttgaaggat tttccaattc 1920
   tgccaggaga aatattcaaa tataaatgga cagtgactgt agaagatggg ccaactaaat 1980
   cagatcctcg gtgcctgacc cgctattact ctagtttcgt taatatggag agagatctag 2040
   cttcaggact cattggccct ctcctcatct gctacaaaga atctgtagat caaagaggaa 2100
   accagataat gtcagacaag aggaatgtca tcctgttttc tgtatttgat gagaaccgaa 2160
   gctggtacct cacagagaat atacaacgct ttctccccaa tccagctgga gtgcagcttg 2220
   aggatccaga gttccaagcc tccaacatca tgcacagcat caatggctat gtttttgata 2280
   gtttgcagtt gtcagtttgt ttgcatgagg tggcatactg gtacattcta agcattggag 2340
   cacagactga cttcctttct gtcttcttct ctggatatac cttcaaacac aaaatggtct 2400
   atgaagacac actcacccta ttcccattct caggagaaac tgtcttcatg tcgatggaaa 2460
   acccaggttt gtttatgcat ccttttttaa aatacattga gtatgcttgc cttttagata 2520
   tagaaatatc tgatgctgtc ttcttcacta aattttgatt acatgatttg acagcaatat 2580
   tgaagagtct aacagccagc acgcaggttg gtaagtactg tgggaacatc acagattttg 2640
   gctccatgcc ctaaagagaa attggctttc agattatttg gattaaaaac aaagactttc 2700
   ttaagagatg taaaattttc atgatgtttt cttttttgct aaaactaaag aattaacgcg 2760
   tattctttta catttcaggt ctatggattc tggggtgcca caactcagac tttcggaaca 2820
   gaggcatgac cgccttactg aaggtttcta gttgtgacaa gaacactggt gattattacg 2880
   aggacagtta tgaagatatt tcagcatact tgctgagtaa aaacaatgcc attgaaccaa 2940
   gaagacgtcg acgagaaata actcgtacta ctcttcagtc agatcaagag gaaattgact 3000
   atgatgatac catatcagtt gaaatgaaga aggaagattt tgacatttat gatgaggatg 3060
   aaaatcagag cccccgcagc tttcaaaaga aaacacgaca ctattttatt gctgcagtgg 3120
   agaggctctg ggattatggg atgagtagct ccccacatgt tctaagaaac agggctcaga 3180
   gtggcagtgt ccctcagttc aagaaagttg ttttccagga atttactgat ggctccttta 3240
   ctcagccctt ataccgtgga gaactaaatg aacatttggg actcctgggg ccatatataa 3300
   gagcagaagt tgaagataat atcatggtaa ctttcagaaa tcaggcctct cgtccctatt 3360
   ccttctattc tagccttatt tcttatgagg aagatcagag gcaaggagca gaacctagaa 3420
   aaaactttgt caagcctaat gaaaccaaaa cttacttttg gaaagtgcaa catcatatgg 3480
   cacccactaa agatgagttt gactgcaaag cctgggctta tttctctgat gttgacctgg 3540
   aaaaagatgt gcactcaggc ctgattggac cccttctggt ctgccacact aacacactga 3600
   accctgctca tgggagacaa gtgacagtac aggaatttgc tctgtttttc accatctttg 3660
   atgagaccaa aagctggtac ttcactgaaa atatggaaag aaactgcagg gctccctgca 3720
   atatccagat ggaagatccc acttttaaag agaattatcg cttccatgca atcaatggct 3780
   acataatgga tacactacct ggcttagtaa tggctcagga tcaaaggatt cgatggtatc 3840
   tgctcagcat gggcagcaat gaaaacatcc attctattca tttcagtgga catgtgttca 3900
   ctgtacgaaa aaaagaggag tataaaatgg cactgtacaa tctctatcca ggtgtttttg 3960
   agacagtgga aatgttacca tccaaagctg gaatttggcg ggtggaatgc cttattggcg 4020
   agcatctaca tgctgggatg agcacacttt ttctggtgta cagcaataag tgtcagactc 4080
   ccctgggaat ggcttctgga cacattagag attttcagat tacagcttca ggacaatatg 4140
   gacagtgggc cccaaagctg gccagacttc attattccgg atcaatcaat gcctggagca 4200
   ccaaggagcc cttttcttgg atcaaggtgg atctgttggc accaatgatt attcacggca 4260
   tcaagaccca gggtgcccgt cagaagttct ccagcctcta catctctcag tttatcatca 4320
   tgtatagtct tgatgggaag aagtggcaga cttatcgagg aaattccact ggaaccttaa 4380
   tggtcttctt tggcaatgtg gattcatctg ggataaaaca caatattttt aaccctccaa 4440
   ttattgctcg atacatccgt ttgcacccaa ctcattatag cattcgcagc actcttcgca 4500
   tggagttgat gggctgtgat ttaaatagtt gcagcatgcc attgggaatg gagagtaaag 4560
   caatatcaga tgcacagatt actgcttcat cctactttac caatatgttt gccacctggt 4620
   ctccttcaaa agctcgactt cacctccaag ggaggagtaa tgcctggaga cctcaggtga 4680
   ataatccaaa agagtggctg caagtggact tccagaagac aatgaaagtc acaggagtaa 4740
   ctactcaggg agtaaaatct ctgcttacca gcatgtatgt gaaggagttc ctcatctcca 4800
   gcagtcaaga tggccatcag tggactctct tttttcagaa tggcaaagta aaggtttttc 4860
   agggaaatca agactccttc acacctgtgg tgaactctct agacccaccg ttactgactc 4920
   gctaccttcg aattcacccc cagagttggg tgcaccagat tgccctgagg atggaggttc 4980
   tgggctgcga ggcacaggac ctctactgac tc 5012
<210> 5
   <211> 1412
   <212> PRT
   <213> artificial sequence
<220>
   <223> FVIII sans domaine B
<400> 5
   Met Gln Ser Asp Leu Gly Glu Leu Pro Val Asp Ala Arg Phe Pro Pro
   1 5 10 15
   Arg Val Pro Lys Ser Phe Pro Phe Asn Thr Ser Val Val Tyr Lys Lys
   20 25 30
   Thr Leu Phe Val Glu Phe Thr Asp His Leu Phe Asn Ile Ala Lys Pro
   35 40 45
   Arg Pro Pro Trp Met Gly Leu Leu Gly Pro Thr Ile Gln Ala Glu Val
   50 55 60
   Tyr Asp Thr Val Val Ile Thr Leu Lys Asn Met Ala Ser His Pro Val
   65 70 75 80
   Ser Leu His Ala Val Gly Val Ser Tyr Trp Lys Ala Ser Glu Gly Ala
   85 90 95
   Glu Tyr Asp Asp Gln Thr Ser Gln Arg Glu Lys Glu Asp Asp Lys Val
   100 105 110
   Phe Pro Gly Gly Ser His Thr Tyr Val Trp Gln Val Leu Lys Glu Asn
           115                 120                 125
   Gly Pro Met Ala Ser Asp Pro Leu Cys Leu Thr Tyr Ser Tyr Leu Ser
       130                 135                 140
   His Val Asp Leu Val Lys Asp Leu Asn Ser Gly Leu Ile Gly Ala Leu
   145                 150                 155                 160
   Leu Val Cys Arg Glu Gly Ser Leu Ala Lys Glu Lys Thr Gln Thr Leu
                   165                 170                 175
   His Lys Phe Ile Leu Leu Phe Ala Val Phe Asp Glu Gly Lys Ser Trp
               180                 185                 190
   His Ser Glu Thr Lys Asn Ser Leu Met Gln Asp Arg Asp Ala Ala Ser
           195                 200                 205
   Ala Arg Ala Trp Pro Lys Met His Thr Val Asn Gly Tyr Val Asn Arg
       210                 215                 220
   Ser Leu Pro Gly Leu Ile Gly Cys His Arg Lys Ser Val Tyr Trp His
   225                 230                 235                 240
   Val Ile Gly Met Gly Thr Thr Pro Glu Val His Ser Ile Phe Leu Glu
                   245                 250                 255
   Gly His Thr Phe Leu Val Arg Asn His Arg Gln Ala Ser Leu Glu Ile
               260                 265                 270
   Ser Pro Ile Thr Phe Leu Thr Ala Gln Thr Leu Leu Met Asp Leu Gly
           275                 280                 285
   Gln Phe Leu Leu Phe Cys His Ile Ser Ser His Gln His Asp Gly Met
       290                 295                 300
   Glu Ala Tyr Val Lys Val Asp Ser Cys Pro Glu Glu Pro Gln Leu Arg
   305                 310                 315                 320
   Met Lys Asn Asn Glu Glu Ala Glu Asp Tyr Asp Asp Asp Leu Thr Asp
                   325                 330                 335
   Ser Glu Met Asp Val Val Arg Phe Asp Asp Asp Asn Ser Pro Ser Phe
               340                 345                 350
   Ile Gln Ile Arg Ser Val Ala Lys Lys His Pro Lys Thr Trp Val His
           355                 360                 365
   Tyr Ile Ala Ala Glu Glu Glu Asp Trp Asp Tyr Ala Pro Leu Val Leu
       370                 375                 380
   Ala Pro Asp Asp Arg Ser Tyr Lys Ser Gln Tyr Leu Asn Asn Gly Pro
   385 390 395 400
   Gln Arg Ile Gly Arg Lys Tyr Lys Lys Val Arg Phe Met Ala Tyr Thr
   405 410 415
   Asp Glu Thr Phe Lys Thr Arg Glu Ala Ile Gln His Glu Ser Gly Ile
   420 425 430
   Leu Gly Pro Leu Leu Tyr Gly Glu Val Gly Asp Thr Leu Leu Ile Ile
   435 440 445
   Phe Lys Asn Gln Ala Ser Arg Pro Tyr Asn Ile Tyr Pro His Gly Ile
   450 455 460
   Thr Asp Val Arg Pro Leu Tyr Ser Arg Arg Leu Pro Lys Gly Val Lys
   465 470 475 480
   His Leu Lys Asp Phe Pro Ile Leu Pro Gly Glu Ile Phe Lys Tyr Lys
   485 490 495
   Trp Thr Val Thr Val Glu Asp Gly Pro Thr Lys Ser Asp Pro Arg Cys
   500 505 510
   Leu Thr Arg Tyr Tyr Ser Ser Phe Val Asn Met Glu Arg Asp Leu Ala
   515 520 525
   Ser Gly Leu Ile Gly Pro Leu Leu Ile Cys Tyr Lys Glu Ser Val Asp
   530 535 540
   Gln Arg Gly Asn Gln Ile Met Ser Asp Lys Arg Asn Val Ile Leu Phe
   545 550 555 560
   Ser Val Phe Asp Glu Asn Arg Ser Trp Tyr Leu Thr Glu Asn Ile Gln
   565 570 575
   Arg Phe Leu Pro Asn Pro Ala Gly Val Gln Leu Glu Asp Pro Glu Phe
   580 585 590
   Gln Ala Ser Asn Ile Met His Ser Ile Asn Gly Tyr Val Phe Asp Ser
   595 600 605
   Leu Gln Leu Ser Val Cys Leu His Glu Val Ala Tyr Trp Tyr Ile Leu
   610 615 620
   Ser Ile Gly Ala Gln Thr Asp Phe Leu Ser Val Phe Phe Ser Gly Tyr
   625 630 635 640
   Thr Phe Lys His Lys Met Val Tyr Glu Asp Thr Leu Thr Leu Phe Pro
   645 650 655
   Phe Ser Gly Glu Thr Val Phe Met Ser Met Glu Asn Pro Gly Leu Trp
   660 665 670
   Ile Leu Gly Cys His Asn Ser Asp Phe Arg Asn Arg Gly Met Thr Ala
   675 680 685
   Leu Leu Lys Val Ser Ser Cys Asp Lys Asn Thr Gly Asp Tyr Tyr Glu
   690 695 700
   Asp Ser Tyr Glu Asp Ile Ser Ala Tyr Leu Leu Ser Lys Asn Asn Ala
   705 710 715 720
   Ile Glu Pro Arg Arg Arg Arg Arg Glu Ile Thr Arg Thr Thr Leu Gln
   725 730 735
   Ser Asp Gln Glu Glu Ile Asp Tyr Asp Asp Thr Ile Ser Val Glu Met
   740 745 750
   Lys Lys Glu Asp Phe Asp Ile Tyr Asp Glu Asp Glu Asn Gln Ser Pro
   755 760 765
   Arg Ser Phe Gln Lys Lys Thr Arg His Tyr Phe Ile Ala Ala Val Glu
   770 775 780
   Arg Leu Trp Asp Tyr Gly Met Ser Ser Ser Pro His Val Leu Arg Asn
   785 790 795 800
   Arg Ala Gln Ser Gly Ser Val Pro Gln Phe Lys Lys Val Val Phe Gln
   805 810 815
   Glu Phe Thr Asp Gly Ser Phe Thr Gln Pro Leu Tyr Arg Gly Glu Leu
   820 825 830
   Asn Glu His Leu Gly Leu Leu Gly Pro Tyr Ile Arg Ala Glu Val Glu
   835 840 845
   Asp Asn Ile Met Val Thr Phe Arg Asn Gln Ala Ser Arg Pro Tyr Ser
   850 855 860
   Phe Tyr Ser Ser Leu Ile Ser Tyr Glu Glu Asp Gln Arg Gln Gly Ala
   865 870 875 880
   Glu Pro Arg Lys Asn Phe Val Lys Pro Asn Glu Thr Lys Thr Tyr Phe
   885 890 895
   Trp Lys Val Gln His His Met Ala Pro Thr Lys Asp Glu Phe Asp Cys
   900 905 910
   Lys Ala Trp Ala Tyr Phe Ser Asp Val Asp Leu Glu Lys Asp Val His
   915 920 925
   Ser Gly Leu Ile Gly Pro Leu Leu Val Cys His Thr Asn Thr Leu Asn
   930 935 940
   Pro Ala His Gly Arg Gln Val Thr Val Gln Glu Phe Ala Leu Phe Phe
   945                 950                 955                 960
   Thr Ile Phe Asp Glu Thr Lys Ser Trp Tyr Phe Thr Glu Asn Met Glu
                   965                 970                 975
   Arg Asn Cys Arg Ala Pro Cys Asn Ile Gln Met Glu Asp Pro Thr Phe
               980                 985                 990
   Lys Glu Asn Tyr Arg Phe His Ala  Ile Asn Gly Tyr Ile  Met Asp Thr
           995                 1000                1005
   Leu Pro  Gly Leu Val Met Ala  Gln Asp Gln Arg Ile  Arg Trp Tyr
       1010                 1015                 1020
   Leu Leu  Ser Met Gly Ser Asn  Glu Asn Ile His Ser  Ile His Phe
       1025                 1030                 1035
   Ser Gly  His Val Phe Thr Val  Arg Lys Lys Glu Glu  Tyr Lys Met
       1040                 1045                 1050
   Ala Leu  Tyr Asn Leu Tyr Pro  Gly Val Phe Glu Thr  Val Glu Met
       1055                 1060                 1065
   Leu Pro  Ser Lys Ala Gly Ile  Trp Arg Val Glu Cys  Leu Ile Gly
       1070                 1075                 1080
   Glu His  Leu His Ala Gly Met  Ser Thr Leu Phe Leu  Val Tyr Ser
       1085                 1090                 1095
   Asn Lys  Cys Gln Thr Pro Leu  Gly Met Ala Ser Gly  His Ile Arg
       1100                 1105                 1110
   Asp Phe  Gln Ile Thr Ala Ser  Gly Gln Tyr Gly Gln  Trp Ala Pro
       1115                 1120                 1125
   Lys Leu  Ala Arg Leu His Tyr  Ser Gly Ser Ile Asn  Ala Trp Ser
       1130                 1135                 1140
   Thr Lys  Glu Pro Phe Ser Trp  Ile Lys Val Asp Leu  Leu Ala Pro
       1145                 1150                 1155
   Met Ile  Ile His Gly Ile Lys  Thr Gln Gly Ala Arg  Gln Lys Phe
       1160                 1165                 1170
   Ser Ser  Leu Tyr Ile Ser Gln  Phe Ile Ile Met Tyr  Ser Leu Asp
       1175                 1180                 1185
   Gly Lys  Lys Trp Gln Thr Tyr  Arg Gly Asn Ser Thr  Gly Thr Leu
       1190                 1195                 1200
   Met Val  Phe Phe Gly Asn Val  Asp Ser Ser Gly Ile  Lys His Asn
       1205                 1210                 1215
   Ile Phe  Asn Pro Pro Ile Ile  Ala Arg Tyr Ile Arg  Leu His Pro
       1220                 1225                 1230
   Thr His  Tyr Ser Ile Arg Ser  Thr Leu Arg Met Glu  Leu Met Gly
       1235                 1240                 1245
   Cys Asp  Leu Asn Ser Cys Ser  Met Pro Leu Gly Met  Glu Ser Lys
       1250                 1255                 1260
   Ala Ile  Ser Asp Ala Gln Ile  Thr Ala Ser Ser Tyr  Phe Thr Asn
       1265                 1270                 1275
   Met Phe  Ala Thr Trp Ser Pro  Ser Lys Ala Arg Leu  His Leu Gln
       1280                 1285                 1290
   Gly Arg  Ser Asn Ala Trp Arg  Pro Gln Val Asn Asn  Pro Lys Glu
       1295                 1300                 1305
   Trp Leu  Gln Val Asp Phe Gln  Lys Thr Met Lys Val  Thr Gly Val
       1310                 1315                 1320
   Thr Thr  Gln Gly Val Lys Ser  Leu Leu Thr Ser Met  Tyr Val Lys
       1325                 1330                 1335
   Glu Phe  Leu Ile Ser Ser Ser  Gln Asp Gly His Gln  Trp Thr Leu
       1340                 1345                 1350
   Phe Phe  Gln Asn Gly Lys Val  Lys Val Phe Gln Gly  Asn Gln Asp
       1355                 1360                 1365
   Ser Phe  Thr Pro Val Val Asn  Ser Leu Asp Pro Pro  Leu Leu Thr
       1370                 1375                 1380
   Arg Tyr  Leu Arg Ile His Pro  Gln Ser Trp Val His  Gln Ile Ala
       1385                 1390                 1395
   Leu Arg  Met Glu Val Leu Gly  Cys Glu Ala Gln Asp  Leu Tyr
       1400                 1405                 1410

## Description

### Domaine de l'invention

La présente invention relève du domaine de l'hémostase, plus particulièrement de celui de l'hémophilie de type A. Elle concerne des variants du Facteur VIII humain et leurs utilisations.

### Arrière-plan technologique

Le Facteur VIII (FVIII) est principalement synthétisé par les hépatocytes et les cellules endothéliales sinusoïdales. La concentration plasmatique du FVIII est comprise entre 0,1 à 0,2 mg/l ; la forme circulante est inactive et associée au facteur de von Willebrand (vWF). Le FVIII se trouve au centre de la voie endogène de la coagulation sanguine (dite intrinsèque). Quand un vaisseau sanguin est endommagé suite à un traumatisme, une hémorragie est déclenchée. En réponse, le processus d'hémostase se met en place : il s'agit d'une chaîne complexe d'évènements aboutissant à la production d'un caillot sanguin qui vient sceller la blessure. La coagulation sanguine commence quand les plaquettes adhèrent aux parois du vaisseau sanguin endommagé. Si la blessure est sévère, la collecte des plaquettes au niveau de la blessure n'est pas suffisante pour former un bouchon évitant la perte du sang. Interviennent alors les facteurs de la coagulation qui ont pour finalité la formation du réseau de fibrine, généré suite à l'action de la thrombine à partir de molécules de fibrinogènes solubles. La formation de ce réseau constitué de fibres insolubles est primordiale pour le maintien de façon ferme du caillot sanguin. Par cascade, on entend que, séquentiellement et à chaque étape, un précurseur protéique est converti en une protéase activée qui va cliver ou va contribuer en tant que cofacteur au clivage de la prochaine protéine précurseur de la cascade. Ainsi, le FVIII est protéolysé en FVIIIa sous l'action de la thrombine et du facteur Xa. Dans cette forme active pro-coagulante (FVIIIa), le FVIII augmente de façon spectaculaire l'efficacité protéolytique du facteur FIXa vis-à-vis du facteur FX.

L'hémophilie de type A est une maladie hémorragique caractérisée par un déficit en FVIII actif résultant d'une anomalie du gène du FVIII récessif. Dans certains rares cas, l'hémophilie A peut également être déclenchée par l'apparition spontanée d'auto-anticorps dirigés contre ce FVIII. On parle alors d'hémophilie A acquise.

L'hémophilie se traduit par un déficit de la coagulation sanguine en réponse à une hémorragie. Les hémophiles de type A non traités présentent des symptômes tels que des saignements excessifs en cas de blessure et parfois même des hémorragies spontanées, notamment au niveau des articulations. L'hémophilie A est la forme la plus fréquente des maladies associées à la coagulation sanguine. Il faut compter un hémophile A pour 5000 à 10 000 naissances d'enfants mâles. Tous les hémophiles ne sont pas atteints de la même façon et avec la même ampleur. Ainsi, l'hémophilie A est dite : i) sévère quand le taux de FVIII est égal ou inférieur à 1% du taux circulant dit « normal » ; ii) modérée quand le taux de FVIII est compris entre 1 et 5% de la normale ; iii) mineure quand le taux de FVIII est compris entre 5 et 30 % de la normale. La distribution des trois formes d'hémophilie A est la suivante : 50% des patients hémophiles sont atteints de la forme sévère, 10% de la forme modérée et 40% de la forme mineure.

Les anomalies génétiques associées au gène codant pour le FVIII sont nombreuses. Le gène codant pour le FVIII est situé à l'extrémité du bras long du chromosome X (locus Xq28). L'hémophilie de type A est la conséquence d'une anomalie de ce gène. Sa transmission est récessive et liée au chromosome X : hommes et femmes transmettent la maladie mais celle-ci ne se révèle que chez l'homme. Ces anomalies peuvent être des mutations des délétions ou bien des inversions. La majorité des patients présentant des mutations ponctuelles faux-sens sont atteints d'hémophilies mineures ou modérées. Concernant les délétions, elles sont classées en deux types : i) les petites délétions ; ii) les grandes délétions (> 1 kb). La majorité des grandes délétions entraîne un phénotype sévère. Au niveau des inversions génétiques, celle de l'intron 22 est la plus importante et est responsable de la majorité des cas d'hémophilie A sévère (45%). Une autre inversion, celle de l'intron 1, bien que moins fréquente (3%), peut être la cause d'une hémophilie sévère.

En résumé, ces mutations ont pour conséquence soit une diminution de la production de molécules FVIII fonctionnellement normales, soit une production quantitative normale de molécules de FVIII fonctionnellement déficientes.

Le gène du FVIII code pour une chaîne polypeptidique de 2351 acides aminés (aa) (SEQ ID No 2) correspondant à un peptide signal de 19 aa et une protéine mature de 2332 aa (330 kDa) (SEQ ID No 3). La séquence nucléotidique du FVIII précurseur est donnée en SEQ ID N°1 et la séquence protéique correspondante en SEQ ID N°2. Le FVIII précurseur est constitué de la succession des sept domaines fonctionnels suivants : A1, a1, A2, a2, B, a3, A3, C1 et C2, de l'extrémité N- à C-terminale (Vehar et al., 312, 337-342, Nature, 1984).

Le FVIII subit une première protéolyse intra-cellulaire au niveau des arginines 1313 et 1648, produisant un FVIII hétérodimérique constitué : i) d'une chaîne lourde A1-a1-A2-a2-B ; ii) et d'une chaîne légère a3-A3-C1-C2. Il circule dans le plasma sous la forme d'un hétérodimère. La liaison entre les deux chaînes est assurée entre autre par la présence d'une molécule de cuivre chélatée au niveau des domaines A1 et A3. Immédiatement après sa sécrétion dans le plasma, le FVIII interagit de façon très affine avec le facteur von Willebrand (vWF) qui le protége des protéases. FVIII et vWF forment un complexe non-covalent dont la liaison se fait principalement au niveau de deux régions du FVIII : la région amino-terminale et la région carboxy-terminale en 2303-2332 (domaine C2) de la chaîne légère. Lors de la coagulation, le FVIII est clivé par la thrombine et le facteur Xa en trois endroits: i) la thrombine clive en Arginine1689 de la chaîne légère et en Arginine372 et Arginine740 de la chaîne lourde ; ii) le facteur Xa clive le FVIII en Arginine336, Arginine372 et Arginine740. Parmi ces clivages, deux sont communs (Arginine372 et Arginine740). Les clivages en Arginine372 et Arginine1689 sont indispensables pour que le FVIII participe à la cascade de coagulation. Ces clivages entraînent l'activation du FVIII, encore appelé FVIIIa, (a pour « activé ») : en plus de l'activité du FVIIIa, ces clivages ont pour conséquence l'excision du domaine B de 170 kDa et la dissociation du FVIIIa du vWF.

Le domaine B du FVIII, défini par les acides aminés 741 à 1648, peut-être délété, totalement ou en partie, sans perte de l'activité du FVIII recombinant (Toole et al., 83 (16), 5939-5942, Proc. Natl. Acad. Sci. USA, 1986 ; Eaton et al., 25 (26), 8343-8347, Biochemistry, 1986 ; Langer et al., 82, 16-25, Behring Inst. Mitt, 1988 ; Meulien et al., 2(4), 301-6, Protein Eng, 1988 ; et US 4,868,112), y compris sur le FVIII porcin (US 6,458,563 ; WO01/68109 ; US 6,770,744), qui peut dans certains cas être utilisé en substitution du FVIII humain.

Des mutations, la plupart ponctuelles, peuvent être insérées à différents sites du FVIII sans induire de perte de l'activité pro-coagulante du FVIII (US 5,744,446 ; US 5,859,204 ; US 6,060,447 ; US 6,180,371 ; US 6,228,620 ; US 6,376,463; EP 1561757; WO02/24723 ; WO97/49725). EP1502921 et WO2005/111074 décrivent des variants de FVIII humain présentant une stabilité améliorée.

On citera aussi les brevets (US 2003/0083257 ; WO2005/040213 ; et US 6,780,614) décrivant des modifications de l'ADNc du FVIII dans le but d'augmenter sa production en cellules animales. Les modifications de l'ADNc se retrouvent dans les brevets : US2002/165177 ; US2002/0182684 ; EP1048726 ; EP1283263.

Le nombre d'unités de FVIII administré s'exprime en unités internationales (UI) par rapport au standard OMS du FVIII. L'activité du FVIII s'exprime soit en pourcentage (par rapport au plasma humain normal) soit en Unités Internationales (par rapport à un standard international). Une unité internationale (UI) d'activité FVIII est équivalente à la quantité de FVIII contenue dans un ml de plasma humain normal. Les dosages de FVIII plasmatique peuvent être réalisés soit par test chronométrique soit par test chromogénique.

Le traitement général de l'hémophilie A (sévère et modérée) est un traitement de substitution prophylactique ou curatif, qui repose sur l'injection répétée du facteur de coagulation déficient ou une perfusion de celui-ci. Les patients atteints d'hémophilie A sont traités par différents types de FVIII d'origine plasmatique ou recombinante : i) recombinants ; ii) produits plasmatiques semi-purifiés ; iii) produits plasmatiques purifiés sur colonnes classiques ou d'immuno-affinité. Les premiers concentrés de FVIII recombinants contenaient de l'albumine comme agent stabilisateur. Il s'agissait du Kogenate® (Bayer), de l'Helixate® (fabriqué par Bayer, distribué par Aventis), et du Recombinate® (Baxter). De nouvelles versions sans albumine ont été mises au point, telles que le Kogenate® FS (Bayer), l'Helixate® FS (Bayer), et le ReFacto^{MC}(Wyeth). Il reste tout de même des traces d'albumine issues du milieu de culture cellulaire utilisé lors de l'étape de production de ces protéines recombinantes.

Le FVIII humain recombinant reste à être optimisé. En effet, il est relativement instable dans les conditions physiologiques, peu actif et peu concentré dans le sang (0,1 µg/ml à 0,2 µg/ml) avec une demi-vie de 10h à 12h.

Le traitement substitutif présente des complications spécifiques au FVIII mettant en échec les traitements habituellement utilisés chez environ 30 % des hémophiles A sévère. En effet, suite aux traitements, des anticorps dirigés contre le FVIII recombinant exogène peuvent apparaître. Ces anticorps anti-FVIII induits inhibent l'activité pro-coagulante du FVIII d'où leur appellation d'« anticorps inhibiteurs » ou encore « inhibiteurs ». Les perfusions avec du FVIII sont rendues inefficaces par ces anticorps, et ont pour conséquence une aggravation de la quantité d'anticorps inhibiteurs lors d'un phénomène appelé « réponse anamnestique ».

Rapidement, les patients ne peuvent plus être traités par du FVIII. Une mesure du « titre » des inhibiteurs est alors effectuée. Ce titre s'exprime en unités internationales Bethesda (UB). 1 UB d'inhibiteurs correspond à l'inhibition de la moitié de la quantité de FVIII présente dans 1 ml de plasma normal. Un titre est dit « bas » lorsque la concentration est inférieure à 10 UB, et « haut » lorsqu'elle est supérieure à 10 UB.

Lorsque le taux d'inhibiteurs est relativement bas, les patients hémophiles peuvent être traités à base de concentrés de FVIII tels que le Kogenate® FS, l'Helixate® FS, le Recombinate®, et le ReFacto^{MC} cités précédemment, mais ceci avec un risque important de faire monter le titre d'inhibiteurs qui doit être alors particulièrement surveillé.

Une des solutions pour le traitement contre les anticorps inhibiteurs est l'induction d'une tolérance immune qui consiste en l'administration de fortes doses de FVIII selon le protocole « de Bonn ». Chez certains patients, la concentration en anticorps inhibiteurs est telle qu'il n'est pas possible de les traiter par une surcharge de FVIII pour des raisons de toxicité.

Une deuxième approche appelée « Protocole de Bonn-Malmo » repose d'une part sur l'immuno-adsorption des inhibiteurs ex *vivo* suivie immédiatement de la réinjection du sang, et d'autre part de l'injection de fortes doses de FVIII combinée à l'administration d'agents immuno-suppresseurs. Ces traitements ont un coût extrêmement élevé en FVIII recombinant et ont obtenu un succès partiel.

Une autre approche consiste à apporter des facteurs coagulants permettant de "court-circuiter" le besoin en FVIII dans la voie intrinsèque de coagulation en utilisant : i) le complexe pro-thrombinique activé (FEIBA® VH, Factor Eight Inhibitor Bypassing Activity ; Baxter) d'origine plasmatique contenant les Facteurs II, VII, IX, et X ; ii) le Facteur VIIa activé recombinant (rFVIIa ; NovoSeven® / Niastase® ; NovoNordisk).

Ces approches ont remporté de francs succès, assombris tout de même par l'apparition dans ce type de thérapie d'effets secondaires (tels que des saignements supplémentaires ou au contraire l'apparition de thromboses associés à la fréquence d'administration).

Il faut noter que le taux circulant de FVIII va augmenter dès l'injection puis décroître progressivement en fonction de sa demi-vie. Pour le FVIII, elle varie entre 8 et 16 heures, en moyenne 12 heures. Le problème de la répétition des injections se pose.

Une autre voie consiste à utiliser un FVIII porcin, dans le but d'échapper aux anticorps dirigés contre le FVIII humain. Des patients ayant développé des inhibiteurs contre le FVIII humain ont été traités avec succès par du FVIII porcin semi-purifié (Hyate:C). Mais ce succès reste partiel car après plusieurs injections de FVIII porcin, des inhibiteurs dirigés contre ce FVIII se développent également comme mentionné dans US2004/0249134. Ce phénomène peut entraîner l'arrêt du traitement. Ipsen et Octagen codéveloppent actuellement un FVIII porcin recombinant avec l'université d'Emory (USA), appelé OBI-1, qui constitue un produit de remplacement du Hyate:C (W02005107776).

L'administration de FVIII porcin ne représente donc pas une solution définitive au traitement de l'hémophilie A à inhibiteurs.

Comme nous le constatons, il n'existe pas à ce jour de traitement idéal pour les personnes atteintes d'hémophilie de type A, avec ou sans inhibiteurs. Les différents problèmes rencontrés avec les traitements à base de FVIII commerciaux associés à l'apparition de ces anticorps inhibiteurs ont motivé les efforts pour concevoir rapidement un nouveau FVIII à activité spécifique pro-coagulante maintenue et ayant perdu les épitopes reconnus par les anticorps inhibiteurs.

Peu d'études se sont penchées sur les spécificités épitopiques des anticorps « inhibiteurs ». Certains anticorps inhibiteurs semblent reconnaître des zones restreintes de la molécule FVIII : i) le domaine C2 de la chaîne légère (2181-2321) ; ii) le domaine A2 de la chaîne lourde (484-509) ; iii) le domaine A3 (1694-2019) (Prescott et al., 89, 3663-3671, Blood, 1997 ; Barrow et al. , 95, 557-561, 2000, Blood).

Le domaine C2 (18-kDa) compris entre la Sérine 2173 et la Tyrosine 2332 comprend le domaine de liaison aux phospholipides membranaires et une partie du domaine de fixation du vWF. Les anticorps inhibiteurs dirigés contre le domaine C2, bloquent principalement cette liaison aux phospholipides nécessaire à l'activité pro-coagulante mais aussi la liaison au vWF. Des mutations sur les positions Méthionine 2199, Phenylalanine 2200, Valine 2223, Lysine 2227, Leucine 2251 et Leucine 2252 ont démontré l'importance de ces acides amines dans l'activité du FVIII et la liaison aux phospholipides et/ou au vWF (Pratt et al., 402 , 439-442, Nature, 1999).

Les anticorps anti-A2 inhibent la fonction du FVIIIa en tant que cofacteur du Facteur X (Lollar et al., 93, 2497-2504, J. Clin. Invest, 1994). L'épitope principal du A2 a été localisé entre l'Arginine 484 et la Leucine 508 (Healey et al., 270, 14505-14509, J. Biol. Chem, 1995).

Les anticorps dirigés contre le domaine A3 et/ou C2 empêchent la stabilisation de l'interaction entre le FVIII et le vWF et interfèrent aussi dans la liaison de la chaîne légère du FVIII au FIX activé.

Les inhibiteurs sont très hétérogènes d'un patient à l'autre, et la spécificité épitopique peut varier au cours du temps. L'étude de la cinétique de l'inhibition du FVIII permet de distinguer deux types d'allo-anticorps : les anticorps de type I, neutralisant complètement le FVIII exogène et les anticorps de type II, n'inhibant jamais totalement l'activité du FVIII. Les anticorps de type II n'inhibent pas complètement l'activité pro-coagulante du FVIII car ils ne sont pas saturables ou possèdent une affinité décroissante en fonction de leur concentration.

Des régions susceptibles d'être reconnues par les anticorps inhibiteurs sont citées dans les brevets US2003/147900 et WO00/48635. Ces régions exposées et antigéniques du FVIII sont comprises entre les positions 1649-2019, 108-355, 403-725 et 2085-2249.

D'autre part, US 2005/0256304 décrit la série de positions suivante sur le FVIII humain sur lesquelles des substitutions seraient susceptibles de diminuer l'antigénicité : 197, 198, 199, 201, 202, 407, 411, 412, 419, 515, 517, 613, 617, 636, 637, 638, 639, 823, 1011, 1013, 1208, 1209, 1210, 1254, 1255, 1257, 1262, 1264, 1268, 1119, 1120, 1121, 1122, et 1123.

La diminution de l'antigénicité du FVIII humain peut être obtenue par glycosylation des sites de reconnaissance des inhibiteurs. Cette méthode est décrite dans US 6,759,216 et JP2004141173.

Une autre alternative consiste à substituer les épitopes du FVIII humain reconnus habituellement par les inhibiteurs dans les domaines : i) A2 (484-509) ; ii) A3 (1694-2019), a3 (1649-1687) ; iii) C2 (2181-2321). Cette solution repose sur l'utilisation d'une protéine recombinante hybride, un FVIII humain/porcin.

Les principales cibles des anticorps inhibiteurs sont situées sur les domaines A2 et C2 du FVIII (Saenko et al., Haemophilia, 2002). Il est en effet admis que 90% des anticorps inhibiteurs sont dirigés contre les domaines A2 et C2 humains (Barrow et al., 95, 564-569, Blood, 2000). De plus, il a été démontré que les inhibiteurs humains ont une activité faible sur le FVIII porcin (Koshihara et al., Blood, 1995).

Il est donc attendu qu'une substitution des épitopes du FVIII humain par des séquences porcines permette l'obtention d'une molécule hybride moins réactive vis-à-vis des anticorps inhibiteurs. Ainsi, les domaines humains A2 et C2 ont été remplacés par leur équivalent porcin (Lubin et al., 269, 8639-8641, Journal of Biological Chemistry, 1994). Toutefois, des anticorps dirigés contre le FVIII porcin finissent, ici encore, par apparaître lors du traitement de patients à inhibiteurs.

De nombreux brevets décrivent des hybrides humain/animal de FVIII, ayant conservé une activité pro-coagulante. Nous définissons par hybride humain/animal toute combinaison (substitution) d'au minimum un acide aminé entre une séquence de FVIII humain et une séquence de FVIII d'origine animale. Ces hybrides ont été produits, d'une part, par substitution de régions (sous-unités fonctionnelles ou domaines structuraux) par les régions animales correspondantes. Ainsi les brevets US 5,888,974 ; US 5,663,060 ; US 5,583,209 ; EP1359222 ; US 5,744,446 ; WO93/20093 ; et WO95/24427 décrivent des molécules hybrides de FVIII issues de combinaisons de chaînes légères et lourdes issues de FVIII humains et non humains, et/ou issues de combinaisons de domaines de FVIII humains/porcins.

US 5,744,446 décrit des variants humains/porcins de FVIII présentant des séquences humaines du domaine A2 substituées par les séquences murines ou porcines correspondantes. Les fragments du domaine A2 substitués sont : 373-540 ; 373-508, 445-508, 484-508, 404-508, 489-508 et 484-489.

US 5,364,771 décrit le mode de purification d'hybrides de FVIII issus de combinaisons de chaînes légères et lourdes de FVIII humains et non humains : FVIII humain dont le domaine A2 est substitué par le domaine A2 porcin.

D'autre part, dans certains brevets, ces hybrides ont été générés par substitution ponctuelle d'un ou de quelques acide aminés du FVIII humain par l' (les) acide(s) aminé(s) équivalent(s) chez l'animal (porcin, canin ou murin). Par exemple, US2004/0197875 décrivent des modifications des charges des codons sur certaines positions du FVIII humain. Ces positions sont définies par rapport à la séquence du FVIII porcin. EP1454916 décrit l'introduction de codons porcins dans l'ADNc humain.

Parmi ces brevets, des études ont porté sur la génération de FVIII hybride humain/porcin au niveau du domaine A2. EP1359222 décrit une étude de la séquence du domaine A2 porcin, dans le but de générer un tel hybride. US2003/166536 ; US 6,376,463 ; WO00/71141 décrivent des substitutions d'acides aminés du FVIII humain au niveau d'épitopes importants du domaine A2, entre les positions 484 et 508 : 486, 490, 491, 493, 494, 496, 498, 499, 500, 502, 503, 504, 505, 506, 507 pour WO0071141 ; et 485, 487, 488, 489, 492, 495, 501, 508 pour US 5,859,204. En particulier, des substitutions par une Alanine sont réalisées sur les positions : Arginine 484, Proline 485, Tyrosine 487, Sérine 488, Arginine 489, Proline 492, Valine 495, Phenylalanine 501, et Isoleucine 508. Ces substitutions possédant une antigénicité réduite pourraient présenter un intérêt thérapeutique.

De même, dans US 6,180,371, l'Arginine 484 est remplacée par la Sérine, la Proline 485 par l'Alanine, l'Arginine 489 par la Glycine, la Proline 492 par la Leucine. Pour ces variants, l'inhibition de la fonction pro-coagulante du FVIII par les anticorps a été atténuée ou n'existe plus. L'intérêt thérapeutique d'un double ou triple mutant sur l'Arginine 484, l'Arginine 489, et la Phenylalanine 501, où chaque codon est remplacé par une Alanine est suggéré.

On trouve également des brevets qui décrivent des variants de FVIII dont les substitutions n'affectent que le domaine C2.

US2004/249134 ; WO03/047507 ; WO02/24723 ; US 6,770,744 décrivent des substitutions au niveau des positions Méthionine 2199, Phenylalanine 2200, Valine 2223, Lysine 2227, Leucine 2251 et Leucine 2252. Ces substitutions ont été réalisées sur un FVIII dépourvu de domaine B. Les acides aminés des positions 2215, 2220, 2320, 2195, 2196, 2290 et 2313 sont substitués par une Alanine.

Concernant la position 2223, une Valine est substituée par une Alanine, par comparaison entre le FVIII humain et porcin. Cette mutation est citée dans l'article de Pratt, "structure of the C2 domain of human FVIII (402, 439-442 Nature, 1999), et dans le brevet US 6,770,744.

Les combinaisons de certaines positions mutées telles que 2199, 2200, 2223 et 2227 sont présentées comme réduisant l'antigénicité du FVIII vis-à-vis de certains anticorps inhibiteurs dirigés contre le domaine C2, tout en maintenant l'activité coagulante du FVIII.

Dans les brevets WO99/46274 et US2005/0079584, l'équipe de J. Lollar propose une région pouvant présenter un intérêt dans la création d'un FVIII moins immunogène : de 2181 à 2243. Cette région a été délimitée de façon très grossière par une étude d'antigénicité d'hybrides humains/porcins. C'est un alignement entre FVIII humain et porcin de la séquence 2181/2243 qui a révélé 17 différences au niveau des positions : 2181, 2182, 2195, 2196, 2197, 2199, 2207, 2216, 2222, 2224, 2225, 2226, 2227, 2228, 2234, 2238, 2243. L'équipe de J. Lollar émet l'hypothèse suivante : une substitution au niveau de ces 17 positions par une Alanine, une Methionine, une Serine, une Glycine, ou bien une Leucine, serait susceptible de générer un FVIII pouvant échapper à des anticorps inhibiteurs. Cette supposition n'est étayée par aucune étude d'antigénicité avec les mutants concernés.

Finalement, des brevets tels que US 6,180,371 ; US2002/182670 ; US 2003/068785 ; US2005/079584; WO99/46274 ; US 7,012,132; WO2005/046583 décrivent la génération d'hybrides humain/porcin pour lesquels les substitutions touchent à la fois des positions des domaines A2 et C2 du FVIII dans le but d'une réduction de l'inhibition par les anticorps inhibiteurs reconnaissant les deux domaines. Notamment, WO2005/046583 décrit des substitutions d'acides aminés sur le domaine A2 et C2 au niveau des positions 484, 489, 492, 2199, 2200, 2251 et 2252. Le FVIII utilisé présente une délétion du domaine B. Seule la position 484, présente une Arginine substituée par une Alanine.

En résumé, bien que de nombreux travaux fassent référence à de nouveaux variants de FVIII, la nécessité d'un nouveau FVIII moins immunogène reste d'actualité, parce qu'aucun FVIII modifié permettant le traitement des patients à inhibiteurs n'est aujourd'hui sur le marché. Par ailleurs, des variants présentant une activité spécifique ou une capacité à être sécrété améliorée sont également d'un grand intérêt pour faciliter la production du FVIII recombinant ou pour améliorer le traitement des patients.

### Résumé de l'invention

Le présent document propose donc de nouveaux variants améliorés du FVIII. Ces variants peuvent avoir perdu les épitopes reconnus par les anticorps inhibiteurs tout en ayant conservé l'essentiel de leur activité pro-coagulante, ou présenter une activité spécifique améliorée ou encore avoir une capacité de sécrétion améliorée. Ces variants peuvent aussi présenter une combinaison de ces caractéristiques. Par exemple, ce document concerne des variants moins immunogènes et présentant une activité spécifique améliorée et/ou une capacité de sécrétion améliorée. De même, il concerne des variants présentant une activité spécifique améliorée et/ou une capacité de sécrétion améliorée.

Un premier objet concerne un variant amélioré du FVIII humain ou d'un dérivé biologiquement actif de celui-ci comprenant une substitution d'au moins un acide aminé sélectionné parmi le groupe constitué des résidus en positions 462, 409, 507, 629, 400, 562, 403, 518, 414, 496, 421, 493, 486, et 494 du domaine A2 et des résidus en positions 2206, 2212, 2226, 2244, 2261, 2275, 2280, 2281, 2282, 2289, 2294, 2311, 2312, et 2316 du domaine C2. Dans un mode de réalisation particulier, le variant de FVIII humain ou du dérivé biologiquement actif de celui-ci présente une unique substitution. Dans un autre mode de réalisation particulier, le variant de FVIII humain ou du dérivé biologiquement actif de celui-ci comprend en outre une substitution d'au moins un acide aminé sélectionné parmi le groupe constitué du résidu en position 2202 du domaine C2 et du résidu en position 437 du domaine A2. Dans un mode de réalisation particulier, le variant de FVIII humain ou du dérivé biologiquement actif de celui-ci comprend la substitution d'au moins deux, trois, quatre, cinq, six, sept, huit, neuf, dix, onze, douze, treize, quatorze ou quinze acides aminés, de préférence sélectionnés parmi les groupes décrits ci-dessus. De préférence, l'acide aminé est substitué par un acide aminé sélectionné parmi une Alanine, une Méthionine, une Sérine, une Glycine, et une Leucine. De manière encore plus préférée, l'acide aminé est substitué par une Alanine. De préférence, le dérivé biologiquement actif de FVIII est un FVIII présentant une délétion partielle ou totale du domaine B.

Dans un mode de réalisation particulier, le variant présente une diminution de l'antigénicité vis-à-vis des anticorps inhibiteurs en comparaison du FVIII humain naturel et conserve une activité pro-coagulante au moins égale à 50 % celle du FVIII humain naturel. Dans un mode de réalisation préféré, ce document concerne un variant amélioré du FVIII humain ou d'un dérivé biologiquement actif de celui-ci comprenant une substitution d'au moins un acide aminé sélectionné parmi le groupe constitué des résidus en positions 462, 409, 507, et 629 du domaine A2 et des résidus en positions 2289, 2294, 2312, et 2316 du domaine C2. Ce variant peut comprendre en outre une substitution d'au moins un acide aminé sélectionné parmi le groupe constitué du résidu en position 2202 du domaine C2 et du résidu en position 437 du domaine A2. Dans un mode de réalisation encore plus préféré, ce document concerne un variant amélioré du FVIII humain ou d'un dérivé biologiquement actif de celui-ci comprenant la substitution d'au moins un acide aminé sélectionné parmi le groupe constitué des résidus en positions 462, 409, 507, et 629 du domaine A2 Dans un autre mode de réalisation, ce document concerne un variant amélioré du FVIII humain ou d'un dérivé biologiquement actif de celui-ci comprenant ou présentant la combinaison de deux substitutions sélectionnées parmi le groupe consistant en 409 + 462, 409 + 507, 462 + 507, 409 + 629, 462 + 629, 507 + 629. Dans encore un autre mode de réalisation, ce document concerne un variant amélioré du FVIII humain ou d'un dérivé biologiquement actif de celui-ci comprenant ou présentant la combinaison de trois substitutions sélectionnées parmi le groupe consistant en 409 + 462 + 507, 462 + 507 + 629, 409 + 462 + 629, 409 + 507 + 629. Dans un autre mode de réalisation particulier, ce document concerne un variant amélioré du FVIII humain ou d'un dérivé biologiquement actif de celui-ci comprenant ou présentant la combinaison de quatre substitutions sur les positions 409, 462, 507 et 629.

Par ailleurs, ces mutations qui engendrent une levée de l'inhibition par les anticorps inhibiteurs peuvent s'avérer très intéressantes en combinaison avec les mutations qui confèrent une activité spécifique supérieure, en permettant de compenser une éventuelle perte relative d'activité de ces mutants moins antigéniques. Dans un mode de réalisation particulier, le variant présente une activité spécifique améliorée par rapport à celle du FVIII humain naturel. Dans un mode de réalisation préféré, ce document concerne un variant amélioré du FVIII humain ou d'un dérivé biologiquement actif de celui-ci comprenant en outre la substitution d'au moins un acide aminé sélectionné parmi le groupe constitué des résidus en positions 2177, 2183, 2186, 2191, 2196, 2204, 2205, 2213, 2217, 2235, 2258, 2264, 2268 et 2269 du domaine C2.

Ces mutations qui engendrent une levée de l'inhibition par les anticorps inhibiteurs peuvent s'avérer également très intéressantes en combinaison avec les mutations qui confèrent une capacité améliorée à être sécrété, en permettant de compenser une éventuelle perte relative de sécrétion de ces mutants moins antigéniques. Dans un mode de réalisation particulier, ce document concerne un variant amélioré du FVIII humain ou d'un dérivé biologiquement actif de celui-ci comprenant en outre la substitution d'au moins un acide aminé sélectionné parmi le groupe constitué des résidus en positions 2175, 2199, 2200, 2215, 2251, 2252 et 2278 du domaine C2. La production massive de mutants ayant conservés au moins 50% de l'activité du FVIII permet également d'envisager leur utilisation dans le cadre de l'analyse de fonctions supplémentaires de la protéine. En plus d'une modulation de son immunogénicité, de sa sécrétion et de son activité spécifique, les propriétés suivantes du FVIII pourraient être améliorées en utilisant les molécules mutées décrites : - liaison au facteur von Willebrand et donc amélioration de la demi-vie du FVIII ou du FVIIIa circulant; -amélioration de la stabilité intrinsèque de la molécule par stabilisation du domaine A2 et donc augmentation de la durée de son efficacité ; - liaison aux phospholipides issus des plaquettes sanguines, des surfaces cellulaires ou des microparticules circulantes et donc amélioration de la génération de FXa ; - liaison au FIXa et au FX et donc amélioration de la génération de FXa ; - diminution de la liaison du FVIII ou du FVIIIa aux molécules responsables de son catabolisme telles que par exemple Low density Lipoprotein receptor-related protein (LRP), Low density Lipoprotein receptor (LDLR), Very Low density Lipoprotein receptor (VLDLR), la Mégaline ou tout autre récepteur qui pourrait être mis en évidence et donc amélioration de la demi-vie du FVIII circulant ; - diminution de la protéolyse du FVIII ou du FVIIIa par des protéases vasculaires telles que par exemple la protéine C activée, le FXa, le FIXa, et donc augmentation de la durée de son efficacité.

Un deuxième objet concerne un acide nucléique codant un variant du FVIII humain ou d'un dérivé biologiquement actif de celui-ci selon la présente invention, une cassette d'expression comprenant cet acide nucléique, un vecteur, de préférence un vecteur d'expression, comprenant cet acide nucléique ou cette cassette d'expression, et une cellule hôte comprenant un acide nucléique, une cassette d'expression ou un vecteur selon le présent document. Le vecteur peut être sélectionné de préférence parmi un plasmide et un vecteur viral. Le présent document concerne également l'utilisation d'un acide nucléique, d'une cassette d'expression, d'un vecteur d'expression ou d'une cellule hôte pour produire un variant du FVIII humain ou d'un dérivé biologiquement actif de celui-ci selon la présente invention.

Un troisième objet de la présente invention concerne une composition pharmaceutique comprenant un variant du FVIII humain ou d'un dérivé biologiquement actif de celui-ci selon la présente invention. Ainsi, la présente invention concerne un variant du FVIII humain ou un dérivé biologiquement actif de celui-ci selon la présente invention en tant que médicament. La présente invention concerne également un variant du FVIII humain ou un dérivé biologiquement actif de celui-ci selon la présente invention pour le traitement de l'hémophilie de type A. Le traitement peut être curatif ou préventif. Dans un mode de réalisation particulier, le patient à traiter est un patient à inhibiteurs. Dans un autre mode de réalisation, le patient à traiter est un patient hémophile avant l'apparition d'inhibiteurs éventuels. Le présent document concerne également une méthode de traitement de l'hémophilie de type A comprenant l'administration d'un variant du FVIII humain ou d'un dérivé biologiquement actif de celui-ci selon la présente invention.

Un quatrième objet concerne l'utilisation d'un variant du FVIII humain ou d'un dérivé biologiquement actif de celui-ci selon la présente invention pour la préparation d'un médicament destiné au traitement de l'hémophilie de type A. Le traitement peut être curatif ou préventif. Dans un mode de réalisation particulier, le patient à traiter est un patient à inhibiteurs. Dans un autre mode de réalisation, le patient à traiter est un patient hémophile avant l'apparition d'inhibiteurs éventuels. Le présent document concerne également une méthode de traitement de l'hémophilie de type A comprenant l'administration d'un variant du FVIII humain ou d'un dérivé biologiquement actif de celui-ci selon la présente invention.

Un cinquième objet de la présente invention concerne l'utilisation d'un ou plusieurs variants du FVIII humain ou d'un dérivé biologiquement actif de celui-ci selon la présente invention pour le diagnostic du type d'inhibiteur chez un patient hémophile de type A.

### Brève description des figures et tableaux.

**Figure 1** **:** Schéma simplifié de la cascade de coagulation. Ca : étape dépendante du calcium. PL : phospholipides de la membrane des plaquettes sanguines. TF : facteur tissulaire. TFPI : tissue factor pathway inhibitor. Le rôle du FVIIIa est d'augmenter l'efficacité catalytique du FIXa dans l'activation du FX. L'assemblage du FXa et du FVa déclenche une augmentation significative de la génération de thrombine.
**Figure 2** **:** Résultats du crible primaire : Activités brutes de 359 mutants Alanine sur les 795 réalisés = cartographie fonctionnelle de ces 359 positions en terme d'activité FVIII.
**Figure 3** **:** Production du FVIII en milieu de culture ; 8 mutants présentent un taux de production très supérieur au FVIII non muté dans les mêmes conditions.
**Figure 4** **:** Activités spécifiques les plus élevées pour 15 mutants par rapport au FVIII non muté dans les mêmes conditions.
**Figure 5** **:** Exemple de détermination de la levée d'inhibition du sérum TD par le mutant FVIII E518A.
   Expression de la levée d'inhibition en pourcentage = - [(b-a)/a] x 100 ; a = pourcentage d'activité résiduelle du WT (sérum + IgG / sérum - IgG) ; b = pourcentage d'activité résiduelle du mutant (sérum + IgG / sérum - IgG).
**Figure 6** **:** Levée d'inhibition du FVIII-4A2 par rapport au FVIII sauvage face aux anticorps inhibiteurs provenant de 5 patients (TD, GC, PR, SL et FS) mesurée par tests Bethesda.

L'activité résiduelle, mesurée après incubation avec les anticorps inhibiteurs, est divisée par l'activité restante après une incubation avec l'anticorps non-immun ; le pourcentage d'activité résiduelle est ainsi calculé.
**Figure 7** **:** Mesure de la diminution de l'inhibition du mutant FVIII-4A2 par un anticorps anti-domaine A2 (GMA012) et un anticorps polyclonal de lapin.
**Figure 8** **:** Titration comparative sur support solide de FVIII-4A2 par rapport à FVIII sauvage en ELISA par des anticorps anti-domaine C2 (ESH4) et anti-domaine A2 (GMA012).
**Figure 9** **:** Mesure comparative de l'activation par la thrombine de FVIII-4A2 et FVIII-sauvage.
**Figure 10** **:** Mesure comparative de la dissociation du domaine A2 et de la perte d'activité qui en résulte pour FVIII-4A2 et FVIII sauvage après activation par la thrombine (IIa).
**Figure 11** **:** Levée d'inhibition du FVIII-3A2 par rapport au FVIII sauvage face aux anticorps inhibiteurs provenant de 4 patients (TD, GC, SL et FS) mesurée par tests Bethesda.

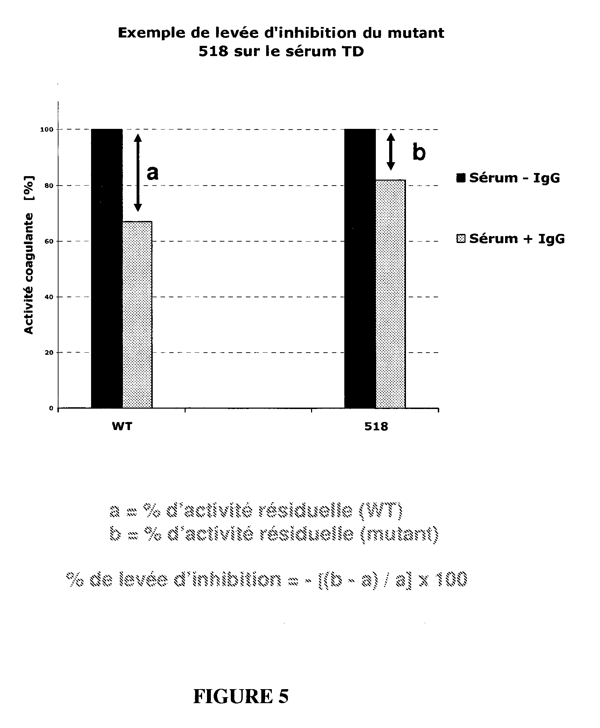

L'activité résiduelle, mesurée après incubation avec les anticorps inhibiteurs, est divisée par l'activité restante après une incubation avec l'anticorps non-immun ; le pourcentage d'activité résiduelle est ainsi calculé.
**Tableau 1 :** Résultats du crible primaire ; liste des 158 mutants Alanine sélectionnés pour le crible secondaire, ayant conservé au minimum 50 % d'activité brute par rapport à l'activité du FVIII non muté.
**Tableau 2 :** Crible secondaire : Tests Bethesda pour 30 mutants présentant une antigénicité modifiée face à 5 sérums de patients hémophiles à inhibiteurs. Les résultats sont les pourcentages de levée d'inhibition pour chaque mutant tels qu'exemplifié dans la figure 5.
**Tableau 3 :** Comparaison de l'activité spécifique et de l'activité brute rapportée à l'activité du FVIII non muté pour les 30 mutants présentant une antigénicité modifiée face à 5 sérums de patients hémophiles à inhibiteurs.
**Tableau 4 :** Liste de tous les doubles mutants FVIII réalisés à partir des 8 mutants simples FVIII409A, FVIII462A, FVIII507A, FVIII629A, FVIII2289A, FVIII2294A, FVIII2312A et FVIII2316A.
**Tableau 5 :** Activités spécifiques chromogéniques et pourcentages de levée d'inhibition des 6 doubles mutants A2 face aux anticorps inhibiteurs de 4 sérums de patients hémophiles TD, GC, SL et PR.

### Description de l'invention

La présente invention propose une solution pour résoudre une complication grave qui peut survenir dans 30 % des traitements de l'hémophilie de type A par le FVIII recombinant: l'apparition d'une réponse immunitaire induite par le traitement et dirigée contre le FVIII recombinant exogène. La solution proposée consiste en la génération de molécules recombinantes de FVIII humain présentant une diminution de l'antigénicité au niveau d'épitopes habituellement reconnus par les anticorps inhibiteurs. Les FVIIIs variants de cette invention ont perdu un ou plusieurs épitopes habituellement reconnus par ces anticorps.

Le présent document propose d'autres solutions consistant en la génération de variants de FVIII humain présentant une amélioration de l'activité spécifique par rapport au FVIII naturel.

Enfin, la présente invention fournit des variants du FVIII présentant une plus grande capacité à être sécrété, ce qui est intéressant pour la production de FVIII recombinant et dans le cadre d'une éventuelle thérapie génique.

Les différentes propriétés qu'engendrent les mutations de ces variants peuvent s'avérer très intéressantes en combinaison. Dans un exemple non limitatif, des mutations qui entraînent une amélioration de l'activité spécifique d'un variant peuvent permettre de compenser une éventuelle perte relative d'activité chez des variants dont les mutations engendrent une levée de l'inhibition par les anticorps inhibiteurs et qui sont donc moins antigéniques. Dans un autre exemple non limitatif, les mutations qui confèrent une capacité supérieure à être sécrétée peuvent s'avérer très intéressantes en combinaison avec les mutations qui engendrent une levée de l'inhibition par les anticorps inhibiteurs, en permettant, par exemple, de compenser une éventuelle perte relative de sécrétion de ces mutants moins antigéniques.

Dans le présent document, la terminologie utilisée pour désigner une substitution est la suivante. S409A indique la substitution du résidu sérine en position 409 de la SEQ ID No 3 par une alanine. Par substitution est entendu le remplacement d'un résidu d'acide aminé par un autre choisi parmi les 19 autres acides aminés ou par un acide aminé non naturel. Les termes « substitution » et « mutation » sont interchangeables. Le signe « + » indique une combinaison de substitutions.

Par « comprendre » est entendu que le variant ou le fragment de celui-ci présente une ou plusieurs substitutions telles qu'indiquées par rapport à la séquence SEQ ID No 3, mais qu'il peut présenter d'autres modifications, notamment des substitutions, des délétions ou des additions.

Par « présenter » est entendu que le variant ou le fragment de celui-ci ne comporte que la ou les substitution(s) indiquée(s) par rapport à la séquence SEQ ID No 3.

Par « variant » est notamment entendu un polypeptide différant d'un polypeptide présentant la séquence SEQ ID No 3 par au moins 1, 2, 3, 4, 5, 6, 7, 8, 9, 10, 11, 12, 13, 14 ou 15 résidu(s), de préférence par 1, 2, 3, 4, 5, 6, 7, 8, 9 ou 10 résidu(s).

Une substitution systématique par une Alanine des acides aminés des domaines A2, A3 ou C2 de FVIII a été réalisée. La production de ces mutants de FVIII humain a été réalisée dans des cellules de mammifères. Le criblage primaire de ces variants a été effectué par rapport à leur activité pro-coagulante. L'activité brute de chaque mutant a été mesurée par test chromogénique et a été comparée à celle du FVIII humain non muté comme référence. La mesure de l'activité des variants de FVIII peut être réalisée au moyen de toute méthode connue de l'homme du métier, de préférence selon la méthode décrite dans l'exemple 3 ci-aprés. Les variants de FVIII sélectionnés pour être les plus actifs du crible primaire ont ensuite été évalués sur un deuxième critère : la perte de l'antigénicité vis-à-vis de sérums de patients hémophiles sélectionnés pour leur capacité à inhiber l'activité du FVIII. Ce crible secondaire par ces anticorps correspond à un test Bethesda modifié. La modification de l'antigénicité des variants de FVIII peut être mesurée au moyen de toute méthode connue de l'homme du métier, de préférence selon la méthode décrite dans l'exemple 4 ci-après.

Des variants améliorés ont pu être sélectionnés. Non seulement certains de ces candidats médicaments ont conservé une activité de coagulation, mais encore ils échappent partiellement à l'inhibition par les anticorps inhibiteurs des sérums de patients hémophiles sélectionnés. Ces FVIIIs ont perdu un ou plusieurs épitopes habituellement reconnus par les anticorps inhibiteurs des sérums de patients. En outre, des candidats médicaments ont montré une activité spécifique de coagulation supérieure à celle du FVIII sauvage. Autre aspect intéressant, des candidats médicaments ont montré une capacité de sécrétion améliorée.

Dans un mode de réalisation, ce document concerne donc des variants du FVIII humain recombinant ayant perdu au minimum un des épitopes habituellement reconnus par les anticorps anti-FVIII dits « inhibiteurs », tout en maintenant une activité de coagulation, de préférence supérieure, similaire ou proche de celle du FVIII non muté.

Le présent document décrit des variants de FVIII humain comprenant au moins une substitution d'un acide aminé par une Alanine ou tout autre acide aminé dans les domaines C2 et A2.

Ce document décrit en particulier 158 mutants Alanine du FVIII humain. Par « mutant Alanine » est entendu un mutant comprenant la substitution d'un acide aminé par un résidu Alanine. En particulier, ces mutants présentent la substitution par une Alanine d'un résidu parmi les positions 2316, 2177, 2181, 2182, 2183, 2186, 2189, 2191, 2197, 2199, 2200, 2204, 2205, 2206, 2212, 2213, 2214, 2217, 2221, 2225, 2226, 2235, 2239, 2242, 2244, 2250, 2251, 2252, 2253, 2256, 2258, 2261, 2263, 2264, 2268, 2269, 2270, 2273, 2274, 2275, 2277, 2278, 2280, 2281, 2282, 2284, 2289, 2292, 2294, 2296, 2311, 2312, 2317, 2321 et 2324 du domaine C2 et les positions 378, 383, 391, 398, 399, 400, 403, 406, 407, 408, 409, 410, 413, 414, 415, 416, 417, 421, 429, 432, 440, 442, 444, 445, 449, 452, 454, 455, 462, 464, 468, 481, 486, 490, 491, 493, 494, 496, 497, 498, 499, 500, 507, 512, 517, 518, 519, 520, 523, 524, 526, 530, 532, 534, 539, 540, 543, 550, 552, 556, 559, 562, 567, 568, 573, 578, 588, 592, 596, 597, 600, 601, 602, 604, 607, 611, 621, 624, 628, 629, 632, 633, 640 et 642 du domaine A2.

Les positions des résidus sont indiquées par rapport à la séquence protéique du FVIII humain de 2332 acides aminés, comme illustrée dans la SEQ ID No 3.

Ce document concerne un variant du FVIII humain ou d'un dérivé biologiquement actif de celui-ci comprenant une substitution d'au moins un acide aminé du domaine C2 sélectionné parmi le groupe constitué des résidus en positions 2316, 2177, 2181, 2182, 2183, 2186, 2189, 2191, 2197, 2199, 2200, 2204, 2205, 2206, 2212, 2213, 2214, 2217, 2221, 2225, 2226, 2235, 2239, 2242, 2244, 2250, 2251, 2252, 2253, 2256, 2258, 2261, 2263, 2264, 2268, 2269, 2270, 2273, 2274, 2275, 2277, 2278, 2280, 2281, 2282, 2284, 2289, 2292, 2294, 2296, 2311, 2312, 2317, 2321 et 2324. Le variant peut comprendre en outre une substitution d'au moins un résidu en position 2175, 2195, 2196, 2202, 2215 et 2222. Le résidu peut être substitué par un acide aminé sélectionné parmi une Alanine, une Méthionine, une Sérine, une Glycine, et une Leucine, de préférence par une Alanine. Ces acides aminés, parmi les vingt acides aminés naturels, sont connus pour diminuer l'antigénicité d'une protéine. La ou les substitutions sur ces positions, en particulier par une Alanine, permet de générer un variant FVIII amélioré, en particulier ayant perdu un ou plusieurs épitopes de reconnaissance par les anticorps inhibiteurs et ayant conservé son activité pro-coagulante. Le présent document concerne également une chaîne légère du FVIII comprenant une substitution d'au moins un acide aminé du domaine C2 sélectionné parmi le groupe constitué des résidus en positions 2316, 2177, 2181, 2182, 2183, 2186, 2189, 2191, 2197, 2199, 2200, 2204, 2205, 2206, 2212, 2213, 2214, 2217, 2221, 2225, 2226, 2235, 2239, 2242, 2244, 2250, 2251, 2252, 2253, 2256, 2258, 2261, 2263, 2264, 2268, 2269, 2270, 2273, 2274, 2275, 2277, 2278, 2280, 2281, 2282, 2284, 2289, 2292, 2294, 2296, 2311, 2312, 2317, 2321 et 2324. Cette chaîne légère peut comprendre en outre une substitution d'au moins un résidu en position 2175, 2195, 2196, 2202, 2215 et 2222.

Ce document concerne également un variant du FVIII humain ou d'un dérivé biologiquement actif de celui-ci comprenant ou contenant une substitution d'au moins un acide aminé du domaine A2, de préférence sélectionné parmi le groupe constitué des résidus en positions 378, 383, 391, 398, 399, 400, 403, 406, 407, 408, 409, 410, 413, 414, 415, 416, 417, 421, 429, 432, 440, 442, 444, 445, 449, 452, 454, 455, 462, 464, 468, 481, 486, 490, 491, 493, 494, 496, 497, 498, 499, 500, 507, 512, 517, 518, 519, 520, 523, 524, 526, 530, 532, 534, 539, 540, 543, 550, 552, 556, 559, 562, 567, 568, 573, 578, 588, 592, 596, 597, 600, 601, 602, 604, 607, 611, 621, 624, 628, 629, 632, 633, 640 et 642. Le variant peut comprendre en outre une substitution d'au moins un résidu en position 377, 379, 405, 434, 437, 485, 488, 489, 492, 495, 501, 508 et 623. Le résidu peut être substitué par un acide aminé sélectionné parmi une Alanine, une Méthionine, une Sérine, une Glycine, et une Leucine, de préférence par une Alanine. La ou les substitutions effectuée sur ces positions, en particulier par une Alanine, permet de générer un variant FVIII amélioré, en particulier ayant perdu un ou plusieurs épitopes de reconnaissance par les anticorps inhibiteurs et ayant conservé son activité pro-coagulante. Le présent document concerne également une chaîne lourde du FVIII, facultativement dénuée totalement ou partiellement de domaine B, comprenant une substitution d'au moins un acide aminé du domaine A2 sélectionné parmi le groupe constitué des résidus en positions 378, 383, 391, 398, 399, 400, 403, 406, 407, 408, 409, 410, 413, 414, 415, 416, 417, 421, 429, 432, 440, 442, 444, 445, 449, 452, 454, 455, 462, 464, 468, 481, 486, 490, 491, 493, 494, 496, 497, 498, 499, 500, 507, 512, 517, 518, 519, 520, 523, 524, 526, 530, 532, 534, 539, 540, 543, 550, 552, 556, 559, 562, 567, 568, 573, 578, 588, 592, 596, 597, 600, 601, 602, 604, 607, 611, 621, 624, 628, 629, 632, 633, 640 et 642. Le variant peut comprendre en outre une substitution d'au moins un résidu en position 377, 379, 405, 434, 437, 485, 488, 489, 492, 495, 501, 508 et 623.

Ce document concerne en outre un variant du FVIII humain ou d'un dérivé biologiquement actif de celui-ci comprenant une substitution d'au moins un acide aminé comprenant ou contenant une substitution d'au moins un acide aminé sélectionné parmi le groupe constitué des résidus en positions 2316, 2177, 2181, 2182, 2183, 2186, 2189, 2191, 2197, 2199, 2200, 2204, 2205, 2206, 2212, 2213, 2214, 2217, 2221, 2225, 2226, 2235, 2239, 2242, 2244, 2250, 2251, 2252, 2253, 2256, 2258, 2261, 2263, 2264, 2268, 2269, 2270, 2273, 2274, 2275, 2277, 2278, 2280, 2281, 2282, 2284, 2289, 2292, 2294, 2296, 2311, 2312, 2317, 2321 et 2324 du domaine C2 et des résidus en positions 378, 383, 391, 398, 399, 400, 403, 406, 407, 408, 409, 410, 413, 414, 415, 416, 417, 421, 429, 432, 440, 442, 444, 445, 449, 452, 454, 455, 462, 464, 468, 481, 486, 490, 491, 493, 494, 496, 497, 498, 499, 500, 507, 512, 517, 518, 519, 520, 523, 524, 526, 530, 532, 534, 539, 540, 543, 550, 552, 556, 559, 562, 567, 568, 573, 578, 588, 592, 596, 597, 600, 601, 602, 604, 607, 611, 621, 624, 628, 629, 632, 633, 640 et 642 du domaine A2. Dans un mode de réalisation particulier, le variant de FVIII humain ou du dérivé biologiquement actif de celui-ci comprend en outre une substitution d'au moins un acide aminé sélectionné parmi le groupe constitué des résidus en positions 2175, 2195, 2196, 2202, 2215 et 2222 du domaine C2 et des résidus en positions 377, 379, 405, 434, 437, 485, 488, 489, 492, 495, 501, 508 et 623 du domaine A2. Dans un mode de réalisation particulier, le variant de FVIII humain ou du dérivé biologiquement actif de celui-ci comprend la substitution d'au moins deux, trois, quatre, cinq, six, sept, huit, neuf, dix, onze, douze, treize, quatorze ou quinze acides aminés, de préférence sélectionnés parmi les groupes décrits ci-dessus.

Dans un mode de réalisation préféré, ce document concerne un variant amélioré du FVIII humain ou d'un dérivé biologiquement actif de celui-ci présentant une antigénicité diminuée et comprenant la substitution d'au moins un acide aminé sélectionné parmi le groupe constitué des résidus en positions 2206, 2212, 2226, 2244, 2261, 2275, 2280, 2281, 2282, 2289, 2294, 2311, 2312, et 2316 du domaine C2 et des résidus en positions 400, 403, 409, 414, 421, 462, 486, 493, 494, 496, 507, 518, 562, et 629 du domaine A2. Dans un autre mode de réalisation, ce variant peut comprendre en outre une substitution d'au moins un acide aminé sélectionné parmi le groupe constitué du résidu en position 2202 du domaine C2 et du résidu en position 437 du domaine A2. Le résidu peut être substitué par un acide aminé sélectionné parmi une Alanine, une Méthionine, une Sérine, une Glycine, et une Leucine, de préférence par une Alanine. Dans un mode de réalisation particulier, ce variant de FVIII humain ou d'un dérivé biologiquement actif de celui-ci présente une unique substitution. Cette substitution unique est de préférence sélectionnée parmi le groupe constitué des substitutions L400A, L400M, L400S, L400G, D403A, D403M, D403S, D403G, D403L, S409A, S409M, S409G, S409L, N414A, N414M, N414S, N414G, N414L, R421A, R421M, R421S, R421G, R421L, L462A, L462M, L462S, L462G, L486A, L486M, L486G, K493M, K493S, K493G, K493L, G494A, G494M, G494L, K496A, K496S, K496G, K496L, E507A, E507M, E507S, E507L, E518A, E518M, E518S, E518G, E518L, R562A, R562M, R562S, R562G, R562L, V629A, V629M, V629S, V629G et V629L dans le domaine A2 et des substitutions S2206A, S2206G, S2206M, S2206L, L2212A, L2212M, L2212S, L2212G, P2226A, P2226M, P2226S, P2226G, P2226L, T2244A, T2244M, T2244S, T2244G, T2244L, L2261A, L2261M, L2261S, L2261G, F2275A, F2275M, F2275S, F2275G, F2275L, V2280A, V2280M, V2280S, V2280G, V2280L, K2281A, K2281M, K2281S, K2281G, K2281L, V2282A, V2282M, V2282S, V2282G, V2282L, S2289A, S2289M, S2289G, S2289L, V2294A, V2294M, V2294S, V2294G, V2294L, Q2311A, Q2311M, Q2311S, Q2311G, Q2311L, S2312A, S2312M, S2312G, S2312L, Q2316A, Q2316M, Q2316S, Q2316G et Q2316L dans le domaine C2. Dans un autre mode de réalisation, ce document concerne un variant du FVIII humain ou d'un dérivé biologiquement actif de celui-ci comprenant au moins une substitution sélectionnée parmi le groupe constitué des substitutions L400A, L400M, L400S, L400G, D403A, D403M, D403S, D403G, D403L, S409A, S409M, S409G, S409L, N414A, N414M, N414S, N414G, N414L, R421A, R421M, R421S, R421G, R421L, L462A, L462M, L462S, L462G, L486A, L486M, L486G, K493M, K493S, K493G, K493L, G494A, G494M, G494L, K496A, K496S, K496G, K496L, E507A, E507M, E507S, E507L, E518A, E518M, E518S, E518G, E518L, R562A, R562M, R562S, R562G, R562L, V629A, V629M, V629S, V629G et V629L dans le domaine A2 et des substitutions S2206A, S2206G, S2206M, S2206L, L2212A, L2212M, L2212S, L2212G, , P2226A, P2226M, P2226S, P2226G, P2226L, T2244A, T2244M, T2244S, T2244G, T2244L, L2261A, L2261M, L2261S, L2261G, F2275A, F2275M, F2275S, F2275G, F2275L, V2280A, V2280M, V2280S, V2280G, V2280L, K2281A, K2281M, K2281S, K2281G, K2281L, V2282A, V2282M, V2282S, V2282G, V2282L, S2289A, S2289M, S2289G, S2289L, V2294A, V2294M, V2294S, V2294G, V2294L, Q2311A, Q2311M, Q2311S, Q2311G, Q2311L, S2312A, S2312M, S2312G, S2312L, Q2316A, Q2316M, Q2316S, Q2316G et Q2316L dans le domaine C2. Ces variants FVIII ont perdu un ou plusieurs épitopes habituellement reconnus par ces anticorps et présentent donc une antigénicité diminuée par rapport au FVIII humain non muté. Ils ont, de plus conservé au minimum 50%, de préférence au minimum 60 ou 75%, d'activité brute par rapport au FVIII humain non muté.

Dans un mode de réalisation encore plus préféré, ce document concerne un variant amélioré du FVIII humain ou d'un dérivé biologiquement actif de celui-ci présentant une antigénicité diminuée et ayant conservé au minimum 100% d'activité brute par rapport au FVIII humain non muté, et comprenant la substitution d'au moins un acide aminé sélectionné parmi le groupe constitué des résidus en positions 409, 462, 507, et 629 du domaine A2 et des résidus en positions 2289, 2294, 2312, et 2316 du domaine C2. Dans un autre mode de réalisation, ce variant peut comprendre en outre une substitution d'au moins un acide aminé sélectionné parmi le groupe constitué du résidu en position 2202 du domaine C2 et du résidu en position 437 du domaine A2. Le résidu peut être substitué par un acide aminé sélectionné parmi une Alanine, une Méthionine, une Sérine, une Glycine, et une Leucine, de préférence par une Alanine. Dans un mode de réalisation particulier, ce variant de FVIII humain ou d'un dérivé biologiquement actif de celui-ci présente une unique substitution. Cette substitution est de préférence sélectionnée parmi le groupe constitué des substitutions S409A, S409M, S409G, S409L, L462A, L462M, L462S, L462G, E507A, E507M, E507S, E507L, V629A, V629M, V629S, V629G, V629L, S2289A, S2289M, S2289G, S2289L, V2294A, V2294M, V2294S, V2294G, V2294L, S2312A, S2312M, S2312G, S2312L, Q2316A, Q2316M, Q2316S, Q2316G et Q2316L. Dans un autre mode de réalisation, ce document concerne un variant du FVIII humain ou d'un dérivé biologiquement actif de celui-ci comprenant au moins une substitution sélectionnée parmi le groupe constitué des substitutions S409A, S409M, S409G, S409L, L462A, L462M, L462S, L462G, E507A, E507M, E507S, E507L, V629A, V629M, V629S, V629G, V629L, S2289A, S2289M, S2289G, S2289L, V2294A, V2294M, V2294S, V2294G, V2294L, S2312A, S2312M, S2312G, S2312L, Q2316A, Q2316M, Q2316S, Q2316G et Q2316L.

Dans un autre mode de réalisation, ce document concerne un variant amélioré du FVIII humain ou d'un dérivé biologiquement actif de celui-ci présentant une antigénicité diminuée et comprenant la combinaison de deux substitutions sélectionnées parmi le groupe consistant en 409 + 462, 409 + 507, 462 + 507, 409 + 629, 462 + 629 et 507 + 629, de préférence 409 + 462, 409 + 507 et 462 + 507. Dans un mode de réalisation particulier, ce variant du FVIII humain ou d'un dérivé biologiquement actif de celui-ci comprend la combinaison de deux substitutions sélectionnée parmi le groupe consistant en S409A+L462A, S409A+L462M, S409A+L462S, S409A+L462G, S409A+E507A, S409A+E507M, S409A+E507S, S409A+E507G, S409A+E507L, S409A+V629A, S409A+V629M, S409A+V629S, S409A+V629G, S409A+V629L, S409M+L462A, S409M+L462M, S409M+L462S, S409M+L462G, S409M+E507A, S409M+E507M, S409M+E507S, S409M+E507G, S409M+E507L, S409M+V629A, S409M+V629M, S409M+V629S, S409M+V629G, S409M+V629L, S409G+L462A, S409G+L462M, S409G+L462S, S409G+L462G, S409G+E507A, S409G+E507M, S409G+E507S, S409G+E507G, S409G+E507L, S409G+V629A, S409G+V629M, S409G+V629S, S409G+V629G, S409G+V629L, S409L+L462A, S409L+L462M, S409L+L462S, S409L+L462G, S409L+E507A, S409L+E507M, S409L+E507S, S409L+E507G, S409L+E507L, S409L+V629A, S409L+V629M, S409L+V629S, S409L+V629G, S409L+V629L, L462A+E507A, L462A+E507M, L462A+E507S, L462A+E507G, L462A+E507L, L462A+V629A, L462A+V629M, L462A+V629S, L462A+V629G, L462A+V629L, L462M+E507A, L462M+E507M, L462M+E507S, L462M+E507G, L462M+E507L, L462M+V629A, L462M+V629M, L462M+V629S, L462M+V629G, L462M+V629L, L462S+E507A, L462S+E507M, L462S+E507S, L462S+E507G, L462S+E507L, L462S+V629A, L462S+V629M, L462S+V629S, L462S+V629G, L462S+V629L, L462G+E507A, L462G+E507M, L462G+E507S, L462G+E507G, L462G+E507L, L462G+V629A, L462G+V629M, L462G+V629S, L462G+V629G, L462G+V629L, E507A+V629A, E507A+V629M, E507A+V629S, E507A+V629G, E507A+V629L, E507M+V629A, E507M+V629M, E507M+V629S, E507M+V629G, E507M+V629L, E507S+V629A, E507S+V629M, E507S+V629S, E507S+V629G, E507S+V629L, E507G+V629A, E507G+V629M, E507G+V629S, E507G+V629G, E507G+V629L, E507L+V629A, E507L+V629M, E507L+V629S, E507L+V629G et E507L+V629L, de préférence parmi le groupe consistant en S409A+L462A, S409A+L462M, S409A+L462S, S409A+L462G, S409A+E507A, S409A+E507M, S409A+E507S, S409A+E507G, S409A+E507L, S409M+L462A, S409M+L462M, S409M+L462S, S409M+L462G, S409M+E507A, S409M+E507M, S409M+E507S, S409M+E507G, S409M+E507L, S409G+L462A, S409G+L462M, S409G+L462S, S409G+L462G, S409G+E507A, S409G+E507M, S409G+E507S, S409G+E507G, S409G+E507L, S409L+L462A, S409L+L462M, S409L+L462S, S409L+L462G, S409L+E507A, S409L+E507M, S409L+E507S, S409L+E507G, S409L+E507L, L462A+E507A, L462A+E507M, L462A+E507S, L462A+E507G, L462A+E507L, L462M+E507A, L462M+E507M, L462M+E507S, L462M+E507G, L462M+E507L, L462S+E507A, L462S+E507M, L462S+E507S, L462S+E507G, L462S+E507L, L462G+E507A, L462G+E507M, L462G+E507S, L462G+E507G et L462G+E507L

Dans encore un autre mode de réalisation, ce document concerne un variant amélioré du FVIII humain ou d'un dérivé biologiquement actif de celui-ci comprenant la combinaison de trois substitutions sélectionnées parmi le groupe consistant en 409 + 462 + 507, 462 + 507 + 629, 409 + 462 + 629, 409 + 507 + 629, de préférence 409 + 462 + 507. Dans un mode de réalisation particulier, ce variant du FVIII humain ou d'un dérivé biologiquement actif de celui-ci comprend la combinaison de trois substitutions sélectionnée parmi le groupe consistant en S409A+L462A+E507A, S409A+L462A+E507M, S409A+L462A+E507S, S409A+L462A+E507G, S409A+L462A+E507L, S409A+L462M +E507A, S409A+L462M+E507M, S409A+L462M+E507S, S409A+L462M+E507G, S409A+L462M+E507L, S409A+L462S+E507A, S409A+L462S+E507M, S409A+L462S+E507S, S409A+L462S+E507G, S409A+L462S+E507L, S409A+L462G+E507A, S409A+L462G+E507M, S409A+L462G+E507S, S409A+L462G+E507G, S409A+L462G+E507L, S409M+L462A+E507A, S409M+L462A+E507M, S409M+L462A+E507S, S409M+L462A+E507G, S409M+L462A+E507L, S409M+L462M+E507A, S409M+L462M+E507M, S409M+L462M+E507S, S409M+L462M+E507G, S409M+L462M+E507L, S409M+L462S+E507A, S409M+L462S+E507M, S409M+L462S+E507S, S409M+L462S+E507G, S409M+L462S+E507L, S409M+L462G+E507A, S409M+L462G+E507M, S409M+L462G+E507S, S409M+L462G+E507G, S409M+L462G+E507L, S409G+L462A+E507A, S409G+L462A+E507M, S409G+L462A+E507S, S409G+L462A+E507G, S409G+L462A+E507L, S409G+L462M+E507A, S409G+L462M+E507M, S409G+L462M+E507S, S409G+L462M+E507G, S409G+L462M+E507L, S409G+L462S+E507A, S409G+L462S+E507M, S409G+L462S+E507S, S409G+L462S+E507G, S409G+L462S+E507L, S409G+L462G+E507A, S409G+L462G+E507M, S409G+L462G+E507S, S409G+L462G+E507G, S409G+L462G+E507L, S409L+L462A+E507A, S409L+L462A+E507M, S409L+L462A+E507S, S409L+L462A+E507G, S409L+L462A+E507L, S409L+L462M+E507A, S409L+L462M+E507M, S409L+L462M+E507S, S409L+L462M+E507G, S409L+L462M+E507L, S409L+L462S+E507A, S409L+L462S+E507M, S409L+L462S+E507S, S409L+L462S+E507G, S409L+L462S+E507L, S409L+L462G+E507A, S409L+L462G+E507M, S409L+L462G+E507S, S409L+L462G+E507G, S409L+L462G+E507L, S409A+L462A+V629A, S409A+L462A+V629M, S409A+L462A+V629S, S409A+L462A+V629G, S409A+L462A+V629L, S409A+L462M+V629A, S409A+L462M+V629M, S409A+L462M+V629S, S409A+L462M+V629G, S409A+L462M+V629L, S409A+L462S+V629A, S409A+L462S+V629M, S409A+L462S+V629S, S409A+L462S+V629G, S409A+L462S+V629L, S409A+L462G+V629A, S409A+L462G+V629M, S409A+L462G+V629S, S409A+L462G+V629G, S409A+L462G+V629L, S409M+L462A+V629A, S409M+L462A+V629M, S409M+L462A+V629S, S409M+L462A+V629G, S409M+L462A+V629L, S409M+L462M+V629A, S409M+L462M+V629M, S409M+L462M+V629S, S409M+L462M+V629G, S409M+L462M+V629L, S409M+L462S+V629A, S409M+L462S+V629M, S409M+L462S+V629S, S409M +L462S+V629G, S409M+L462S+V629L, S409M+L462G+V629A, S409M+L462G+V629M, S409M+L462G+V629S, S409M+L462G+V629G, S409M+L462G+V629L, S409G+L462A+V629A, S409G+L462A+V629M, S409G+L462A+V629S, S409G+L462A+V629G, S409G+L462A+V629L, S409G+L462M+V629A, S409G+L462M+V629M, S409G+L462M+V629S, S409G+L462M+V629G, S409G+L462M+V629L, S409G+L462S+V629A, S409G+L462S+V629M, S409G+L462S+V629S, S409G+L462S+V629G, S409G+L462S+V629L, S409G+L462G+V629A, S409G+L462G+V629M, S409G+L462G+V629S, S409G+L462G+V629G, S409G+L462G+V629L, S409L+L462A+V629A, S409L+L462A+V629M, S409L+L462A+V629S, S409L+L462A+V629G, S409L+L462A+V629L, S409L+L462M+V629A, S409L+L462M+V629M, S409L+L462M+V629S, S409L+L462M+V629G, S409L+L462M+V629L, S409L+L462S+V629A, S409L+L462S+V629M, S409L+L462S+V629S, S409L+L462S+V629G, S409L+L462S+V629L, S409L+L462G+V629A, S409L+L462G+V629M, S409L+L462G+V629S, S409L+L462G+V629G, S409L+L462G+V629L, S409A+E507A+V629A, S409A+E507A+V629M, S409A+E507A+V629S, S409A+E507A+V629G, S409A+E507A+V629L, S409A+E507M+V629A, S409A+E507M+V629M, S409A+E507M+V629S, S409A+E507M+V629G, S409A+E507M+V629L, S409A+E507S+V629A, S409A+E507S+V629M, S409A+E507S+V629S, S409A+E507S+V629G, S409A+E507S+V629L, S409A+E507G+V629A, S409A+E507G+V629M, S409A+E507G+V629S, S409A+E507G+V629G, S409A+E507G+V629L, S409A+E507L+V629A, S409A+E507L+V629M, S409A+E507L+V629S, S409A+E507L+V629G, S409A+E507L+V629L, S409M+E507A+V629A, S409M+E507A+V629M, S409M+E507A+V629S, S409M+E507A+V629G, S409M+E507A+V629L, S409M +E507M +V629A, S409M+E507M+V629M, S409M+E507M+V629S, S409M+E507M+V629G, S409M+E507M+V629L, S409M+E507S+V629A, S409M+E507S+V629M, S409M+E507S+V629S, S409M+E507S+V629G, S409M+E507S+V629L, S409M+E507G+V629A, S409M+E507G+V629M, S409M+E507G+V629S, S409M+E507G+V629G, S409M+E507G+V629L, S409M+E507L+V629A, S409M+E507L+V629M, S409M+E507L+V629S, S409M+E507L+V629G, S409M+E507L+V629L, S409G+E507A+V629A, S409G+E507A+V629M, S409G+E507A+V629S, S409G+E507A+V629G, S409G+E507A+V629L, S409G+E507M+V629A, S409G+E507M+V629M, S409G+E507M+V629S, S409G+E507M+V629G, S409G+E507M+V629L, S409G+E507S+V629A, S409G+E507S+V629M, S409G+E507S+V629S, S409G+E507S+V629G, S409G+E507S+V629L, S409G+E507G+V629A, S409G+E507G+V629M, S409G+E507G+V629S, S409G+E507G+V629G, S409G+E507G+V629L, S409G+E507L+V629A, S409G+E507L+V629M, S409G+E507L+V629S, S409G+E507L+V629G, S409G+E507L+V629L, S409L+E507A+V629A, S409L+E507A+V629M, S409L+E507A+V629S, S409L+E507A+V629G, S409L+E507A+V629L, S409L+E507M+V629A, S409L+E507M+V629M, S409L+E507M+V629S, S409L+E507M+V629G, S409L+E507M+V629L, S409L+E507S+V629A, S409L+E507S+V629M, S409L+E507S+V629S, S409L+E507S+V629G, S409L+E507S+V629L, S409L+E507G+V629A, S409L+E507G+V629M, S409L+E507G+V629S, S409L+E507G+V629G, S409L+E507G+V629L, S409L+E507L+V629A, S409L+E507L+V629M, S409L+E507L+V629S, S409L+E507L+V629G, S409L+E507L+V629L, L462A+E507A+V629A, L462A+E507A+V629M, L462A+E507A+V629S, L462A+E507A+V629G, L462A+E507A+V629L, L462A+E507M+V629A, L462A+E507M+V629M, L462A+E507M+V629S, L462A+E507M+V629G, L462A+E507M+V629L, L462A+E507S+V629A, L462A+E507S+V629M, L462A+E507S+V629S, L462A+E507S+V629G, L462A+E507S+V629L, L462A+E507G+V629A, L462A+E507G+V629M, L462A+E507G+V629S, L462A+E507G+V629G, L462A+E507G+V629L, L462A+E507L+V629A, L462A+E507L+V629M, L462A+E507L+V629S, L462A+E507L+V629G, L462A+E507L+V629L, L462M+E507A+V629A, L462M+E507A+V629M, L462M+E507A+V629S, L462M+E507A+V629G, L462M+E507A+V629L, L462M+E507M+V629A, L462M+E507M+V629M, L462M+E507M+V629S, L462M+E507M+V629G, L462M+E507M+V629L, L462M+E507S+V629A, L462M+E507S+V629M, L462M+E507S+V629S, L462M+E507S+V629G, L462M+E507S+V629L, L462M+E507G+V629A, L462M+E507G+V629M, L462M+E507G+V629S, L462M+E507G+V629G, L462M+E507G+V629L, L462M+E507L+V629A, L462M+E507L+V629M, L462M+E507L+V629S, L462M+E507L+V629G, L462M+E507L+V629L, L462S+E507A+V629A, L462S+E507A+V629M, L462S+E507A+V629S, L462S+E507A+V629G, L462S+E507A+V629L, L462S+E507M+V629A, L462S+E507M+V629M, L462S+E507M+V629S, L462S+E507M+V629G, L462S+E507M+V629L, L462S+E507S+V629A, L462S+E507S+V629M, L462S+E507S+V629S, L462S+E507S+V629G, L462S+E507S+V629L, L462S+E507G+V629A, L462S+E507G+V629M, L462S+E507G+V629S, L462S+E507G+V629G, L462S+E507G+V629L, L462S+E507L+V629A, L462S+E507L+V629M, L462S+E507L+V629S, L462S+E507L+V629G, L462S+E507L+V629L, L462G+E507A+V629A, L462G+E507A+V629M, L462G+E507A+V629S, L462G+E507A+V629G, L462G+E507A+V629L, L462G+E507M+V629A, L462G+E507M+V629M, L462G+E507M+V629S, L462G+E507M+V629G, L462G+E507M+V629L, L462G+E507S+V629A, L462G+E507S+V629M, L462G+E507S+V629S, L462G+E507S+V629G, L462G+E507S+V629L, L462G+E507G+V629A, L462G+E507G+V629M, L462G+E507G+V629S, L462G+E507G+V629G, L462G+E507G+V629L, L462G+E507L+V629A, L462G+E507L+V629M, L462G+E507L+V629S, L462G+E507L+V629G et L462G+E507L+V629L, de préférence parmi le groupe consistant en S409A+L462A+E507A, S409A+L462A+E507M, S409A+L462A+E507S, S409A+L462A+E507G, S409A+L462A+E507L, S409A+L462M+E507A, S409A+L462M+E507M, S409A+L462M+E507S, S409A+L462M+E507G, S409A+L462M+E507L, S409A+L462S+E507A, S409A+L462S+E507M, S409A+L462S+E507S, S409A+L462S+E507G, S409A+L462S+E507L, S409A+L462G+E507A, S409A+L462G+E507M, S409A+L462G+E507S, S409A+L462G+E507G, S409A+L462G+E507L, S409M+L462A+E507A, S409M+L462A+E507M, S409M+L462A+E507S, S409M+L462A+E507G, S409M+L462A+E507L, S409M+L462M+E507A, S409M+L462M+E507M, S409M+L462M+E507S, S409M+L462M+E507G, S409M+L462M+E507L, S409M+L462S+E507A, S409M+L462S+E507M, S409M+L462S+E507S, S409M+L462S+E507G, S409M+L462S+E507L, S409M+L462G+E507A, S409M+L462G+E507M, S409M+L462G+E507S, S409M+L462G+E507G, S409M+L462G+E507L, S409G+L462A+E507A, S409G+L462A+E507M, S409G+L462A+E507S, S409G+L462A+E507G, S409G+L462A+E507L, S409G+L462M+E507A, S409G+L462M+E507M, S409G+L462M+E507S, S409G+L462M+E507G, S409G+L462M+E507L, S409G+L462S+E507A, S409G+L462S+E507M, S409G+L462S+E507S, S409G+L462S+E507G, S409G+L462S+E507L, S409G+L462G+E507A, S409G+L462G+E507M, S409G+L462G+E507S, S409G+L462G+E507G, S409G+L462G+E507L, S409L+L462A+E507A, S409L+L462A+E507M, S409L+L462A+E507S, S409L+L462A+E507G, S409L+L462A+E507L, S409L+L462M+E507A, S409L+L462M+E507M, S409L+L462M+E507S, S409L+L462M+E507G, S409L+L462M+E507L, S409L+L462S+E507A, S409L+L462S+E507M, S409L+L462S+E507S, S409L+L462S+E507G, S409L+L462S+E507L, S409L+L462G+E507A, S409L+L462G+E507M, S409L+L462G+E507S, S409L+L462G+E507G et S409L+L462G+E507L.

Dans un autre mode de réalisation particulier, ce document concerne un variant amélioré du FVIII humain ou d'un dérivé biologiquement actif de celui-ci comprenant la combinaison de quatre substitutions sur les positions 409, 462, 507 et 629. Dans un mode de réalisation particulier, ce variant du FVIII humain ou d'un dérivé biologiquement actif de celui-ci comprend la combinaison de quatre substitutions sélectionnée parmi le groupe consistant en S409A+L462A+E507A+V629A, S409A+L462A+E507A+V629M, S409A+L462A+E507A+V629S, S409A+L462A+E507A+V629G, S409A+L462A+E507A+V629L, S409A+L462A+E507M+V629A, S409A+L462A+E507M+V629M, S409A+L462A+E507M+V629S, S409A+L462A+E507M+V629G, S409A+L462A+E507M+V629L, S409A+L462A+E507S+V629A, S409A+L462A+E507S+V629M, S409A+L462A+E507S+V629S, S409A+L462A+E507S+V629G, S409A+L462A+E507S+V629L, S409A+L462A+E507G+V629A, S409A+L462A+E507G+V629M, S409A+L462A+E507G+V629S, S409A+L462A+E507G+V629G, S409A+L462A+E507G+V629L, S409A+L462A+E507L+V629A, S409A+L462A+E507L+V629M, S409A+L462A+E507L+V629S, S409A+L462A+E507L+V629G, S409A+L462A+E507L+V629L, S409A+L462M +E507A+V629A, S409A+L462M+E507A+V629M, S409A+L462M+E507A+V629S, S409A+L462M+E507A+V629G, S409A+L462M+E507A+V629L, S409A+L462M+E507M+V629A, S409A+L462M+E507M+V629M, S409A+L462M+E507M+V629S, S409A+L462M+E507M+V629G, S409A+L462M+E507M+V629L, S409A+L462M+E507S+V629A, S409A+L462M+E507S+V629M, S409A+L462M+E507S+V629S, S409A+L462M +E507S+V629G, S409A+L462M+E507S+V629L, S409A+L462M+E507G+V629A, S409A+L462M +E507G+V629M, S409A+L462M+E507G+V629S, S409A+L462M+E507G+V629G, S409A+L462M+E507G+V629L, S409A+L462M+E507L+V629A, S409A+L462M+E507L+V629M, S409A+L462M+E507L+V629S, S409A+L462M+E507L+V629G, S409A+L462M+E507L+V629L, S409A+L462S+E507A+V629A, S409A+L462S+E507A+V629M, S409A+L462S+E507A+V629S, S409A+L462S+E507A+V629G, S409A+L462S+E507A+V629L, S409A+L462S+E507M+V629A, S409A+L462S+E507M+V629M, S409A+L462S+E507M+V629S, S409A+L462S+E507M+V629G, S409A+L462S+E507M+V629L, S409A+L462S+E507S+V629A, S409A+L462S+E507S+V629M, S409A+L462S+E507S+V629S, S409A+L462S+E507S+V629G, S409A+L462S+E507S+V629L, S409A+L462S+E507G+V629A, S409A+L462S+E507G+V629M, S409A+L462S+E507G+V629S, S409A+L462S+E507G+V629G, S409A+L462S+E507G+V629L, S409A+L462S+E507L+V629A, S409A+L462S+E507L+V629M, S409A+L462S+E507L+V629S, S409A+L462S+E507L+V629G, S409A+L462S+E507L+V629L, S409A+L462G+E507A+V629A, S409A+L462G+E507A+V629M, S409A+L462G+E507A+V629S, S409A+L462G+E507A+V629G, S409A+L462G+E507A+V629L, S409A+L462G+E507M+V629A, S409A+L462G+E507M+V629M, S409A+L462G+E507M+V629S, S409A+L462G+E507M +V629G, S409A+L462G+E507M+V629L, S409A+L462G+E507S+V629A, S409A+L462G+E507S+V629M, S409A+L462G+E507S+V629S, S409A+L462G+E507S+V629G, S409A+L462G+E507S+V629L, S409A+L462G+E507G+V629A, S409A+L462G+E507G+V629M, S409A+L462G+E507G+V629S, S409A+L462G+E507G+V629G, S409A+L462G+E507G+V629L, S409A+L462G+E507L+V629A, S409A+L462G+E507L+V629M, S409A+L462G+E507L+V629S, S409A+L462G+E507L+V629G, S409A+L462G+E507L+V629L, S409M+L462A+E507A+V629A, S409M+L462A+E507A+V629M, S409M+L462A+E507A+V629S, S409M+L462A+E507A+V629G, S409M+L462A+E507A+V629L, S409M+L462A+E507M+V629A, S409M+L462A+E507M+V629M, S409M+L462A+E507M+V629S, S409M+L462A+E507M+V629G, S409M+L462A+E507M+V629L, S409M+L462A+E507S+V629A, S409M+L462A+E507S+V629M, S409M+L462A+E507S+V629S, S409M+L462A+E507S+V629G, S409M+L462A+E507S+V629L, S409M+L462A+E507G+V629A, S409M+L462A+E507G+V629M, S409M+L462A+E507G+V629S, S409M+L462A+E507G+V629G, S409M+L462A+E507G+V629L, S409M+L462A+E507L+V629A, S409M+L462A+E507L+V629M, S409M+L462A+E507L+V629S, S409M+L462A+E507L+V629G, S409M+L462A+E507L+V629L, S409M+L462M+E507A+V629A, S409M+L462M+E507A+V629M, S409M+L462M+E507A+V629S, S409M+L462M+E507A+V629G, S409M+L462M+E507A+V629L, S409M+L462M+E507M+V629A, S409M+L462M+E507M+V629M, S409M+L462M+E507M+V629S, S409M+L462M+E507M+V629G, S409M+L462M+E507M+V629L, S409M+L462M+E507S+V629A, S409M+L462M+E507S+V629M, S409M+L462M+E507S+V629S, S409M+L462M+E507S+V629G, S409M+L462M+E507S+V629L, S409M+L462M+E507G+V629A, S409M+L462M+E507G+V629M, S409M+L462M+E507G+V629S, S409M+L462M+E507G+V629G, S409M+L462M+E507G+V629L, S409M+L462M+E507L+V629A, S409M+L462M+E507L+V629M, S409M+L462M+E507L+V629S, S409M+L462M+E507L+V629G, S409M+L462M+E507L+V629L, S409M+L462S+E507A+V629A, S409M+L462S+E507A+V629M, S409M+L462S+E507A+V629S, S409M+L462S+E507A+V629G, S409M+L462S+E507A+V629L, S409M+L462S+E507M+V629A, S409M+L462S+E507M+V629M, S409M +L462S+E507M +V629S, S409M+L462S+E507M+V629G, S409M+L462S+E507M+V629L, S409M+L462S+E507S+V629A, S409M+L462S+E507S+V629M, S409M+L462S+E507S+V629S, S409M+L462S+E507S+V629G, S409M+L462S+E507S+V629L, S409M+L462S+E507G+V629A, S409M+L462S+E507G+V629M, S409M+L462S+E507G+V629S, S409M+L462S+E507G+V629G, S409M+L462S+E507G+V629L, S409M+L462S+E507L+V629A, S409M+L462S+E507L+V629M, S409M+L462S+E507L+V629S, S409M+L462S+E507L+V629G, S409M+L462S+E507L+V629L, S409M+L462G+E507A+V629A, S409M+L462G+E507A+V629M, S409M+L462G+E507A+V629S, S409M+L462G+E507A+V629G, S409M+L462G+E507A+V629L, S409M+L462G+E507M+V629A, S409M+L462G+E507M+V629M, S409M+L462G+E507M+V629S, S409M+L462G+E507M+V629G, S409M+L462G+E507M+V629L, S409M+L462G+E507S+V629A, S409M+L462G+E507S+V629M, S409M+L462G+E507S+V629S, S409M+L462G+E507S+V629G, S409M+L462G+E507S+V629L, S409M+L462G+E507G+V629A, S409M+L462G+E507G+V629M, S409M+L462G+E507G+V629S, S409M+L462G+E507G+V629G, S409M+L462G+E507G+V629L, S409M+L462G+E507L+V629A, S409M+L462G+E507L+V629M, S409M+L462G+E507L+V629S, S409M+L462G+E507L+V629G, S409M+L462G+E507L+V629L, S409G+L462A+E507A+V629A, S409G+L462A+E507A+V629M, S409G+L462A+E507A+V629S, S409G+L462A+E507A+V629G, S409G+L462A+E507A+V629L, S409G+L462A+E507M+V629A, S409G+L462A+E507M+V629M, S409G+L462A+E507M+V629S, S409G+L462A+E507M+V629G, S409G+L462A+E507M+V629L, S409G+L462A+E507S+V629A, S409G+L462A+E507S+V629M, S409G+L462A+E507S+V629S, S409G+L462A+E507S+V629G, S409G+L462A+E507S+V629L, S409G+L462A+E507G+V629A, S409G+L462A+E507G+V629M, S409G+L462A+E507G+V629S, S409G+L462A+E507G+V629G, S409G+L462A+E507G+V629L, S409G+L462A+E507L+V629A, S409G+L462A+E507L+V629M, S409G+L462A+E507L+V629S, S409G+L462A+E507L+V629G, S409G+L462A+E507L+V629L, S409G+L462M+E507A+V629A, S409G+L462M+E507A+V629M, S409G+L462M+E507A+V629S, S409G+L462M+E507A+V629G, S409G+L462M+E507A+V629L, S409G+L462M+E507M+V629A, S409G+L462M+E507M+V629M, S409G+L462M+E507M+V629S, S409G+L462M+E507M+V629G, S409G+L462M+E507M+V629L, S409G+L462M+E507S+V629A, S409G+L462M+E507S+V629M, S409G+L462M+E507S+V629S, S409G+L462M+E507S+V629G, S409G+L462M+E507S+V629L, S409G+L462M+E507G+V629A, S409G+L462M+E507G+V629M, S409G+L462M+E507G+V629S, S409G+L462M+E507G+V629G, S409G+L462M+E507G+V629L, S409G+L462M+E507L+V629A, S409G+L462M+E507L+V629M, S409G+L462M+E507L+V629S, S409G+L462M+E507L+V629G, S409G+L462M+E507L+V629L, S409G+L462S+E507A+V629A, S409G+L462S+E507A+V629M, S409G+L462S+E507A+V629S, S409G+L462S+E507A+V629G, S409G+L462S+E507A+V629L, S409G+L462S+E507M+V629A, S409G+L462S+E507M +V629M, S409G+L462S+E507M+V629S, S409G+L462S+E507M+V629G, S409G+L462S+E507M+V629L, S409G+L462S+E507S+V629A, S409G+L462S+E507S+V629M, S409G+L462S+E507S+V629S, S409G+L462S+E507S+V629G, S409G+L462S+E507S+V629L, S409G+L462S+E507G+V629A, S409G+L462S+E507G+V629M, S409G+L462S+E507G+V629S, S409G+L462S+E507G+V629G, S409G+L462S+E507G+V629L, S409G+L462S+E507L+V629A, S409G+L462S+E507L+V629M, S409G+L462S+E507L+V629S, S409G+L462S+E507L+V629G, S409G+L462S+E507L+V629L, S409G+L462G+E507A+V629A, S409G+L462G+E507A+V629M, S409G+L462G+E507A+V629S, S409G+L462G+E507A+V629G, S409G+L462G+E507A+V629L, S409G+L462G+E507M+V629A, S409G+L462G+E507M+V629M, S409G+L462G+E507M+V629S, S409G+L462G+E507M+V629G, S409G+L462G+E507M+V629L, S409G+L462G+E507S+V629A, S409G+L462G+E507S+V629M, S409G+L462G+E507S+V629S, S409G+L462G+E507S+V629G, S409G+L462G+E507S+V629L, S409G+L462G+E507G+V629A, S409G+L462G+E507G+V629M, S409G+L462G+E507G+V629S, S409G+L462G+E507G+V629G, S409G+L462G+E507G+V629L, S409G+L462G+E507L+V629A, S409G+L462G+E507L+V629M, S409G+L462G+E507L+V629S, S409G+L462G+E507L+V629G, S409G+L462G+E507L+V629L, S409L+L462A+E507A+V629A, S409L+L462A+E507A+V629M, S409L+L462A+E507A+V629S, S409L+L462A+E507A+V629G, S409L+L462A+E507A+V629L, S409L+L462A+E507M+V629A, S409L+L462A+E507M+V629M, S409L+L462A+E507M+V629S, S409L+L462A+E507M+V629G, S409L+L462A+E507M+V629L, S409L+L462A+E507S+V629A, S409L+L462A+E507S+V629M, S409L+L462A+E507S+V629S, S409L+L462A+E507S+V629G, S409L+L462A+E507S+V629L, S409L+L462A+E507G+V629A, S409L+L462A+E507G+V629M, S409L+L462A+E507G+V629S, S409L+L462A+E507G+V629G, S409L+L462A+E507G+V629L, S409L+L462A+E507L+V629A, S409L+L462A+E507L+V629M, S409L+L462A+E507L+V629S, S409L+L462A+E507L+V629G, S409L+L462A+E507L+V629L, S409L+L462M+E507A+V629A, S409L+L462M+E507A+V629M, S409L+L462M+E507A+V629S, S409L+L462M+E507A+V629G, S409L+L462M+E507A+V629L, S409L+L462M+E507M+V629A, S409L+L462M+E507M+V629M, S409L+L462M+E507M+V629S, S409L+L462M+E507M+V629G, S409L+L462M+E507M+V629L, S409L+L462M+E507S+V629A, S409L+L462M+E507S+V629M, S409L+L462M+E507S+V629S, S409L+L462M+E507S+V629G, S409L+L462M+E507S+V629L, S409L+L462M+E507G+V629A, S409L+L462M+E507G+V629M, S409L+L462M+E507G+V629S, S409L+L462M+E507G+V629G, S409L+L462M+E507G+V629L, S409L+L462M+E507L+V629A, S409L+L462M+E507L+V629M, S409L+L462M+E507L+V629S, S409L+L462M+E507L+V629G, S409L+L462M+E507L+V629L, S409L+L462S+E507A+V629A, S409L+L462S+E507A+V629M, S409L+L462S+E507A+V629S, S409L+L462S+E507A+V629G, S409L+L462S+E507A+V629L, S409L+L462S+E507M+V629A, S409L+L462S+E507M+V629M, S409L+L462S+E507M+V629S, S409L+L462S+E507M+V629G, S409L+L462S+E507M+V629L, S409L+L462S+E507S+V629A, S409L+L462S+E507S+V629M, S409L+L462S+E507S+V629S, S409L+L462S+E507S+V629G, S409L+L462S+E507S+V629L, S409L+L462S+E507G+V629A, S409L+L462S+E507G+V629M, S409L+L462S+E507G+V629S, S409L+L462S+E507G+V629G, S409L+L462S+E507G+V629L, S409L+L462S+E507L+V629A, S409L+L462S+E507L+V629M, S409L+L462S+E507L+V629S, S409L+L462S+E507L+V629G, S409L+L462S+E507L+V629L, S409L+L462G+E507A+V629A, S409L+L462G+E507A+V629M, S409L+L462G+E507A+V629S, S409L+L462G+E507A+V629G, S409L+L462G+E507A+V629L, S409L+L462G+E507M+V629A, S409L+L462G+E507M+V629M, S409L+L462G+E507M+V629S, S409L+L462G+E507M+V629G, S409L+L462G+E507M+V629L, S409L+L462G+E507S+V629A, S409L+L462G+E507S+V629M, S409L+L462G+E507S+V629S, S409L+L462G+E507S+V629G, S409L+L462G+E507S+V629L, S409L+L462G+E507G+V629A, S409L+L462G+E507G+V629M, S409L+L462G+E507G+V629S, S409L+L462G+E507G+V629G, S409L+L462G+E507G+V629L, S409L+L462G+E507L+V629A, S409L+L462G+E507L+V629M, S409L+L462G+E507L+V629S, S409L+L462G+E507L+V629G, S409L+L462G+E507L+V629L.

Dans un autre mode de réalisation préféré, ce document concerne un variant amélioré du FVIII humain ou d'un dérivé biologiquement actif de celui-ci présentant une activité spécifique améliorée et comprenant la substitution d'au moins un acide aminé sélectionné parmi le groupe constitué des résidus en positions 2177, 2183, 2186, 2191, 2204, 2205, 2206, 2213, 2217, 2235, 2258, 2264, 2268 et 2269 du domaine C2. Ce variant peut comprendre en outre la substitution de l'acide aminé en position 2196 du domaine C2. Par ailleurs, ces mutations qui confèrent une activité spécifique supérieure peuvent s'avérer très intéressantes en combinaison avec les mutations qui engendrent une levée de l'inhibition par les anticorps inhibiteurs, en permettant, par exemple, de compenser une éventuelle perte relative d'activité de ces mutants moins antigéniques. Ainsi, dans un mode de réalisation particulier, ce document concerne un variant amélioré du FVIII humain ou d'un dérivé biologiquement actif de celui-ci comprenant la substitution d'au moins un acide aminé sélectionné parmi le groupe constitué des résidus en positions 400, 403, 409, 414, 421, 462, 486, 493, 494, 496, 507, 518, 562, et 629 du domaine A2 et des résidus en positions 2206, 2212, 2226, 2244, 2261, 2275, 2280, 2281, 2282, 2289, 2294, 2311, 2312, et 2316 du domaine C2, et comprenant en outre une substitution d'au moins un acide aminé sélectionné parmi le groupe constitué des résidus en positions 2177, 2183, 2186, 2191, 2204, 2205, 2213, 2217, 2235, 2258, 2264, 2268 et 2269 du domaine C2. De manière préférée, ce variant comprend la substitution d'au moins un acide aminé sélectionné parmi le groupe constitué des résidus en positions 409, 462, 507 et 629 du domaine A2 et des résidus en positions 2289, 2294, 2312, et 2316 du domaine C2, et comprend en outre une substitution d'au moins un acide aminé sélectionné parmi le groupe constitué des résidus en positions 2177, 2183, 2186, 2191, 2204, 2205, 2213, 2217, 2235, 2258, 2264, 2268 et 2269 du domaine C2.

Dans un mode de réalisation préféré additionnel, ce document concerne un variant amélioré du FVIII humain ou d'un dérivé biologiquement actif de celui-ci présentant une capacité améliorée à être sécrété et comprenant la substitution d'au moins un acide aminé sélectionné parmi le groupe constitué des résidus en positions 2199, 2200, 2215, 2251, 2252, 2278, et 2316 du domaine C2. Ce variant peut comprendre en outre la substitution de l'acide aminé en position 2175 du domaine C2. Par ailleurs, ces mutations qui confèrent une capacité supérieure à être sécrétée peuvent s'avérer très intéressantes en combinaison avec les mutations qui engendrent une levée de l'inhibition par les anticorps inhibiteurs, en permettant, par exemple, de compenser une éventuelle perte relative de sécrétion de ces mutants moins antigéniques. Ainsi, dans un mode de réalisation particulier, ce document concerne un variant amélioré du FVIII humain ou d'un dérivé biologiquement actif de celui-ci comprenant la substitution d'au moins un acide aminé sélectionné parmi le groupe constitué des résidus en positions 400, 403, 409, 414, 421, 462, 486, 493, 494, 496, 507, 518, 562, et 629 du domaine A2 et des résidus en positions 2206, 2212, 2226, 2244, 2261, 2275, 2280, 2281, 2282, 2289, 2294, 2311, 2312, et 2316 du domaine C2, et comprenant en outre une substitution d'au moins un acide aminé sélectionné parmi le groupe constitué des résidus en positions 2175, 2199, 2200, 2215, 2251, 2252 et 2278 du domaine C2. De manière préférée, ce variant comprend la substitution d'au moins un acide aminé sélectionné parmi le groupe constitué des résidus en positions 409, 462, 507 et 629 du domaine A2 et des résidus en positions 2289, 2294, 2312, et 2316 du domaine C2, et comprend en outre une substitution d'au moins un acide aminé sélectionné parmi le groupe constitué des résidus en positions 2175, 2199, 2200, 2215, 2251, 2252 et 2278 du domaine C2.

La production massive de mutants ayant conservés au moins 50% de l'activité du FVIII permet également d'envisager leur utilisation dans le cadre de l'analyse de fonctions supplémentaires de la protéine. En plus d'une modulation de son immunogénicité, de sa sécrétion et de son activité spécifique, les propriétés suivantes du FVIII pourraient être améliorées en utilisant les molécules mutées décrites : - liaison au facteur von Willebrand et donc amélioration de la demi-vie du FVIII ou du FVIIIa circulant ; -amélioration de la stabilité intrinsèque de la molécule par stabilisation du domaine A2 et donc augmentation de la durée de son efficacité ; - liaison aux phospholipides issus des plaquettes sanguines, des surfaces cellulaires ou des microparticules circulantes et donc amélioration de la génération de FXa ; - liaison au FIXa et au FX et donc amélioration de la génération de FXa ; - diminution de la liaison du FVIII ou du FVIIIa aux molécules responsables de son catabolisme telles que par exemple Low density Lipoprotein receptor-related protein (LRP), Low density Lipoprotein receptor (LDLR), Very Low density Lipoprotein receptor (VLDLR), la Mégaline ou tout autre récepteur qui pourrait être mis en évidence et donc amélioration de la demi-vie du FVIII circulant ; - diminution de la protéolyse du FVIII ou du FVIIIa par des protéases vasculaires telles que par exemple la protéine C activée, le FXa, le FIXA, et donc augmentation de la durée de son efficacité.

De préférence, le dérivé biologiquement actif de FVIII est un FVIII présentant une délétion partielle ou totale du domaine B. Le variant FVIII humain de la présente invention n'est pas un FVIII hybride. Il ne porte pas de substitution du domaine A2 ou C2 ou d'un segment d'au moins 15 acides aminés consécutifs de ceux-ci par le domaine d'un FVIII d'une autre espèce. En particulier, les segments du domaine A2 373-540, 373-508, 445-508, 484-508, 404-508, 489-508 et/ou 484-489 ne sont pas substitués par ceux d'une autre espèce. Dans un mode de réalisation particulier, la séquence polypeptidique du variant diffère de celle du FVIII humain tel que décrit dans la SEQ ID No 3 par 1, 2, 3, 4, 5, 6, 7, 8, 9, 10, 11, 12, 13, 14 ou 15 substitutions, de préférence par 1, 2, 3, 4, 5, 6, 7 ou 8 substitutions, sans tenir compte d'éventuelle délétion ou troncature. Dans un mode de réalisation particulier, le variant comprend une unique substitution. Dans un autre mode de réalisation particulier, le variant comprend une combinaison de 1 à 8 substitutions sélectionnées dans un groupe selon la présente invention.

Par « anticorps inhibiteurs » ou « inhibiteurs » est entendu tout anticorps qui reconnaît ou se fixe sur FVIII et inhibe l'activité biologique de celui-ci, en particulier son activité pro-coagulante. En particulier, de tels anticorps peuvent reconnaître de préférence i) le domaine C2 de la chaîne légère (2181-2321) ; ii) le domaine A2 de la chaîne lourde (484-509) ; ou iii) le domaine A3 (1694-2019). A titre d'exemple d'anticorps inhibiteurs commercialisés, on peut citer le ESH-8 (inhibiteur fort ; région reconnue 2248/2285 ; 6300 BU/mg ; anti-C2 ; America Diagnostica), le GMA-8015 (anti-A2 ; Green Mountain), l'anticorps ESH-4 anti-C2 (inhibiteur fort ; région 2303/2332; AmericaDiagnostica), l'anticorps Bo2C11 anti-C2 (Jacquemin et al., 92(2), 496-506, Blood, 1998).

Les « patients à inhibiteurs » sont des patients qui présentent des anticorps inhibiteurs du FVIII dans leurs sérums. Selon les patients, le spectre de reconnaissance de ces anticorps est variable. Un FVIII amélioré selon la présente invention est un FVIII qui permet d'échapper au moins partiellement à une ou plusieurs classes d'anticorps inhibiteurs.

Par « dérivé biologiquement actif du FVIII » on entend toute protéine ou peptide dérivé du FVIII humain qui conserve une activité pro-coagulante du FVIII. Par exemple, un tel dérivé biologiquement actif du FVIII peut être un FVIII dont le domaine B (741-1648) a été partiellement ou totalement supprimé (Toole et al., 83 (16), 5939-5942, Proc. Natl. Acad. Sci. USA, 1986 ; Pittman 1993, Blood 81 :2925-2935; Eaton et al., 25 (26), 8343-8347, Biochemistry, 1986 ; Langer et al., 82, 16-25, Behring Inst. Mitt, 1988 ; Meulien et al., 2(4), 301-6, Protein Eng, 1988 ; et US 4,868,112). En outre, ce terme désigne également des mutants du FVIII avec un domaine A2 stabilisé (WO 97/40145), des mutants du FVIII permettant une expression plus forte (Swaroop et al. 1997, JBC 272:24121-24124), des mutants du FVIII présentant une diminution d'antigénicité (Lollar 1999 Thromb. Haemost. 82:505-508), un FVIII reconstitué à partir des chaînes légères et lourdes exprimées séparément (Oh et al. 1999, Exp. Mol. Med. 31 :95-100), des mutants du FVIII présentant une diminution de liaison aux récepteurs associés au catabolisme du FVIII comme HSPG (heparan sulfate proteoglycans) et LRP (low density lipoprotein receptor related protein) (Ananyeva et al. 2001 , TCM, 11 :251-257), des mutants du FVIII présentant une activité spécifique améliorée (US2004/0249134). Sont également considérés les variants du FVIII dans lesquels des segments du FVIII sont remplacés par des segments correspondants du facteur V (Marquette et al. 1995, JBC, 270:10297-10303, Oertel et al. 1996, Thromb. Haemost. 75:36-44). Par ailleurs, ce terme désigne tout FVIII comprenant une ou plusieurs substitutions, délétions ou additions. Par exemple, il comprend les variants décrits dans l'introduction de la présente demande, en particulier ceux comprenant des mutations ponctuelles. Notamment, il comprend un FVIII moins sensible au clivage par APC (protéine C activée) comprenant des mutations des Arginines 336 et 562 et dans la région comprise entre les positions 2001-2020, tel que décrit dans la demande WO 2006/027111. II comprend également un mutant FVIII stabilisé dans lequel une ou des Cystéines ont été introduites de manière à créer un ou des ponts disulfures, par exemple entre les domaines A2 et A3 (WO02103024 ; Gale et Pellequer, 1(9), 1966-71, J Thromb Haemost, 2003). Les brevets JP2005112855 et RU2244556/RU2253475 décrivent des compositions biologiquement stables et exemptes d'albumine respectivement, permettant de stabiliser le FVIII seul ou en association avec le vWF. Ce terme désigne également tout FVIII ayant été modifié par la conjugaison de groupement fonctionnel, par exemple une PEGylation, une glycosylation (par exemple US2005009148, US2003077752 etc ... Par ailleurs, le variant peut comprendre des liaisons peptidiques modifiées pour résister à l'hydrolyse.

En particulier, le variant présente une diminution de l'antigénicité vis-à-vis des anticorps inhibiteurs en comparaison du FVIII humain naturel et conserve une activité pro-coagulante au moins égale à 50 % celle du FVIII humain naturel. Par exemple, un test adapté est le test de coagulation en une ou deux étapes décrit dans Rizza et al. (Rizza et al. 1982 Coagulation assay of Factor VIIIa and FIXa in Bloom ed. The Hemophilias. NY Churchchill Livingston 1992). Dans un mode de réalisation préféré, le variant conserve une activité pro-coagulante égale à celle du FVIII humain naturel. Dans un mode de réalisation encore plus préférée, le variant présente une activité pro-coagulante supérieure à celle du FVIII humain naturel.

L'activité pro-coagulante du FVIII est mesurée par tout moyen connu de l'homme du métier. De préférence, cette activité pro-coagulante est mesurée par test chronométrique ou par test chromogénique. De manière encore plus préférée, l'activité de FVIII est mesurée par test chronométrique, par exemple tel que décrit par Von Clauss (17, 237, A.Acta Haematologica, 1957) ou par test chronométrique tel que décrit par Rosen (Scand J Haematol 33 (Suppl 40), 139-145 (1984).

La présente invention concerne un acide nucléique codant un variant FVIII humain selon la présente invention. La présente invention concerne également une cassette d'expression d'un acide nucléique selon la présente invention. Elle concerne en outre un vecteur comprenant un acide nucléique ou une cassette d'expression selon la présente invention. Le vecteur peut être sélectionné parmi un plasmide et un vecteur viral.

L'acide nucléique peut être de l'ADN (ADNc ou ADNg), de l'ARN, un mélange des deux. Il peut être sous forme simple chaîne ou en duplexe ou un mélange des deux. Il peut comprendre des nucléotides modifiés, comprenant par exemple une liaison modifiée, une base purique ou pyrimidique modifiée, ou un sucre modifié. Il peut être préparé par toutes méthodes connues de l'homme du métier, dont la synthèse chimique, la recombinaison, la mutagenèse, etc...

La cassette d'expression comprend tous les éléments nécessaires à l'expression du variant FVIII humain selon la présente invention, notamment les éléments nécessaires à la transcription et à la traduction dans la cellule hôte. La cellule hôte peut être procaryote ou eucaryote. En particulier, la cassette d'expression comprend un promoteur et un terminateur, facultativement un amplificateur. Le promoteur peut être procaryote ou eucaryote. Des exemples de promoteurs procaryotes préférés sont les suivants : LacI, LacZ, pLacT, ptac, pARA, pBAD, les promoteurs d'ARN polymérase de bactériophage T3 or T7, le promoteur de la polyhèdrine, le promoteur PR ou PL du phage lambda. Des exemples de promoteurs eucaryotes préférés sont les suivants : promoteur précoce du CMV, promoteur de la thymidine kinase de HSV, promoteur précoce ou tardif de SV40, le promoteur de la métallothionéine-L de souris, et les régions LTR de certains rétrovirus. De manière générale, pour le choix d'un promoteur adapté, l'homme du métier pourra avantageusement se référer à l'ouvrage de Sambrook et al. (1989) ou encore aux techniques décrites par Fuller et al. (1996; Immunology in Current Protocols in Molecular Biology).

La présente invention concerne un vecteur portant un acide nucléique ou une cassette d'expression codant pour un variant FVIII humain selon la présente invention. Le vecteur est de préférence un vecteur d'expression, c'est-à-dire qu'il comprend les éléments nécessaires à l'expression du variant dans la cellule hôte. La cellule hôte peut être un procaryote, par exemple *E. coli,* ou un eucaryote. L'eucaryote peut être un eucaryote inférieur comme une levure (par exemple, *S cerevisiae)* ou un champignon (par exemple du genre *Aspergillus)* ou un eucaryote supérieur comme une cellule d'insecte, de mammifère ou de plante. La cellule peut être une cellule mammifère, par exemple COS, CHO (US 4,889,803 ; US 5,047,335). Dans un mode de réalisation particulier, la cellule est non-humaine et non-embryonnaire. Le vecteur peut être un plasmide, un phage, un phagemide, un cosmide, un virus, un YAC, un BAC, un plasmide pTi *d'Agrobacterium,* etc... Le vecteur peut comprendre de préférence un ou plusieurs éléments sélectionnés parmi une origine de réplication, un site de clonage multiple et un gène de sélection. Dans un mode de réalisation préféré, le vecteur est un plasmide. Des exemples non-exhaustifs de vecteurs procaryotes sont les suivants : pQE70, pQE60, pQE-9 (Qiagen), pbs, pD10, phagescript, psiX174, pbluescript SK, pbsks, pNH8A, pNH16A, pNH18A, pNH46A (Stratagene); ptrc99a, pKK223-3, pKK233-3, pDR540, pBR322, et pRIT5 (Pharmacia), pET (Novagen). Des exemples non-exhaustifs de vecteurs eucaryotes sont les suivants : pWLNEO, pSV2CAT, pPICZ, pcDNA3.1 (+) Hyg (Invitrogen), pOG44, pXT1, pSG (Stratagene); pSVK3, pBPV, pCI-neo (Stratagene), pMSG, pSVL (Pharmacia); et pQE-30 (QLAexpress). Les vecteurs viraux peuvent être de manière non-exhaustive des adénovirus, des AAV, des HSV, des lentivirus, etc... De préférence, le vecteur d'expression est un plasmide ou un vecteur viral.

La séquence codant le FVIII selon la présente invention peut comprendre ou ne pas comprendre le peptide signal. Dans le cas où elle ne le comprend pas, une méthionine peut être éventuellement ajoutée à l'extrémité N-terminale. Dans une autre alternative, un peptide signal hétérologue peut être introduit. Ce peptide signal hétérologue peut être dérivé d'un procaryote tel que *E. coli* ou d'un eucaryote, notamment une cellule mammifère, d'insecte ou d'une levure. Par ailleurs, la séquence nucléotidique peut également comprendre des segments d'introns, notamment d'introns hétérologues. Ces segments d'introns peuvent permettre d'améliorer l'expression du variant FVIII. De telles constructions sont décrites dans la demande WO 2005/040213. Par exemple, la séquence nucléotidique peut comprendre la séquence SEQ ID No 5 modifié pour coder le variant FVIII comprenant la ou les substitutions selon la présente invention.

Le présent document concerne l'utilisation d'un acide nucléique, d'une cassette d'expression ou d'un vecteur selon la présente invention pour transformer ou transfecter une cellule. Le présent document concerne une cellule hôte comprenant un acide nucléique, une cassette d'expression ou un vecteur codant un variant FVIII humain et son utilisation pour produire un variant FVIII humain recombinant selon la présente invention. Dans un mode de réalisation particulier, la cellule est non-humaine et non-embryonnaire. Elle concerne également une méthode de production d'un variant FVIII humain recombinant selon la présente invention comprenant la transformation ou transfection d'une cellule par un acide nucléique, une cassette d'expression ou un vecteur selon la présente invention ; la mise en culture de la cellule transfectée/transformée ; et la récolte du variant FVIII humain produit par la cellule. Dans un mode de réalisation alternatif, la méthode de production d'un variant FVIII humain recombinant selon la présente invention comprenant la fourniture d'une cellule comprenant un acide nucléique, une cassette d'expression ou un vecteur selon la présente invention ; la mise en culture de la cellule transfectée/transformée ; et la récolte du variant FVIII humain produit par la cellule. En particulier, la cellule peut être transformée/transfectée de manière transitoire ou stable par l'acide nucléique codant le variant. Cet acide nucléique peut être contenu dans la cellule sous forme d'épisome ou sous forme chromosomique. Les méthodes de production de protéines recombinantes sont bien connues par l'homme du métier. Par exemple, on peut citer les modes spécifiques décrits dans WO0170968 pour une production dans une lignée cellulaire immortalisée humaine, WO2005123928 pour une production dans une plante, US2005229261 pour une production dans le lait d'un animal transgénique, etc...

Le présent document concerne des compositions pharmaceutiques comprenant les variants du FVIII humain selon la présente invention, ainsi que l'utilisation de ces variants FVIII pour la préparation d'un médicament destiné au traitement d'une hémophilie de type A. De préférence, l'hémophilie de type A est sévère et modérée. Ce traitement pourra être curatif ou préventif. Dans un mode de réalisation particulier, les patients traités sont des patients à inhibiteurs.

Ainsi, les variants FVIII de l'invention peuvent être utilisés chez deux catégories majeures de patients hémophiles : - ceux présentant des anticorps inhibiteurs du FVIII installés, grâce leur capacité à échapper à ces anticorps inhibiteurs et - ceux qui n'en présentent pas encore, grâce à leur risque réduit de provoquer un développement d'anticorps inhibiteurs comparés aux molécules utilisées actuellement. Ces variants du FVIII seront utilisables par tous les patients atteints d'hémophilie de type A.

La présente invention concerne donc une composition pharmaceutique comprenant un variant FVIII selon la présente invention. La composition pharmaceutique peut comprendre en outre des composés permettant de stabiliser le mutant FVIII, par exemple de l'albumine sérique, le vWF (facteur de von Willebrand) ou un fragment de celui-ci comprenant le site de liaison du FVIII, des facteurs de coagulation dépendants de la vitamine K, et des polysaccharides tel que le sucrose. La présente invention peut également concerner une composition pharmaceutique comprenant un acide nucléique codant un mutant FVIII selon la présente invention, un vecteur ou une cellule hôte selon la présente invention. Une telle composition pourrait être utile dans le cadre d'une thérapie génique. La composition pharmaceutique peut comprendre en outre un support ou un excipient pharmaceutiquement acceptable. De tels supports et excipients sont bien connus de l'homme du métier (Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed. , Pharmaceutical Press[2000]) et comprennent les solutions physiologiques salées et les tampons phosphates. Le variant FVIII selon la présente invention peut également être formulé dans la composition pharmaceutique avec des phospholipides ou équivalents, par exemple sous forme de liposome, nanoparticule, etc... (WO2004071420 ; WO2004091723). La composition pharmaceutique peut comprendre en outre un ou plusieurs autres principes actifs.

La présente invention concerne également un variant FVIII selon la présente invention en tant que médicament. Elle concerne en outre un acide nucléique codant un mutant FVIII, une cassette d'expression, un vecteur ou une cellule selon la présente invention en tant que médicament.

Les variants de FVIII humain de l'invention peuvent être utilisés comme traitement de substitution dans le cas d'une hémophilie A sévère et modérée. La possibilité d'une utilisation de façon continue avec un risque réduit de développement d'anticorps inhibiteurs est un avantage majeur sur les différents FVIII recombinants humains, ou hybrides existants.

Ces variants améliorés de FVIII humain ont de préférence pour but le traitement des patients à inhibiteurs déjà installés, mais aussi le traitement préventif.

De plus, une administration systématique de ce FVIII pourrait être envisagée à titre prophylactique chez tout patient atteint d'hémophilie de type A. On peut aussi imaginer réduire les risques d'hémorragie par exemple lors d'interventions chirurgicales, ou bien prévenir l'apparition des inhibiteurs. Il peut également être envisagé d'administrer ce FVIII dans le cadre d'un traitement d'urgence, par exemple lors d'hémorragie accidentelle, pathologique ou provoquée par un acte chirurgicale.

Les compositions pharmaceutiques de l'invention sont appropriées pour l'administration orale, sublinguale, sous-cutanée, intramusculaire, intraveineuse, topique, locale, intratrachéale, intranasale, transdermique, rectale, intraoculaire, intra- auriculaire, ledit principe actif pouvant être administré sous forme unitaire d'administration. De préférence, les compositions pharmaceutiques sont appropriées pour l'administration intraveineuse, sous-cutanée, ou intramusculaire.

Les dosages du traitement peuvent varier selon la sévérité de la déficience en FVIII. En règle générale, le dosage est ajusté pour la fréquence, la durée et les unités en fonction de la sévérité et de la durée des épisodes de saignement du patient considéré. Le FVIII est dosé de manière à stopper les saignements, par exemple à l'aide de tests standard de coagulation. Une dose efficace de variant FVIII selon la présente invention peut comprendre, sans y être limitée, entre environ 5 à 50 unité/kg de poids corporel, de préférence 10 et 50, de manière encore plus préféré 20 à 40. La fréquence d'administration peut par exemple être de tous les 8 à 24 heures. La durée du traitement peut par exemple être de 1 à 10 jours, ou jusqu'à ce que les saignements s'arrètent. (Voir par exemple, Roberts, H. R., and M. R. Jones, "Hemophilia and Related Conditions--Congenital Deficiencies of Prothrombin (Factor II, Factor V, and Factors VII to XII), "Ch. 153,1453-1414,1460, in Hematology, Williams, W. J., et al., ed. (1990)).

Le traitement peut prendre la forme d'une seule injection en intraveineuse ou d'une administration périodique ou continue sur une période de temps étendue, selon la nécessité. Le traitement peut également être administré par voie sous-cutanée ou orale avec des liposomes en une ou plusieurs doses à des intervalles de temps différents.

Le présent document concerne l'utilisation d'un variant du FVIII humain ou d'un dérivé biologiquement actif de celui-ci selon la présente invention pour la préparation d'un médicament destiné au traitement des troubles de la coagulation, en particulier de l'hémophilie de type A. Le traitement peut être curatif ou préventif. Dans un mode de réalisation particulier, le patient à traiter est un patient à inhibiteurs. Le présent document concerne également une méthode de traitement de l'hémophilie de type A comprenant l'administration d'un variant du FVIII humain ou d'un dérivé biologiquement actif de celui-ci selon la présente invention.

Le présent document concerne également l'utilisation d'un acide nucléique codant un variant FVIII selon la présente invention pour la préparation d'un médicament destiné au traitement des troubles de la coagulation, en particulier de l'hémophilie de type A.

Le variant FVIII de la présente invention peut également être combiné avec un autre composé actif. Par exemple, le présent document concerne également l'utilisation d'un variant FVIII selon la présente invention en combinaison avec le facteur IXa pour la traitement des troubles de la coagulation, et en particulier l'hémophilie de type A ou de type B. Cette combinaison est décrite dans la demande WO2004103397.

La présente invention concerne en outre l'utilisation d'un ou plusieurs variants du FVIII humain ou d'un dérivé biologiquement actif de celui-ci selon la présente invention pour le diagnostic du type d'inhibiteurs chez un patient hémophile de type A. En particulier, la présence d'anticorps inhibiteurs est testée dans des échantillons de sérum ou de fluides biologiques (lymphe, urine, etc...). La détection d'anticorps inhibiteurs peut être effectuée par ELISA, immunodétection par transfert électrophorétique, dosage radio-immunologique, et des tests d'activité du FVIII par ex, test de coagulation).

En effet, les inventeurs ont révélé sur le FVIII humain sauvage des positions reconnues de façon spécifique par les inhibiteurs. Ces positions peuvent être utilisées individuellement, combinées au sein d'un même domaine, ou combinées entre les domaines A2 et C2, afin de mettre en évidence le ou les types d'anticorps inhibiteurs présents chez un hémophile. En effet, le besoin de diagnostic des anticorps inhibiteurs est capital. Titrer ces inhibiteurs est un pré-requis avant tout traitement de substitution. Les inventeurs proposent donc d'exploiter les présents résultats dans le cadre de diagnostics d'anticorps inhibiteurs. Un test Bethesda (dosage du titre d'inhibiteurs) chez un patient hémophile peut-être réalisé avant et après passage sur un ELISA où l'antigène de capture correspondra aux variants de FVIII de la présente invention pris séparément ou bien combinés. Le titre d'inhibiteurs chutera obligatoirement pour le contrôle réalisé avec un FVIII sauvage. Le variant ou la combinaison de variants dont le titre d'inhibiteur reste inchangé est utilisé comme traitement pour le patient hémophile à inhibiteur. Ce diagnostic permet donc de contrôler et cibler la délivrance du variant de FVIII humain de la présente invention.

Ainsi, le présent document concerne une méthode de traitement comprenant :
un test de reconnaissance des anticorps inhibiteurs contenus dans un échantillon sérique du patient sur un ou plusieurs mutants FVIII selon la présente invention ;
la sélection du ou des mutants FVIII non-reconnu(s) par lesdits anticorps inhibiteurs ; et
l'administration d'un ou plusieurs des mutants FVIII sélectionnées en b).

De préférence, le test de reconnaissance entre l'échantillon du patient et le ou les variants FVIII selon la présente invention est réalisé par un test Bethesda. A titre de témoin, un test de reconnaissance est de préférence réalisé sur le FVIII naturel.

Le présent document concerne un kit diagnostic comprenant un ou plusieurs variants FVIII selon la présente invention.

Le présent document concerne également l'utilisation d'un ou plusieurs variants du FVIII humain ou d'un dérivé biologiquement actif de celui-ci selon la présente invention pour la préparation d'un médicament destiné au traitement de l'hémophilie de type A chez des patients à inhibiteurs ne présentant pas dans leur sérum d'anticorps reconnaissant ledit ou lesdits mutants du FVIII humain ou d'un dérivé biologiquement actif de celui-ci.

### Exemples

### Exemple 1 : Biologie moléculaire

L'ADN complémentaire du FVIII contenant deux introns tronqués du facteur IX en position 1 et 13 (5012 pb) (SEQ ID No 4) a été cloné entre les sites de restriction Not I et Xho1 dans un vecteur (pcDNA3.1 GS, Invitrogen) permettant l'expression de la protéine en cellules de mammifères. L'ensemble pcDNA/FVIII correspondait à un plasmide d'une taille de 10439 pb. Ce gène comprend les 5 domaines fonctionnels A1, A2, A3, C1, et C2 essentiels à l'activité du FVIII. Comme il a déjà été montré que le domaine B ne joue aucun rôle prédominant dans la fonction pro-coagulante du FVIII, les inventeurs ont choisi de produire le FVIII avec une délétion de ce domaine. Les zones codant pour les domaines A1 et A2 comprennent chacune un intron. L'insertion de ces deux régions introniques parmi les exons codants améliore significativement l'expression du FVIII humain. La séquence protéique codée par ce gène est décrite dans SEQ ID No 5.

La stratégie de mutagenèse a consisté en la génération de façon systématique de tous les variants Alanine simples des domaines ciblés du FVIII, c'est-à-dire A2, A3 et C2. Les variants ont été générés par la méthode de Massive Mutagenesis® décrite dans US2004/0048268.

Comme cité précédemment, il a été montré que les domaines A2, C2 et A3 sont les cibles privilégiées dans la reconnaissance du FVIII par les anticorps inhibiteurs. Chaque acide aminé de ces domaines fonctionnels a été substitué par une Alanine, hormis la partie intronique du domaine A2. Une série de 795 oligonucléotides (32 mers) a été élaborée et produite afin d'introduire la mutation Alanine sur les positions : i) 376 à 719 [A2] ; ii) 2173 à 2325 [C2] ; iii) 1691 à 2025 [A3]. Le système de numérotation des mutations du FVIII humain utilisé dans cette invention est celui défini par Wood et al. (312, 330 - 337, Nature, 1984). Après mutagenèse dirigée, les inventeurs ont contrôlé par deux séquençages successifs que chacun des mutants de la banque contenait la mutation Alanine à la position souhaitée. Cette collection de mutants Alanine réalisée sur les domaines C2, A2 et A3 du FVIII, correspond à la première banque systématique de mutants dirigés jamais réalisée sur cette molécule.

### Exemple 2 : Expression des mutants Alanine du FVIII humain en cellules de mammifères COS-7

Le FVIII est habituellement exprimé en cellules de mammifères (Toole et al., 312, 342-347, Nature, 1984 ; Gitschier et al., 312, 326-330, Nature, 1984 ; Wood et al., 312, 330-337, Nature, 1984 ; Vehar et al., 312, 337-342, Nature, 1984 ; WO8704187 ; WO 8808035 ; WO8803558 ; US4757006).

Afin de réaliser les transfections des cellules COS-7 par les constructions pcDNA /FVIII natif ou muté, ces cellules ont été trypsinisées lorsqu'elles atteignaient 90% de confluence. Les cellules COS-7 ont été réensemencées suivant le ratio ¼ (c'est-à-dire de façon à ce qu'elles représentent, une fois adhérées sur la surface, une confluence de 25% environ). La transfection des cellules COS-7 est transitoire et a été réalisée en plaques de culture 90 mm (6 ml par puit) lorsque les cellules atteignent 70-80% de confluence. La transfection a été réalisée avec environ 6µg d'ADN pour un volume de 18 µl de FuGENE-6 (Roche, Meylan, France)

Avant transfection, le FuGENE-6 a été dilué en milieu IMDM sans sérum et incubé à température ambiante pendant 5 min. Le mélange FuGENE-6/ADN a été laissé 15 minutes à température ambiante puis déposé sur les cellules en milieu complet en goutte à goutte. Un premier surnageant contenant le FVIII a été récupéré 24 heures après transfection, puis 6 ml de milieu frais ont été remis sur les cellules. Le surnageant de culture a été récupéré 48 heures plus tard (6 ml), centrifugé, aliquoté et stocké à -20°C dans l'attente du test d'activité coagulante (chromogénique). Le niveau d'expression moyen du FVIII sauvage a été estimé par ELISA (kit ELISA commercial Stago) et était compris entre 20 et 60 ng/ml.

Tous les réactifs de culture cellulaire ont été fournis par Invitrogen. Les cellules COS-7 (African green monkey SV40 transformed kidney cells) ont été cultivées dans des conditions standards de culture (37°C en atmosphère humide contenant 5% CO2) en utilisant le milieu Iscove's Modified Dulbecco's Medium (IMDM). L'IMDM a été supplémenté en un analogue de la L-glutamine (le glutamax), en sérum de veau foetal décomplémenté (10% final) et en antibiotiques à raison de 40 unités/ml de pénicilline et 0,1 mg/ml de streptomycine.

### Exemple 3 : Criblage primaire : Analyse fonctionnelle des mutants Alanine du FVIII humain

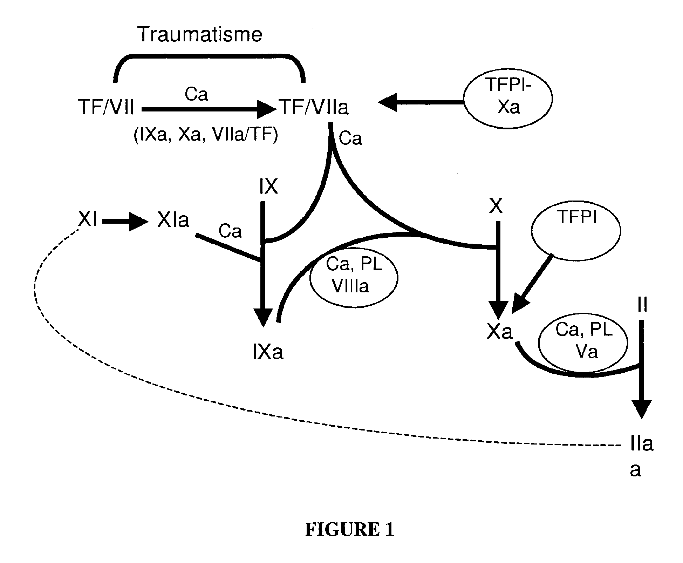

Le criblage primaire correspond à la mesure de l'activité coagulante brute (Figure 1) obtenue à partir d'un même volume de surnageant de culture de cellules COS-7. Deux tests différents de mesure de l'activité coagulante ont été utilisés lors du criblage primaire, le test chronométrique et le test chromogénique.

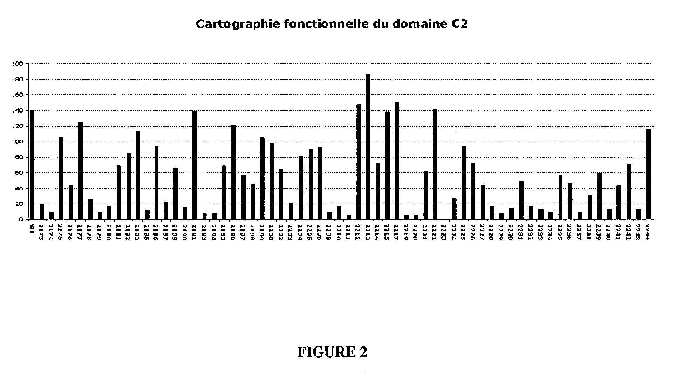

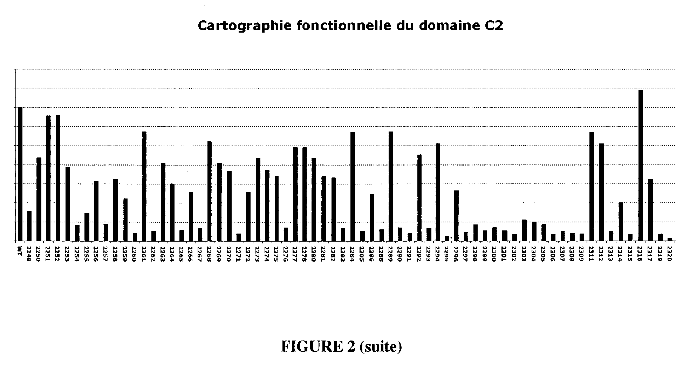

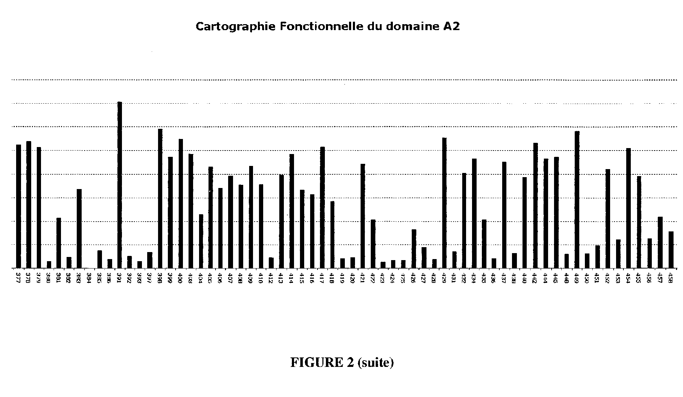

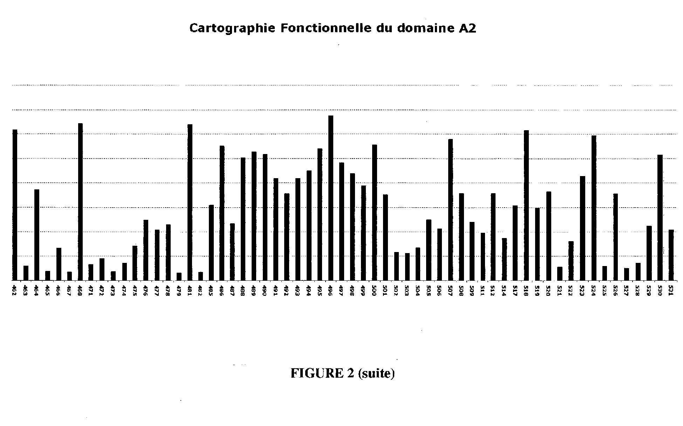

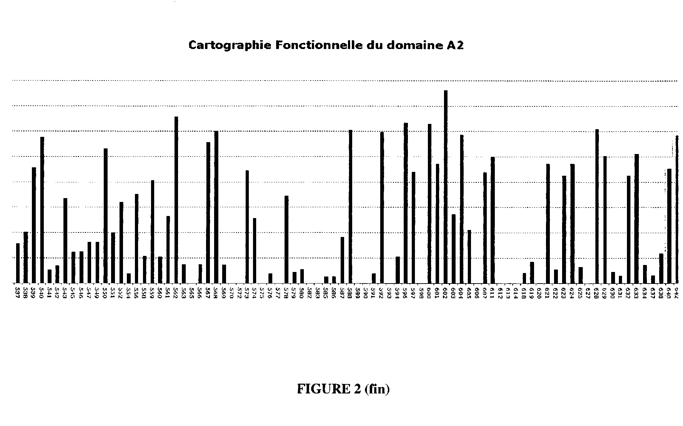

L'activité chronométrique est mesurée suite à l'incubation d'une dilution en tampon imidazole des molécules de FVIII à tester en présence de plasma déficient en FVIII (Stago). Le début de la coagulation est initié par l'ajout de calcium et la mesure du temps de formation du caillot est déterminée sur un automate MDA-II (Biomérieux, Marcy-l'étoile). L'activité coagulante des 795 mutants Alanine a été mesurée par le test chronométrique ; celui-ci a été réalisé sur une plateforme robotisée du Centre National du Traitement de l'Hémophilie (Hospices Civils de Lyon). L'activité chronométrique de l'ensemble des mutants Alanine a été rapportée à l'activité d'un FVIII sauvage utilisé comme étalon interne de chaque transfection. Les résultats de ces mesures d'activité brute rapportée à celle du FVIII non muté ont permis de définir deux catégories de mutants : i) les mutants ayant conservé au moins 50% de l'activité du FVIII sauvage ; ii) les mutants dont l'activité comparée à celle du FVIII sauvage est inférieure à 50%. La figure 2 présente l'activité coagulante de 359 mutants Alanine sur les 795 analysés. Ces données représentent une cartographie fonctionnelle de chacun de ces résidus du FVIII en terme d'activité coagulante ; une activité coagulante supprimée par une mutation vers un résidu Alanine démontre le caractère essentiel du résidu concernée pour l'activité du FVIII.

158 mutants ayant conservé une activité brute supérieure à 50% de celle du FVIII non muté ont été sélectionnés par ce test chronométrique pour le crible secondaire. Leurs activités ont tout d'abord été confirmées par le deuxième test d'activité coagulante, le test chromogénique comme mentionné plus haut. Ce test a également été réalisé sur une plateforme robotisée au Centre National du Traitement de l'Hémophilie (Hospices Civils de Lyon). La mesure de l'activité chromogénique des 158 mutants Alanine sélectionnés a été réalisée grâce à l'utilisation du kit Coamatic Factor VIII (Chromogenix, Instrumentation Laboratory, Milan, Italie) en suivant les indications du fournisseur. Brièvement, les surnageants de culture (50 µl) ont été dilués dans le tampon de dilution fourni et préincubés 4 minutes à 37°C. Le milieu réactionnel (50 µl), préchauffé à 37°C, a été ensuite ajouté durant 4 minutes, puis 50 µl de milieu de révélation à 37°C ont été ajoutés. L'apparition du produit au cours du temps a immédiatement été mesuré à 405 nm dans un spectrophotomètre après agitation de la microplaque. L'apparition du produit est donnée en mUDO/min. Lorsque les valeurs sont supérieures à 200 mUDO/min, une dilution supérieure a été appliquée et le dosage re-effectué.

Le tableau 1 présente les activités des 158 mutants ayant conservé une activité brute supérieure à 50% de celle du FVIII non muté. Ces 158 mutants ont été retenus afin d'être testés en crible secondaire.

### Exemple 4: Crible secondaire : Evaluation de la perte d'antigénicité pour les anticorps inhibiteurs du FVIII humain

Le crible secondaire correspond à un test semblable au test Bethesda, réalisé comme suit sur les 158 mutants sélectionnés à l'issue du crible primaire ; ce test comprend une étape de mise en présence d'un sérum (ou d'anticorps) inhibiteur avec une molécule de FVIII à tester ou un étalon référent et une étape de mesure d'activité coagulante du FVIII par test chronométrique :
Les surnageants de culture obtenus après 48h de présence avec les cellules COS transfectées par différentes constructions de FVIII ont été utilisés. Ces surnageants ont été produits en milieu complet (IMDM (Invitrogen), 10 % sérum de veau foetal, 2 mM L-glutamine, 100 U/ml pénicilline, 100 mg/ml streptomycine). Les surnageants ont été dilués avec du milieu complet frais pour obtenir une activité chronométrique finale comprise aux alentours de 10-20 % (1 unité FVIII = 100% d'activité = 200 ng/ml). Le surnageant de culture dilué ou non (140 µl) a été ajouté à 150 µl de plasma humain dépleté en FVIII (Stago, Asnières, France). Une dilution d'anticorps (10 µl) a ensuite été ajoutée au mélange. Ces anticorps sont des fractions IgG purifiées sur protéine A issus de patients hémophiles avec inhibiteurs. Une fraction IgG d'un témoin non-hémophile a été obtenue de manière similaire. Les titres Bethesda des anticorps inhibiteurs sont identiques à l'activité inhibitrice issu du plasma. Le protocole de purification ne dénature donc pas l'activité inhibitrice des anticorps. Ceux-ci ont été préalablement dilués en milieu complet frais, la mesure étant faite soit avec une dilution fixe d'anticorps soit avec une série de dilutions. La concentration fixe d'anticorps utilisée a été celle ayant permis d'inhiber 50 % d'une solution de FVIII recombinant standard à 12,5 % d'activité. Les échantillons ont été incubés 1h30 à 37°C au bain-marie. L'activité coagulante a été ensuite mesurée sur un automate de type MDA-II (Biomérieux, Marcy-l'Etoile). L'activité coagulante a été rapportée à celle d'une gamme étalon établie à partir d'un FVIII identique produit de manière stable par la lignée CHO. Les résultats sont présentés sous forme d'un pourcentage qui représente la levée de l'inhibition de l'activité coagulante d'un mutant donné par les anticorps inhibiteurs d'un sérum de patient. Ce pourcentage a été calculé tel que montré dans la figure 5 pour le mutant FVIII E518A. L'expression de la levée d'inhibition est un pourcentage = - [(b-a)/a] x 100 avec a = pourcentage d'activité résiduelle du WT (sérum + IgG / sérum - IgG) et b = pourcentage d'activité résiduelle du mutant (sérum + IgG / sérum - IgG).

Le tableau 2 présente les pourcentages de levée d'inhibition pour 30 mutants simples sur 5 sérums de patients hémophiles. Ces mutants ont été sélectionnés à l'issue du crible secondaire réalisé sur les 158 mutants issus du crible primaire. Plusieurs mutants montrent des pourcentages de levée d'inhibition significatifs sur certains sérums tels que le mutant 2316 face aux sérums TD et SL, le mutant 2294 face au sérum GC, le mutant 403 face au sérum FS ou le mutant 2275 face au sérum PR.

Les sérums de patients ont été sélectionnés pour leurs titres Bethesda élevés (supérieurs à 10UB) et leurs profils d'inhibiteurs différents. Ces patients ne peuvent plus être traités par des injections de FVIII et doivent avoir recours à des bypassing agents. Ainsi, l'obtention de mutants Alanine du FVIII permettant de lever, même partiellement, l'inhibition de l'activité du FVIII par les anticorps inhibiteurs d'un de ces patients, constitue une avancée très importante dans les possibilités futures de traitement des patients hémophiles à inhibiteurs. Les différentes données accumulées sur un grand nombre de mutants ainsi que les différents sérums testés vont permettre de générer des combinaisons de mutations aboutissant à un FVIII amélioré pouvant échapper à une majorité d'anticorps inhibiteurs tout en conservant son activité pro-coagulante.

La reproductibilité du niveau d'expression du FVIII en fonction des transfections a été vérifiée par le suivi de l'activité spécifique du FVIII sauvage. En effet, les activités spécifiques calculées à partir des mesures d'antigènes (kit ELISA commercial Stago) ont été identiques pour les FVIII sauvages issus de différentes transfections. De la même façon, la concentration en antigène a été mesurée pour les mutants ayant conservé au moins 50% de l'activité du FVIII sauvage et leur activité spécifique en a été déduite. L'activité spécifique correspond à l'activité brute obtenue par test chromogénique (mUDO/min) rapportée à la concentration en protéine (ng/ml) obtenue à l'aide d'un ELISA (Kit FVIII de Stago). Des données comparant les activités brutes et spécifiques des 30 mutants retenus à l'issue du crible secondaire sont données dans le tableau 3.

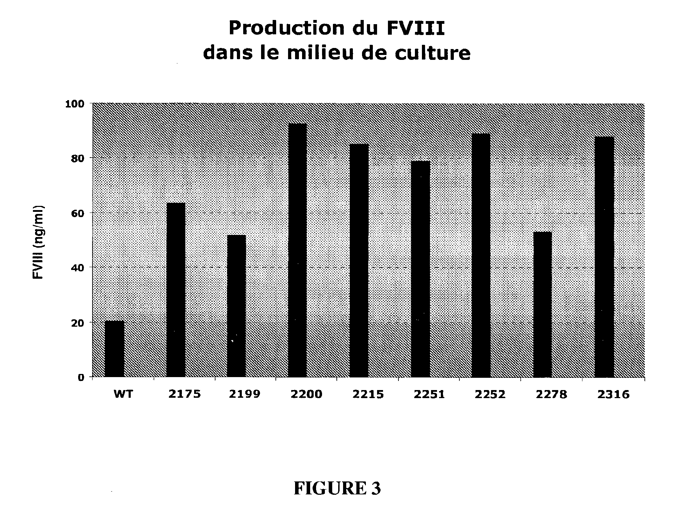

Les huit mutants 2175, 2199, 2200, 2215, 2251, 2252, 2278 & 2316 Alanine du FVIII ont montré une capacité très supérieure à la moyenne à être sécrété dans le milieu de production des cellules COS utilisées dans le cadre de cette invention. La figure 3 présente les données obtenues pour ces huit mutants. L'activité coagulante brute de ceux-ci a été déterminée par test chromogénique. La concentration obtenue pour ceux-ci est environ de 2 à 4 fois supérieure à celle du FVIII sauvage. Cette propriété est intéressante en terme de production de FVIII recombinant ; elle pourrait permettre d'abaisser les coûts de production d'un FVIII de nouvelle génération. Egalement, elle peut s'avérer avantageuse dans le cadre d'une thérapie génique appliquée à des patients hémophiles. Par ailleurs, ces mutations qui confèrent une capacité supérieure à être sécrétée peuvent s'avérer très intéressantes en combinaison avec les mutations qui engendrent une levée de l'inhibition par les anticorps inhibiteurs, en permettant, par exemple, de compenser une éventuelle perte relative de sécrétion de ces mutants moins antigéniques.

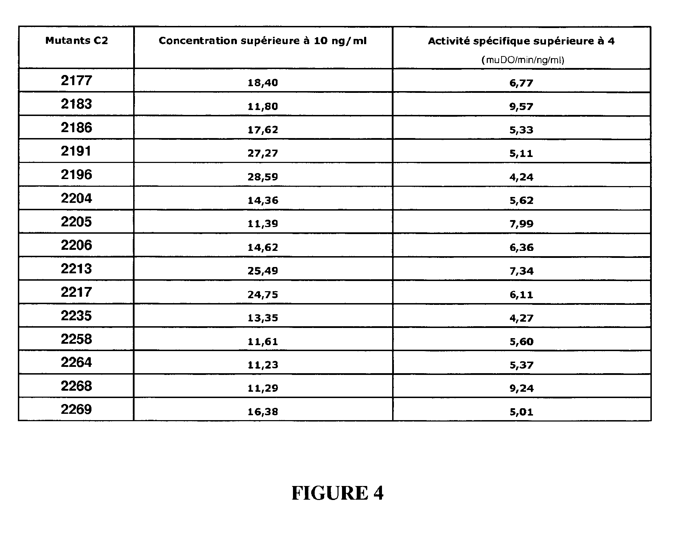

Les 15 mutants 2177, 2183, 2186, 2191, 2196, 2204, 2205, 2206, 2213, 2217, 2235, 2258, 2264, 2268 et 2269 ont montré une activité spécifique très supérieure à celle du FVIII sauvage, tout en maintenant un niveau de production élevé, proche de celui du FVIII sauvage (concentration supérieure à 10 ng/ml). Les activités spécifiques de ces 15 mutants sont montrées dans la figure 4. L'activité coagulante brute de ces mutants a été déterminée par test chromogénique. Cette propriété est intéressante dans la mesure où elle permettrait des doses d'injection de FVIII aux patients, plus faibles ou plus espacées dans le temps. Par ailleurs, ces mutations qui confèrent une activité spécifique supérieure peuvent s'avérer très intéressantes en combinaison avec les mutations qui engendrent une levée de l'inhibition par les anticorps inhibiteurs, en permettant de compenser une éventuelle perte relative d'activité de ces mutants moins antigéniques.

### Exemple 5 : Sélection et combinaison des meilleurs mutants simples du crible secondaire

Parmi les 30 mutants simples qui ont été sélectionnés à l'issue du crible secondaire, 8 d'entre eux ont été choisis pour que leurs mutations respectives soient combinées, l'objectif étant d'obtenir un effet cumulatif / additif des propriétés remarquables de chacune. Les critères de sélection de ces mutants ont été complexes et ont tenu compte des paramètres suivants :
- levée d'inhibition d'au minimum 25% pour au moins l'un des sérums testés provenant de patients hémophiles à inhibiteurs ;
- activité brute de coagulation au minimum 100% par rapport au FVIII non muté ; et,
- bon niveau d'expression de manière répétée.

Les 8 mutants sélectionnés sont les mutants 409, 462, 507 et 629 provenant du domaine A2 et les mutants 2289, 2294, 2312 et 2316 provenant du domaine C2. Comme mentionné précédemment, le critère de sélection a tenu compte d'une activité spécifique (activité de coagulation rapportée à l'expression) élevée, comme montré dans le tableau 3. Ce niveau d'activité spécifique doit également être constant au cours des différentes manipulations.

Les 28 doubles mutants résultants de la combinaison des 8 mutations simples 409, 462, 507, 629, 2289, 2294, 2312 et 2316 (6 doubles mutants A2 + 6 doubles mutants C2 + 16 doubles mutants A2-C2 présentés dans le tableau 4) ont été construits par des méthodes de mutagénèse connues de l'homme du métier. L'expression transitoire de ces mutants a été réalisée en cellules de mammifères COS-7 comme décrit dans l'exemple 2. Leur niveau d'expression et leur niveau d'activité ont été mesurés de la manière décrite dans les exemples précédents respectivement par test ELISA et activité chromogénique (mUDO/ng/ml). Ces 28 mutants ont été évalués à leur tour pour leur résistance à l'inhibition par des anticorps de patients hémophiles. Les doubles mutants A2 présentent une levée d'inhibition significative pour un ou l'ensemble des anticorps issus des sérum de patients, alors que les combinaisons contenant des mutations du domaine C2 (6 doubles mutants C2 + 16 doubles mutants A2-C2) présentent une levée d'inhibition négligeable voire inexistante.

Le tableau 5 présente les activités spécifiques des 6 doubles mutants A2 et leurs pourcentages de levée d'inhibition face à 4 sérum de patients hémophiles TD, GC, SL et PR calculés de la même manière que dans l'exemple 4. Les doubles mutants particulièrement préférés résistent de façon significative à 3 anticorps de patients sur 4 au minimum. On note ici l'effet cumulatif des 4 mutations simples du domaine A2. Le choix s'est donc porté sur la combinaison des 4 mutations 409, 507, 462, et 629. Les mutants triples et le mutant quadruple composés de ces 4 mutations 409, 507, 462, et 629 ont également été construits.

### Exemple 6 : Construction et caractérisation d'un mutant quadruple (FVIII-4A2)

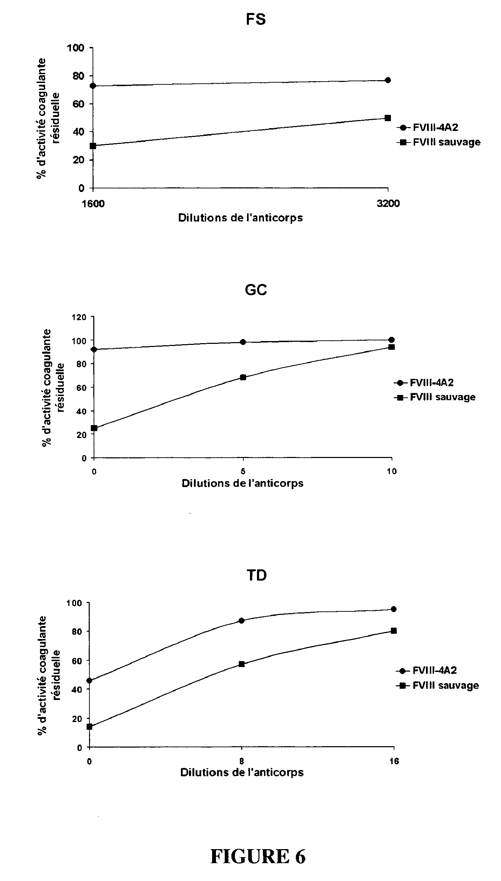

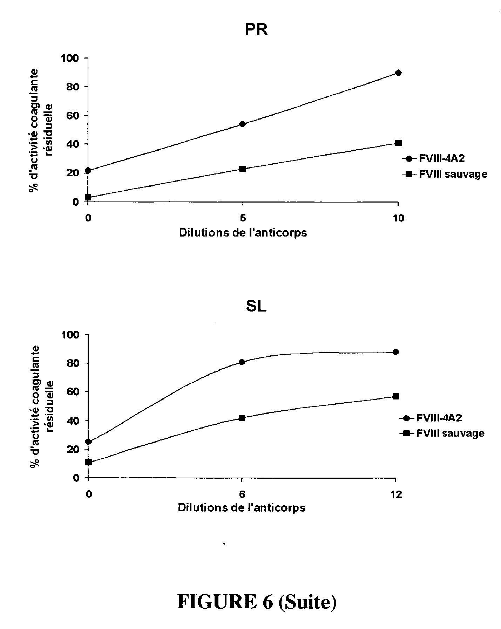

Le mutant quadruple issu de la combinaison des 4 mutations de A2 retenues, 409, 462, 507, 629 a été construit par une méthode de mutagénèse classique connue de l'homme du métier. Le mutant quadruple a été produit dans une lignée CHO dont l'obtention est détaillée dans l'exemple 9. Ce mutant a été également caractérisé pour sa résistance à l'inhibition par des anticorps de 5 patients hémophiles FS, TD, GC, PR et SL. L'activité résiduelle mesurée après incubation avec un anticorps inhibiteur est divisée par l'activité restante après une incubation avec l'anticorps non-immun. Le pourcentage d'activité résiduelle est ainsi calculé et présenté sur les courbes de la figure 6. Ces courbes présentent l'activité résiduelle de FVIII-4A2 après sa mise en contact avec différentes dilutions d'anticorps issus des différents patients à inhibiteurs. Il apparaît clairement que l'utilisation du mutant FVIII-4A2 permet de conserver une activité chronométrique beaucoup plus importante suite à une incubation avec des anticorps inhibiteurs. Ainsi, des gains d'activité résiduelle pour les plus fortes concentrations en anticorps inhibiteurs vont de 230 à 450 %, ce pourcentage d'activité résiduelle dépendant à la fois de l'origine de l'anticorps inhibiteur et de la concentration utilisée.

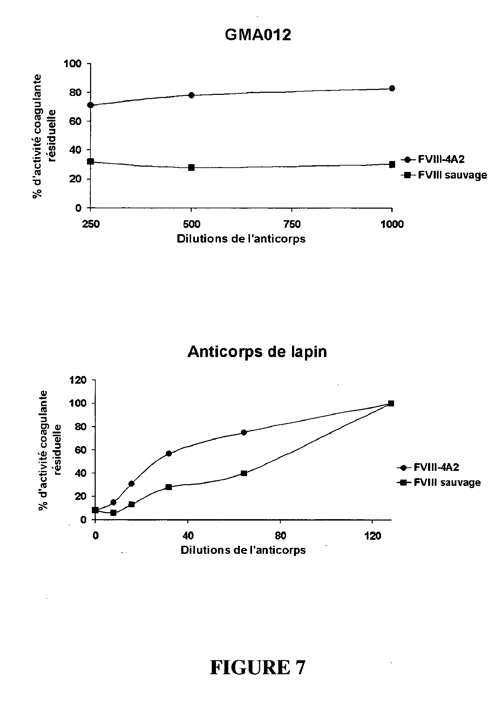

Afin de déterminer si la liaison directe des anticorps à FVIII-4A2 est affectée, trois anticorps supplémentaires ont été utilisés avec le même protocole que précédemment à la place des sérums de patients : un anticorps anti-domaine A2 (GMA012, Green Mountain Antibodies), un anticorps anti-domaine C2 (ESH4, American Diagnostica) et un anticorps polyclonal de lapin, purifiés suivant le même protocole que les anticorps de patient. Les résultats de ces contrôles sont montrés dans la figure 7 pour les deux anticorps anti-domaine A2, anticorps polyclonal de lapin et GMA012. Clairement, les modifications introduites touchant le domaine A2 dans FVIII-4A2 font que FVIII-4A2 échappe à l'anticorps anti-domaine A2, GMA012 et à l'anticorps polyclonal de lapin (montré). En revanche aucune différence significative de l'inhibition de FVIII-4A2 par rapport à un FVIII sauvage n'est observée pour ESH4 (non montré). Ces résultats corroborent les résultats de levée d'inhibition montrant d'une part que les mutations introduites dans le domaine A2 permettent l'échappement aux anticorps des patients et d'autre part que le domaine C2 de FVIII-4A2 est intact puisque reconnu similairement au FVIII sauvage. Ce dernier point est important pour l'activité de FVIII-4A2 puisque le domaine C2 est celui qui est responsable des interactions avec le facteur Von Willebrand et avec les cofacteurs nécessaires à la pleine activité du FVIII (liaison aux phospholipides et au calcium).

### Exemple 7 : Caractérisation du mutant FVIII-4A2

### a) ELISA

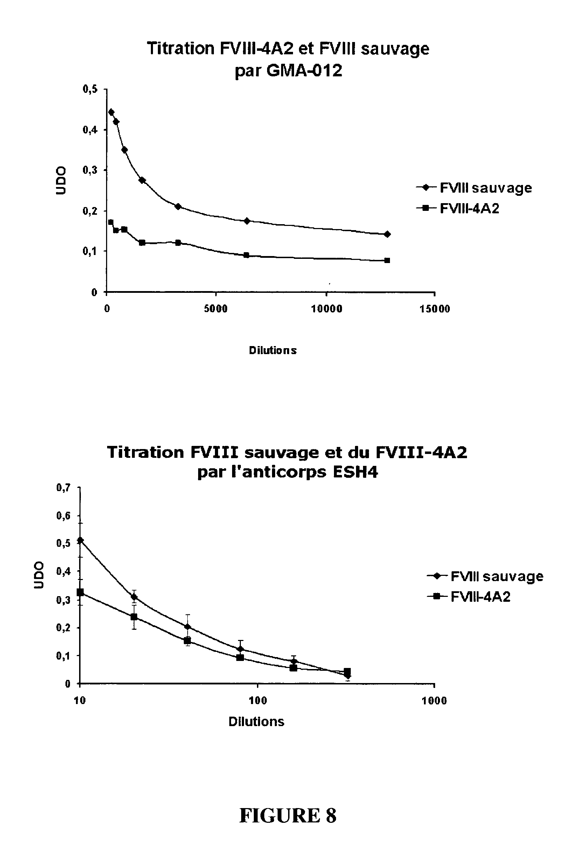

FVIII-4A2 est produit par la même lignée cellulaire CHO que le FVIII sauvage, selon le protocole détaillé ci-après (exemple 9). Il est purifié par le même procédé (protocole également détaillé ci-après, exemple 9) et lui a été donc comparé lors des analyses fonctionnelles. La concentration de FVIII-4A2 est mesurée par l'intermédiaire d'un kit ELISA (cf. protocole ci-dessous). Afin de s'assurer que les mutations introduites ne perturbent pas la quantification du FVIII muté par ce kit, des contrôles supplémentaires ont été réalisés avec une batterie d'anticorps monoclonaux. Ainsi, des concentrations similaires de FVIII sauvage et FVIII-4A2 sont reconnues de manière identique par l'anticorps ESH-4 dirigé contre le domaine C2 de la chaîne légère. Conformément aux mesures de levée d'inhibition, on constate une diminution importante de la reconnaissance de FVIII-4A2 par l'anticorps GMA-012 comparé au FVIII sauvage. Ces résultats sont présentés dans la figure 8.

Le protocole des tests ELISA nécessaires à ces expériences est le suivant :
Pour fixer le produit d'intérêt sur les parois de la plaque ELISA (Nunc Maxisorb), le réactif est dilué au moins 5 fois en 50 mM CAPS pH 9,0 et est incubé la nuit à 4°C. Le puits est ensuite lavé 2 fois en tampon TBS-T (50 mM Tris-HCl pH 8,0, 100 mM NaCl, 5 mM MgCl₂, 0,01 % Tween 20, 0,05 % BSA). Le puits est ensuite saturé durant 1h par TBS-3% BSA (50 mM Tris-HCl pH 8,0, 100 mM NaCl, 5 mM MgCl₂, 0,01% Tween-20, 3% BSA). Le réactif interagissant avec celui fixé sur la plaque est ensuite dilué en TBS-3% BSA puis incubé 1h30 à température ambiante. Trois lavages en TBS-T sont ensuite réalisés. Les anticorps primaires et secondaires couplés à la peroxydase du radis noir (HRP) sont dilués en TBS-3% et ajoutés respectivement 1h30 à température ambiante. Les anticorps secondaires sont dilués au 2000^{ème}. Entre les deux incubations d'anticorps trois lavages en TBS-T sont effectués. Ils sont répétés avant l'ajout du substrat, un mélange OPD/urée (Sigma). La réaction enzymatique est arrêtée par 2,5 M H₂SO₄. La densité optique est mesurée à 490 nm.

### b) Mesure de l'activité spécifique

L'activité spécifique du mutant FVIII-4A2 est établie en divisant l'activité chromogénique par la concentration. Ces activités spécifiques sont comparées avec celle du type sauvage. L'activité chromogénique du FVIII sauvage est d'environ 15 +/- 1 UDO/min.µg. et celle de FVIII-4A2 est de l'ordre de 27 +/- 11 UDO/min.µg soit une activité supérieure.

### c) Activation par la thrombine

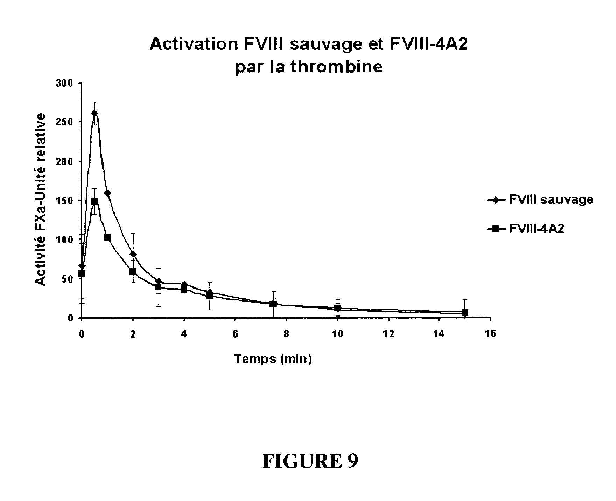

Les facteurs VIII sauvage et FVIII-4A2 (0,125 U ou 25 ng) sont dilués dans un tampon 40 mM HEPES, 100 mM NaCl, 5 mM CaCl2 contenant 10 µM d'un mélange Phosphatidylcholine : Phosphatidylsérine (80 : 20) et 0,1 mg/ml BSA puis incubés 5 min à 37°C. La thrombine (0,05 U) est ajoutée et son action est mesurée à différents temps. A chaque temps un aliquot est prélevé et incubé avec un mélange d'hirudine (0,5U), de facteur IXa (50 nM) et facteur X (200 nM) dilués dans le même tampon que précédemment. Le FXa est alors généré. Le substrat du FXa pNAPEP-25 est ajouté immédiatement et l'apparition du produit chromogène est mesuré à 405 nm. La vitesse initiale est mesurée et la quantité de FXa générée par minute est calculée. Les FVIII sauvage et FVIII-4A2 présentent un profil de réponse à la thrombine identique, avec une augmentation rapide de l'activité FVIII qui culmine 1-2 min après l'ajout de la thrombine, suivi d'une diminution rapide de cette activité avec une demi-vie de l'ordre de 2 à 3 min. Les résultats présentés dans la figure 9 indiquent que FVIII-4A2 est reconnu par la thrombine de manière identique au FVIII sauvage avec une baisse relative d'activité dont pourrait être responsable une des 4 mutations.

### d) Dissociation du domaine A2

Les FVIII sauvage et FVIII-4A2 sont activés comme décrit précédemment pendant 1 min. L'hirudine est alors ajoutée et le FVIIIa est laissé pendant différents temps à 37°C. Des aliquots sont prélevés à ces temps et incubés dans le mélange des phospholipides, FIXa et FX. La génération de FXa est réalisée durant 5 min. Un tampon Stop est alors ajouté (Tris 50 mM pH 8,8; NaCl 475 mM, EDTA 9 mM). La quantité de FXa générée est mesurée comme décrite.

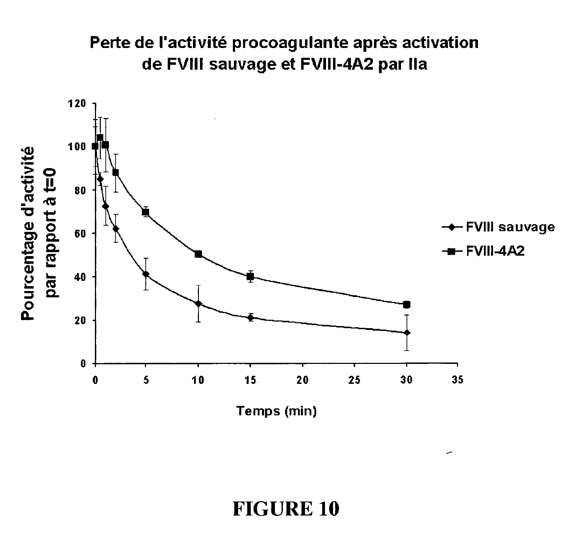

Le FVIIIa est incubé différents temps avant de mesurer son activité résiduelle. La perte d'activité mesurée au cours du temps correspond à la dissociation du domaine A2. Les FVIII sauvage et FVIII-4A2 ont des pertes d'activité dont le profil est similaire mais dont les cinétiques divergent. En effet la demi-vie du FVIII sauvage est de 3 min alors que celle de FVIII-4A2 est de 11 min. Cette augmentation de stabilité peut expliquer l'augmentation de l'activité spécifique déterminée en essai chromogénique. Lors de cet essai, le FVIIIa est incubé 4 min avant de mettre le substrat. Le FVIII sauvage perd ainsi plus rapidement son activité durant cet essai que le FVIII-4A2. Ces résultats sont présentés dans la figure 10.

### Exemple 8 : Construction et caractérisation des mutants FVIII-3A2

Les 4 triples mutants FVIII-3A2 ont été construits : FVIII-3A2 (409-462-507), FVIII-3A2 (462-507-629), FVIII-3A2 (409-462-629), FVIII-3A2 (409-507-629).

Mesure de l'activité spécifique de FVIII-3A2 (409-462-507)

L'activité spécifique du mutant FVIII-3A2 (409-462-507) est établie en divisant l'activité chromogénique ou l'activité chronométrique par la concentration. Ces activités spécifiques sont comparées avec celle du FVIII sauvage. L'activité chromogénique du FVIII-3A2 (409-462-507) correspond à 98% de l'activité chromogénique du FVIII sauvage. Ces résultats démontrent que l'absence de mutation au niveau de la position 629 permet d'obtenir une activité de coagulation plus importante pour FVIII-3A2 que pour FVIII-4A2.

### Résistance à l'inhibition de FVIII-3A2 (409-462-507)

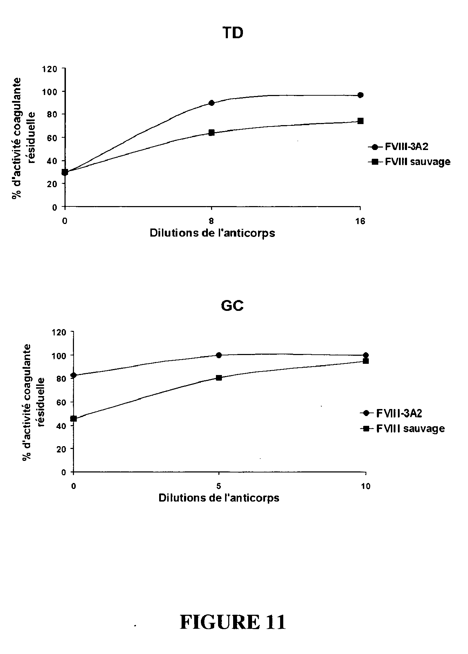

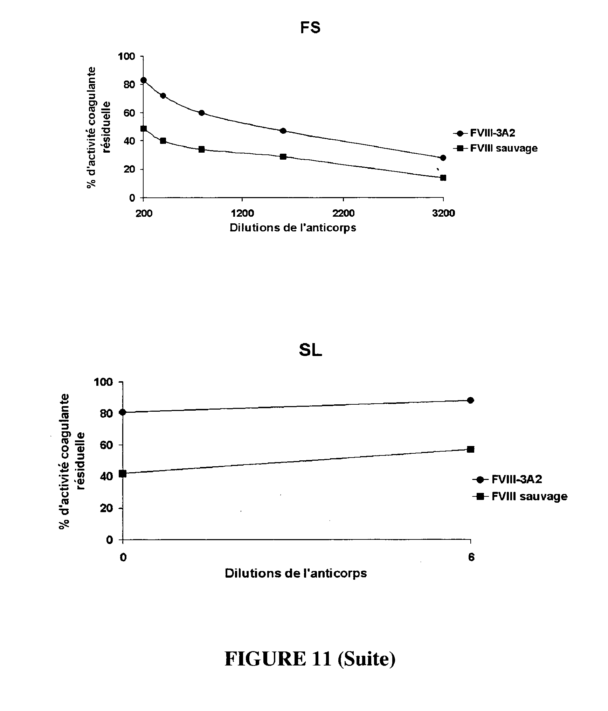

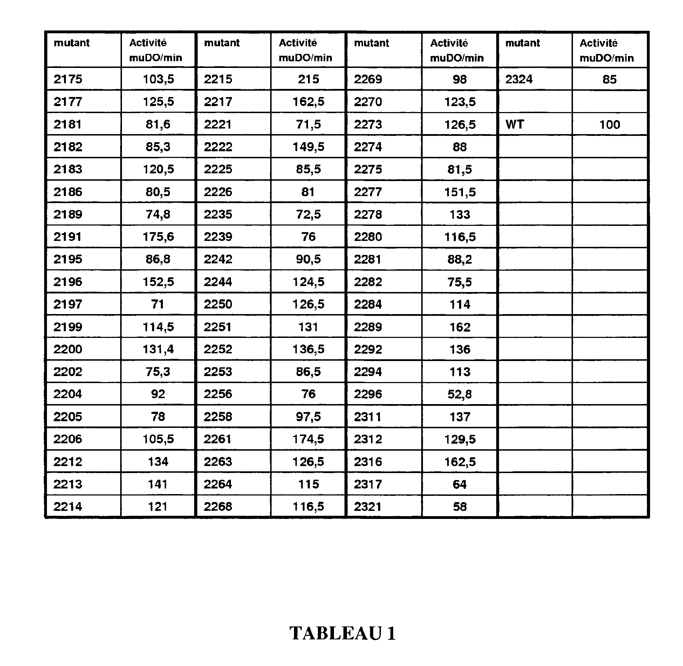

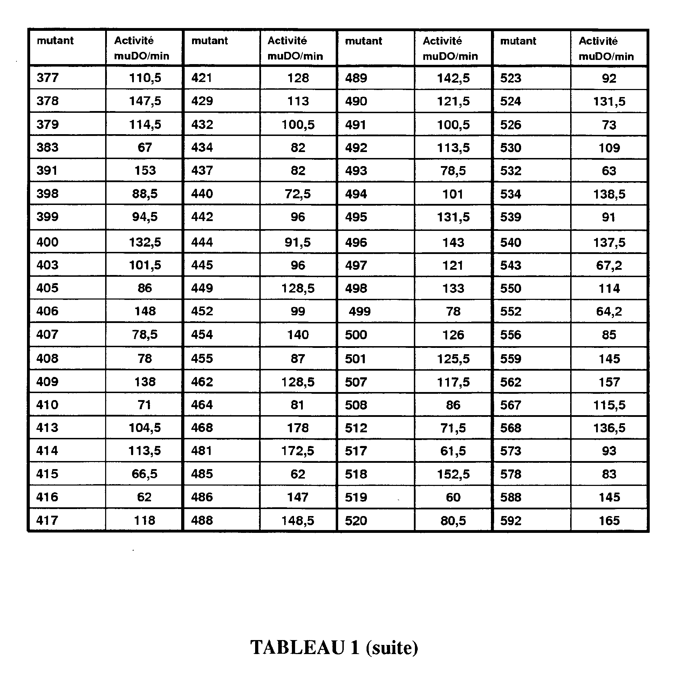

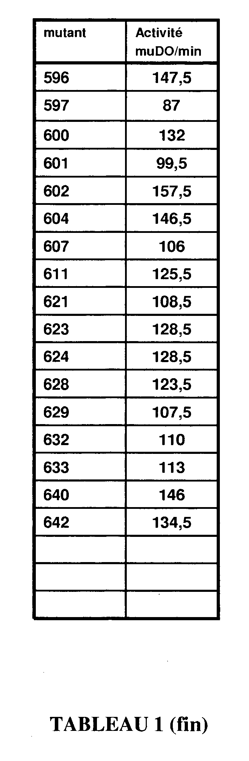

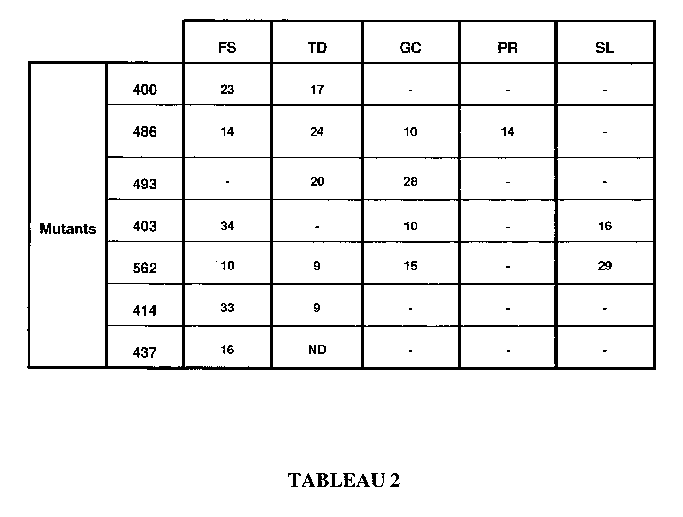

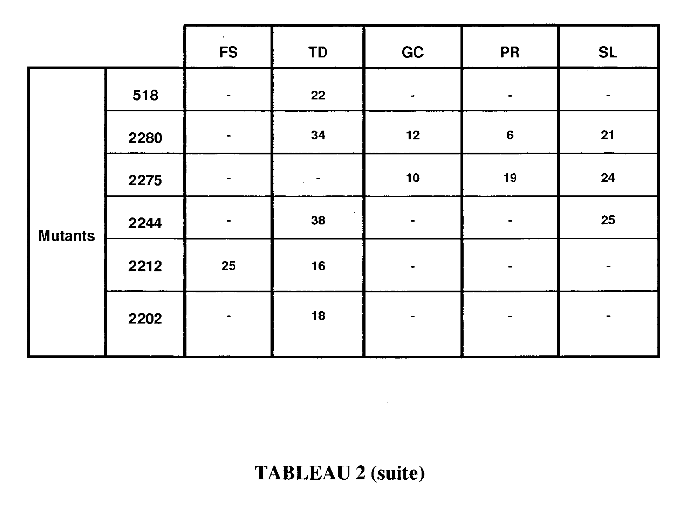

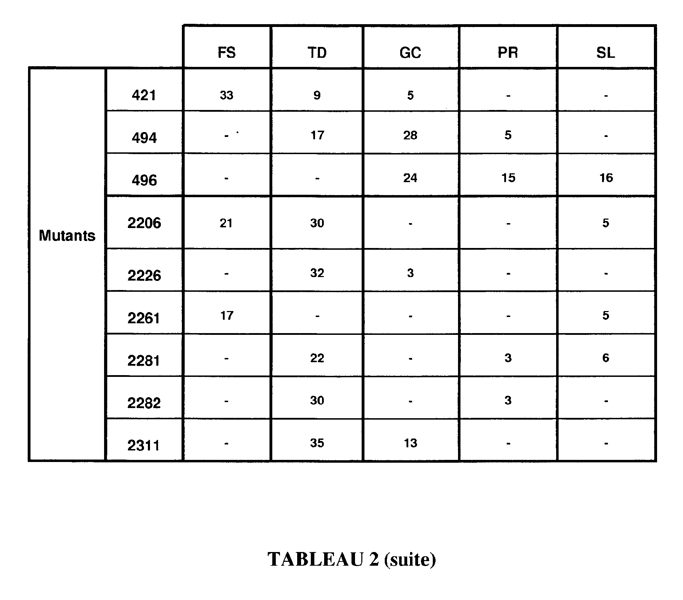

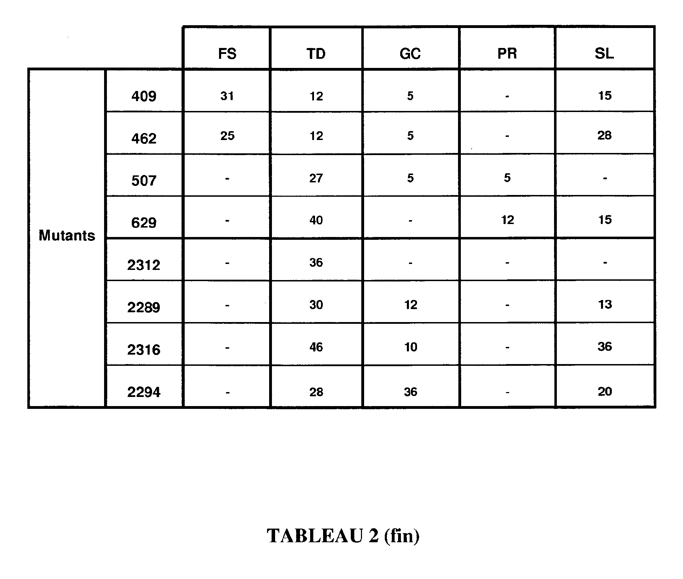

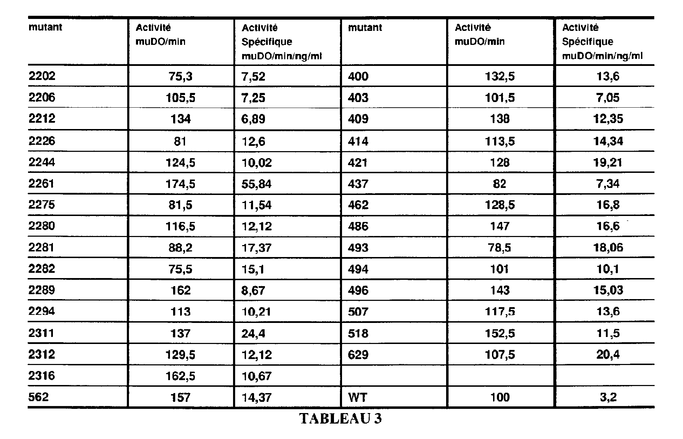

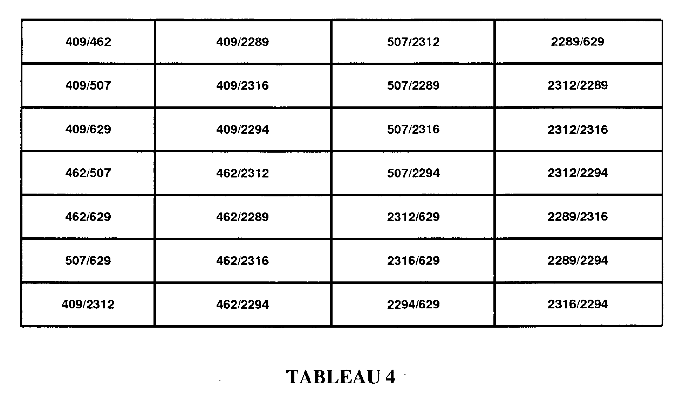

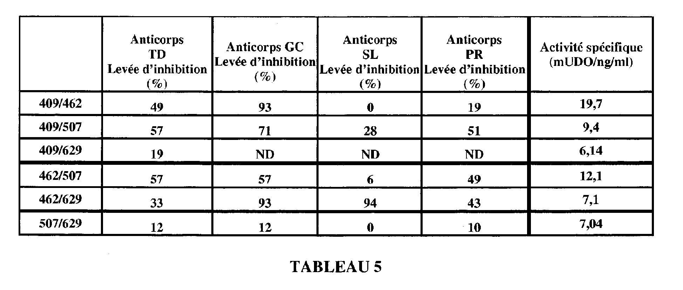

Ce mutant a été également caractérisé pour sa résistance à l'inhibition par des anticorps de 4 patients hémophiles FS, TD, GC et SL. L'activité résiduelle mesurée après incubation avec un anticorps inhibiteur est divisée par l'activité restante après une incubation avec l'anticorps non-immun. Le pourcentage d'activité résiduelle est ainsi calculé et présenté sur les courbes de la figure 11. Ces courbes présentent l'activité résiduelle de FVIII-3A2 (409-462-507) après sa mise en contact avec différentes dilutions d'anticorps issus des différents patients à inhibiteurs. Il apparaît clairement que l'utilisation du mutant FVIII-3A2 (409-462-507) permet de conserver une activité chronométrique beaucoup plus importante suite à une incubation avec des anticorps inhibiteurs. La combinaison des mutations 409-462-507 permet donc d'obtenir une levée d'inhibition plus importante se traduisant par un gain d'activité résiduelle. Ce pourcentage d'activité résiduelle dépend à la fois de l'origine de l'anticorps inhibiteur et de la concentration utilisée.

### Exemple 9 : Production d'une lignée CHO exprimant le FVIII-4A2 et purification / production du FVIII

### Production de la lignée CHO

Une lignée CHO (ECACC 85050302) exprimant le FVIII a été générée comme décrit dans Plantier et al. Thrombosis and Haemostasis 2001 86 p596. Brièvement, les cellules sont maintenues à 37°C sous 5% de CO₂ en atmosphère humide. Elles croissent en IMDM contenant 10 % de sérum de veau foetal et 1% de Penicilline-Streptomycine. Les cellules (7x10⁶) trypsinées et resuspendues en PBS sont éléctroporées en présence d'un ADNc d'intérêt (7 µg). Elles sont ensuite réensemencées en présence de Généticine (0,6 mg/ml). Après sélection, des clones individuels sont repiqués et amplifiés. Leur capacité à synthétiser du FVIII est déterminée par la mesure de l'activité chromogénique du milieu de culture. Les meilleurs clones producteurs sont amplifiés et mis à produire en triple-flasque. La production se déroule durant 5 jours pendant lesquels les cellules sont incubées durant la journée en milieu complet, lavées trois fois puis incubées pendant la nuit en milieu IMDM contenant 1% de BSA à la place du sérum. Le milieu avec BSA est récolté, centrifugé à 2500 rpm pendant 10 min à 4°C et stocké à -30°C. Les cellules sont remises en milieu complet pour la journée.

### Purification et production des mutants FVIII (FVIII-3A2 et FVIII-4A2)

Le protocole de purification est basé sur la technique décrite par Jenkins et al. (Blood 2004). Le milieu de culture est décongelé et 40 % (pN) de (NH₄)₂SO₄ sont ajoutés. Le milieu est laissé sur la nuit à 4°C sous agitation. Il est ensuite centrifugé à 14000 rpm durant 30 min à 4°C. Le culot est repris par 1/10^{ème} du volume en tampon 20 mM MES pH 6,0, 100 mM NaCl, 5 mM CaCl₂, 0,01 % Tween-20 et dialysé sur la nuit contre un tampon similaire mais contenant 200 mM NaCl.Le dialysat est centrifugé 10 min à 13000 rpm à température ambiante puis chargé à 2 ml/min sur une colonne de FPLC Sepharose FF. La colonne a été préalablement équilibrée dans le même tampon. Le FVIII est élué par un gradient de NaCl de 0,2 à 1M. Les fractions contenant l'activité chromogénique la plus forte sont mises en commun et dialysées contre un tampon 50 mM HEPES pH 7,4, 100 mM NaCl , 5mM NaCl et 0,01 % Tween-20. Le dialysat est ensuite aliquoté et stocké à-80°C. La qualité de la protéine est évaluée après migration en SDS-PAGE 10% acrylamide par coloration au nitrate d'argent et en immunoblot. La concentration du FVIII est mesurée par l'intermédiaire du kit Asserachrom FVIII :Ag (Stago, Asnières, France).

## Revendications

1. Variant de FVIII humain ou d'un dérivé biologiquement actif de celui-ci conservant une activité pro-coagulante du FVIII humain, **caractérisé en ce qu'**il comprend une substitution du résidu en position 462 du domaine A2, la position étant indiquée dans la SEQ ID No 3, et **en ce que** ledit variant conserve une activité pro-coagulante au moins égale à 50 % celle du FVIII humain naturel et présente une diminution de l'antigénicité vis-à-vis des anticorps inhibiteurs en comparaison du FVIII humain naturel.

2. Variant selon la revendication 1, **caractérisé en ce qu'**il comprend une unique substitution.

3. Variant selon la revendication 1, **caractérisé en ce qu'**il comprend en outre une substitution d'au moins un acide aminé sélectionné parmi le groupe constitué du résidu en position 2202 du domaine C2 et du résidu en position 437 du domaine A2, les positions étant indiquées dans la SEQ ID No 3.

4. Variant selon la revendication 1, **caractérisé en ce qu'**il comprend ou présente une combinaison de deux substitutions sélectionnées parmi le groupe consistant en 409 + 462, 462 + 507 et 462 + 629, les positions étant indiquées dans la SEQ ID No 3.

5. Variant selon la revendication 1, **caractérisé en ce qu'**il comprend ou présente une combinaison de trois substitutions sélectionnées parmi le groupe consistant en 409 + 462 + 507, 462 + 507 + 629 et 409 + 462 + 629, les positions étant indiquées dans la SEQ ID No 3, de préférence une combinaison de quatre substitutions sur les positions 409, 462, 507 et 629, les positions étant indiquées dans la SEQ ID No 3.

6. Variant selon les revendications 1-5, **caractérisé en ce qu'**il comprend en outre une substitution d'au moins un acide aminé sélectionné parmi le groupe constitué des résidus en positions 2177, 2183, 2186, 2191, 2196, 2204, 2205, 2213, 2217, 2235, 2258, 2264, 2268 et 2269 du domaine C2, les positions étant indiquées dans la SEQ ID No 3.

7. Variant selon les revendications 1-6, **caractérisé en ce qu'**il comprend en outre une substitution d'au moins un acide aminé sélectionné parmi le groupe constitué des résidus en positions 2175, 2199, 2200, 2215, 2251, 2252 et 2278 du domaine C2, les positions étant indiquées dans la SEQ ID No 3.

8. Variant selon l'une quelconque des revendications 1-7, **caractérisé en ce que** la séquence polypeptidique du variant diffère de la séquence SEQ ID No 3 par 1, 2, 3, 4, 5, 6, 7, 8, 9, 10, 11, 12, 13, 14 ou 15 substitutions, sans tenir compte d'éventuelle(s) délétion(s) ou troncature(s), de préférence l'acide aminé étant substitué par un acide aminé sélectionné parmi une Alanine, une Méthionine, une Sérine, une Glycine, et une Leucine, de manière plus préférée une Alanine.

9. Variant selon l'une quelconque des revendications 1-8, **caractérisé en ce qu'**il présente une délétion partielle ou totale du domaine B.

10. Acide nucléique codant un variant selon l'une quelconque des revendications 1-9.

11. Cassette ou vecteur d'expression d'un acide nucléique selon la revendication 10.

12. Composition pharmaceutique comprenant un variant selon l'une quelconque des revendications 1-9.

13. Variant selon l'une quelconque des revendications 1-9 en tant que médicament.

14. Variant selon l'une quelconque des revendications 1-9 pour l'utilisation dans le traitement de l'hémophilie de type A.

15. Variant selon la revendication 14, **caractérisé en ce que** le patient à traiter est un patient hémophile à inhibiteurs ou un patient hémophile avant l'apparition d'inhibiteurs.

16. Utilisation d'un ou plusieurs variants selon l'une quelconque des revendications 1-9 pour le diagnostic du type d'inhibiteurs chez un patient hémophile de type A.

## Patentansprüche

1. Variante des humanen FVIII oder eines biologisch aktiven Derivates davon, die eine prokoagulierende Aktivität des humanen FVIII konserviert, **dadurch gekennzeichnet, dass** sie eine Substitution des Restes an Position 462 der Domäne A2 umfasst, wobei die Position in der SEQ ID NR: 3 angegeben ist, und **dadurch gekennzeichnet, dass** besagte Variante eine prokoagulierende Aktivität von wenigstens 50% des natürlichen humanen FVIII konserviert und eine Verringerung der Antigenität gegenüber Hemmantikörpern verglichen mit dem natürlichen humanen FVIII aufweist.

2. Variante gemäß Anspruch 1, **dadurch gekennzeichnet, dass** sie eine einzige Substitution umfasst.

3. Variante gemäß Anspruch 1, **dadurch gekennzeichnet, dass** sie außerdem eine Substitution wenigstens einer Aminosäure umfasst, ausgewählt aus der Gruppe, die aus dem Rest an Position 2202 der Domäne C2 und dem Rest an Position 437 der Domäne A2 besteht, wobei die Positionen in der SEQ ID NR: 3 angegeben sind.

4. Variante gemäß Anspruch 1, **dadurch gekennzeichnet, dass** sie eine Kombination aus zwei Substitutionen umfasst oder aufweist, ausgewählt aus der Gruppe, bestehend aus 409 + 462, 462 + 507 und 462 + 629, wobei die Positionen in der SEQ ID NR: 3 angegeben sind.

5. Variante gemäß Anspruch 1, **dadurch gekennzeichnet, dass** sie eine Kombination aus drei Substitutionen, ausgewählt aus der Gruppe, bestehend aus 409 + 462 + 507, 462 + 507 + 629 und 409 + 462 + 629, wobei die Positionen in der SEQ ID NR: 3 angegeben sind, vorzugsweise eine Kombination aus vier Substitutionen an den Positionen 409, 462, 507 und 629, wobei die Positionen in der SEQ ID NR: 3 angegeben sind, umfasst oder aufweist.

6. Variante gemäß Ansprüchen 1 bis 5, **dadurch gekennzeichnet, dass** sie außerdem eine Substitution wenigstens einer Aminosäure umfasst, ausgewählt aus der Gruppe, die aus den Resten an Positionen 2177, 2183, 2186, 2191, 2196, 2204, 2205, 2213, 2217, 2235, 2258, 2264, 2268 und 2269 der Domäne C2 besteht, wobei die Positionen in der SEQ ID NR: 3 angegeben sind.

7. Variante gemäß Ansprüchen 1 bis 6, **dadurch gekennzeichnet, dass** sie außerdem eine Substitution wenigstens einer Aminosäure umfasst, ausgewählt aus der Gruppe, die aus den Resten an Positionen 2175, 2199, 2200, 2215, 2251, 2252 und 2278 der Domäne C2 besteht, wobei die Positionen in der SEQ ID NR: 3 angegeben sind.

8. Variante gemäß einem der Ansprüche 1 bis 7, **dadurch gekennzeichnet, dass** die Polypeptidsequenz der Variante sich von der Sequenz SEQ ID NR: 3 durch 1, 2, 3, 4, 5, 6, 7, 8, 9, 10, 11, 12, 13, 14 oder 15 Substitutionen unterscheidet, ohne (eine) eventuelle Deletion(en) oder Verkürzung(en) zu berücksichtigen, wobei vorzugsweise die Aminosäure durch eine Aminosäure substituiert ist, die aus Alanin, Methionin, Serin, Glycin und Leucin, bevorzugt Alanin, ausgewählt ist.

9. Variante gemäß einem der Ansprüche 1 bis 8, **dadurch gekennzeichnet, dass** sie eine partielle oder totale Deletion der Domäne B aufweist.

10. Nukleinsäure, die eine Variante gemäß einem der Ansprüche 1 bis 9 codiert.

11. Expressionskassette oder Expressionsvektor einer Nukleinsäure gemäß Anspruch 10.

12. Pharmazeutische Zusammensetzung, umfassend eine Variante gemäß einem der Ansprüche 1 bis 9.

13. Variante gemäß einem der Ansprüche 1 bis 9 als Medikament.

14. Variante gemäß einem der Ansprüche 1 bis 9 zur Verwendung in der Behandlung von Hämophilie des Typs A.

15. Variante gemäß Anspruch 14, **dadurch gekennzeichnet, dass** der zu behandelnde Patient ein hämophiler Patient mit Hemmkörpern ist oder ein hämophiler Patient vor der Entwicklung von Hemmkörpern ist.

16. Verwendung einer oder mehrerer Varianten gemäß einem der Ansprüche 1 bis 9 für die Bestimmung des Hemmkörpertyps bei einem Patienten mit Hämophilie des Typs A.

## Claims

1. Human FVIII variant or a biologically active derivative thereof retaining a procoagulant activity of human FVIII, **characterized in that** it comprises a substitution of the residue at position 462 of the A2 domain, the position being indicated in SEQ ID No. 3, and **in that** said variant retains a procoagulant activity at least equal to 50% of that of natural human FVIII and has decreased antigenicity towards inhibitory antibodies as compared to natural human FVIII.

2. Variant according to claim 1, **characterized in that** it comprises a single substitution.

3. Variant according to claim 1, **characterized in that** it further comprises a substitution of at least one amino acid selected from the group consisting of the residue at position 2202 of the C2 domain and the residue at position 437 of the A2 domain, the positions being indicated in SEQ ID No. 3.

4. Variant according to claim 1, **characterized in that** it comprises or consists of a combination of two substitutions selected from the group consisting of 409 + 462, 462 + 507 and 462 + 629, the positions being indicated in SEQ ID No. 3.

5. Variant according to claim 1, **characterized in that** it comprises or consists of a combination of three substitutions selected from the group consisting of 409 + 462 + 507, 462 + 507 + 629 and 409 + 462 + 629, the positions being indicated in SEQ ID No. 3, preferably a combination of four substitutions at positions 409, 462, 507 and 629, the positions being indicated in SEQ ID No. 3.

6. Variant according to claims 1 to 5, **characterized in that** it further comprises a substitution of at least one amino acid selected from the group consisting of the residues at positions 2177, 2183, 2186, 2191, 2196, 2204, 2205, 2213, 2217, 2235, 2258, 2264, 2268 and 2269 of the C2 domain, the positions being indicated in SEQ ID No. 3.

7. Variant according to claims 1 to 6, **characterized in that** it further comprises a substitution of at least one amino acid selected from the group consisting of the residues at positions 2175, 2199, 2200, 2215, 2251, 2252 and 2278 of the C2 domain, the positions being indicated in SEQ ID No. 3.

8. Variant according to any one of claims 1 to 7, **characterized in that** the polypeptide sequence of the variant differs from the sequence SEQ ID No. 3 by 1, 2, 3, 4, 5, 6, 7, 8, 9, 10, 11, 12, 13, 14 or 15 substitutions, without including optional deletion(s) or truncation(s), preferably the amino acid being substituted by an amino acid selected from an Alanine, a Methionine, a Serine, a Glycine, and a Leucine, more preferably an Alanine.

9. Variant according to any one of claims 1 to 8, **characterized in that** it consists in a partial or whole deletion of domain B.

10. Nucleic acid coding for a variant according to any one of claims 1 to 9.

11. Expression vector or cassette of a nucleic acid according to claim 10.

12. Pharmaceutical composition comprising a variant according to any one of claims 1 to 9.

13. Variant according to any one of claims 1 to 9 as medicament.

14. Variant according to any one of claims 1 to 9 for use in the treatment of hemophilia A.

15. Variant according to claim 14, **characterized in that** the patient to be treated is a hemophiliac patient with inhibitors or a hemophiliac patient before the development of inhibitors.

16. Use of one or more variants according to any one of claims 1 to 9 for the diagnosis of inhibitor type in a patient with hemophilia A.
